# EUROPEAN PATENT APPLICATION

(11) **EP 4 730 355 A1**
(43) Date of publication of application: **22.04.2026**
(21) Application number: 24806685.4
(22) Date of filing: 20.05.2024
(51) Int. Cl.: G16H 50/20

(54) **TYPING SYSTEM AND METHOD FOR HR-POSITIVE/HER2-NEGATIVE BREAST CANCER**

(30) Priority: 18.05.2023 CN 202310567930; 19.09.2023 CN 202311210734; 19.09.2023 CN 202311210735; 19.09.2023 WO PCT/CN2023/119869
(71) Applicant: Lumitype Medical (Huzhou) Co., Ltd., Huzhou, Zhejiang 313205 (CN)
(72) Inventor: SHAO, Zhimin, Shanghai 200032 (CN); JIANG, Yizhou, Shanghai 200032 (CN); HU, Xin, Shanghai 200032 (CN); JIN, Xi, Shanghai 200032 (CN); ZHAO, Shen, Shanghai 200032 (CN); ZHOU, Yifan, Shanghai 200032 (CN); ZHAO, Yaxin, Shanghai 200032 (CN); SU, Guanhua, Shanghai 200032 (CN); XIAO, Wenxuan, Shanghai 200032 (CN); ZHANG, Huyunlong, Shanghai 200032 (CN)
(74) Representative: HGF
(86) International application number: PCT/CN2024/094317
(87) International publication number: WO 2024/235337

(57) **Abstract**

The present invention relates to a novel typing system and method for HR-positive/HER2-negative breast cancer. According to a multi-omics result of HR-positive/HER2-negative breast cancer, HR-positive/HER2-negative breast cancer has four subtypes, namely a classic luminal subtype, an immunomodulatory subtype, a proliferative subtype, and a receptor tyrosine kinase-driven subtype. Different treatment schemes are adopted for the four subtypes, so that accurate treatment for different subtypes of HR-positive/HER2-negative breast cancer can be achieved. The present invention further relates to a typing method for HR-positive/HER2-negative breast cancer based on Al digital pathology or a classifier gene expression.

## Description

### Cross-References

The present application claims priority to Chinese Application No. 202310567930.4 filed on May 18, 2023, Chinese Application No. 202311210735.2 filed on September 19, 2023, Chinese Application No. 202311210734.8 filed on September 19, 2023, and PCT Application No. PCT/CN2023/119869 filed on September 19, 2023, each priority application being incorporated by reference in its entirety for all purposes.

### Technical Field

The present invention relates to the field of cancer diagnosis and treatment, particularly to the typing of HR-positive/HER2-negative breast cancer.

### Background

Luminal-subtype breast cancer mainly consists of breast cancer with positive expression of estrogen receptor (ER) or progesterone receptor (PR) (collectively hormone receptor HR) and negative expression of human epidermal growth factor receptor 2 (HER2) (HR⁺ HER2-) and accounts for two-thirds of all breast cancer. Moreover, the vast majority of breast cancer recurrence and metastasis cases are patients with luminal subtype breast cancer.

Luminal-subtype breast cancer is highly reactive to endocrine therapy, but there is still a risk of recurrent metastases at distant locations in patients due to factors such as intertumoral heterogeneity and endocrine resistance. Despite the emergence of numerous immunotherapies, targeted therapies and other drugs targeting luminal subtype breast cancer in recent years, systematic guidance for accurate treatment of luminal subtype breast cancer is still lacking. Therefore, it is necessary to further explore the heterogeneity of luminal subtype breast cancer in order to construct a more accurate treatment system for luminal subtype breast cancer.

### Summary

The present invention provides an accurate treatment scheme for HR-positive/HER2-negative breast cancer, which is achieved by establishing a novel typing system for HR-positive/HER2-negative breast cancer. Specifically, the present invention provides a method for implementing accurate treatment for patients with HR-positive/HER2-negative breast cancer, and the method comprises: determining the subtype of HR-positive/HER2-negative breast cancer in the patient, with the subtype being one of the following four: classic luminal subtype (SNF1), immunomodulatory subtype (SNF2), proliferative subtype (SNF3), and receptor tyrosine kinase-driven subtype (SNF4); The following scheme is adopted to implement accurate treatment for the patient: endocrine therapy is administered to patients with classic luminal subtype, immunotherapy is administered to patients with immunomodulatory subtype, cyclin-dependent kinase (CDK)4/6 inhibitors, or poly(adenosine diphosphate-ribose) polymerase (PARP) inhibitors, or a combination of CDK4/6 inhibitors and PARP inhibitors are administered to patients with proliferative subtype, and tyrosine kinase inhibitors such as platelet-derived growth factor receptor (PDGFR) signaling pathway inhibitors are administered to patients with receptor tyrosine kinase-driven subtype.

The present invention provides a typing system for HR-positive/HER2-negative breast cancer, wherein the system divides HR-positive/HER2-negative breast cancer into the following four subtypes: classic luminal subtype, immunomodulatory subtype, proliferative subtype, and receptor tyrosine kinase-driven subtype, wherein the immunohistochemical scores of these four subtypes are respectively:
classic luminal subtype: α-SMA < 20%, CD8 < 3%, Ki67 < 25%, the above three conditions are simultaneously satisfied;
immunomodulatory subtype: α-SMA < 20%, CD8 ≥ 3%, the above two conditions are simultaneously satisfied;
proliferative subtype: α-SMA < 20%, CD8 < 3%, Ki67 ≥ 25%, the above three conditions are simultaneously satisfied;
receptor tyrosine kinase-driven subtype: α-SMA ≥ 20%.

The present invention further provides a typing method for HR-positive/HER2-negative breast cancer, wherein the method divides HR-positive/HER2-negative breast cancer into the following four subtypes: classic luminal subtype, immunomodulatory subtype, proliferative subtype, and receptor tyrosine kinase-driven subtype, and the method comprises: performing immunohistochemical scoring on the breast cancer and making the following determinations:
if α-SMA < 20%, CD8 < 3%, and Ki67 < 25%, and the above three conditions are simultaneously satisfied, the breast cancer is determined as classic luminal subtype; or
if α-SMA < 20% and CD8 ≥ 3%, and the above two conditions are simultaneously satisfied, the breast cancer is determined as immunomodulatory subtype; or
if α-SMA < 20%, CD8 < 3%, and Ki67 ≥ 25%, and the above three conditions are simultaneously satisfied, the breast cancer is determined as proliferative subtype; or
if α-SMA ≥ 20%, the breast cancer is determined as receptor tyrosine kinase-driven subtype.

In some embodiments, prior to performing immunohistochemistry, the tumor sample is stained, for example stained with hematoxylin-eosin.

The present invention further provides a typing system for HR-positive/HER2-negative breast cancer, and the system comprises:
1) a whole-slide digital pathology image data preprocessing module, wherein the module is configured to segment whole-slide digital pathology images of tumor tissue samples of HR-positive/HER2-negative breast cancer into multiple enlarged image patches;
2) a first neural network module, wherein the first neural network module is a tissue type segmentation neural network module, and is configured to classify the image patches into different tissue types and assign corresponding tissue labels;
3) a second neural network module connected in series with the first neural network module, wherein the second neural network module is a subtype prediction neural network module, and is configured to perform the typing on the samples based on the image patches with the tissue labels;
   wherein the typing refers to dividing HR-positive/HER2-negative breast cancer into the following four subtypes: classic luminal subtype, immunomodulatory subtype, proliferative subtype, and receptor tyrosine kinase-driven subtype.

The present invention further provides a chip, wherein the chip comprises a processor configured to invoke and execute a computer program from a memory, such that a device equipped with the chip performs the typing method of the present invention.

The present invention further provides a computer-readable storage medium having a computer program stored thereon, and when the computer program is executed by a processor, the typing method of the present invention is implemented.

The present invention provides an accurate treatment scheme for HR-positive/HER2-negative breast cancer.

In one of the aforementioned accurate treatment schemes of the present invention, the HR-positive/HER2-negative breast cancer is identified as classic luminal subtype, and the patient is subjected to endocrine therapy or administered a PI3K inhibitor or a CDK4/6 inhibitor.

In one of the aforementioned accurate treatment schemes of the present invention, the HR-positive/HER2-negative breast cancer is identified as immunomodulatory subtype, and the patient is subjected to immunotherapy.

In one of the aforementioned accurate treatment schemes of the present invention, the HR-positive/HER2-negative breast cancer is identified as proliferative subtype, and the patient is administered a cyclin-dependent kinase (CDK)4/6 inhibitor, or a poly(adenosine diphosphate-ribose) polymerase (PARP) inhibitor, or a combination of a CDK4/6 inhibitor and a PARP inhibitor.

In one of the aforementioned accurate treatment schemes of the present invention, the HR-positive/HER2-negative breast cancer is identified as receptor tyrosine kinase-driven subtype, and the patient is administered a tyrosine kinase inhibitor.

In a preferred embodiment, the endocrine therapy comprises administering an estrogen receptor antagonist, preferably administering 4-hydroxytamoxifen and fulvestrant.

In a preferred embodiment, the immunotherapy comprises administering an immune checkpoint inhibitor, preferably administering a PD-1 antibody, PD-L1 antibody, or CTLA4 antibody.

In a preferred embodiment, the tyrosine kinase inhibitor is a VEGFR or PDGFR signaling pathway inhibitor, preferably famitinib, sorafenib, regorafenib, vandetanib, afatinib, lapatinib, pyrotinib, axitinib, sunitinib, foretinib, apatinib, or sitravatinib.

In a preferred embodiment, the CDK4/6 inhibitor is abemaciclib; and the PARP inhibitor is olaparib.

The present invention further provides the use of the substance in the preparation of a medicament for accurate treatment of HR-positive/HER2-negative breast cancer, specifically including the following use:
(1) the use of an estrogen receptor antagonist in the preparation of a medicament for the treatment of classic luminal subtype breast cancer. The estrogen receptor antagonist is preferably 4-hydroxytamoxifen and fulvestrant.
(2) the use of an immune checkpoint inhibitor in the preparation of a medicament for the treatment of immunomodulatory subtype breast cancer. The immune checkpoint inhibitor is preferably a PD-1 antibody, PD-L1 antibody, or CTLA4 antibody.
(3) the use of a CDK4/6 inhibitor, or a PARP inhibitor, or a combination of a CDK4/6 inhibitor and a PARP inhibitor in the preparation of a medicament for the treatment of proliferative subtype breast cancer. The CDK4/6 inhibitor is preferably abemaciclib; the PARP inhibitor is preferably olaparib.
(4) the use of a tyrosine kinase inhibitor in the preparation of a medicament for the treatment of receptor tyrosine kinase-driven subtype breast cancer. The tyrosine kinase inhibitor is preferably a VEGFR or PDGFR signaling pathway inhibitor, or preferably famitinib, sorafenib, regorafenib, vandetanib, afatinib, lapatinib, pyrotinib, axitinib, sunitinib, foretinib, apatinib, or sitravatinib.

The present invention is based on a novel typing system for HR-positive/HER2-negative breast cancer, establishing a new typing method for HR-positive/HER2-negative breast cancer, and further establishing an accurate treatment system for HR-positive/HER2-negative breast cancer. The typing system and method for HR-positive/HER2-negative breast cancer based on digital pathology and artificial intelligence provided by the present invention can achieve typing of HR-positive/HER2-negative breast cancer patients in a simple and efficient manner clinically, thereby providing the premise and means for achieving accurate diagnosis and treatment of HR-positive/HER2-negative breast cancer at the clinical level.

Based on the sensitivity of different subtypes of HR-positive/HER2-negative breast cancer to different treatment schemes, the present invention, by implementing distinct accurate treatment schemes, can achieve better therapeutic effects than conventional breast cancer treatment schemes, leading to a more favorable prognosis.

### Brief Description of the Drawings

FIG. 1 shows a summary of the four-subtype classification of HR-positive/HER2-negative breast cancer of the present invention.
FIG. 2 shows the distribution of PAM50 subtypes in the multi-omics cohort of the present invention.
FIG. 3 shows the determination of the optimal number of clusters in the SNF clustering analysis of the present invention.
FIG. 4 shows a silhouette plot of the SNF clustering of the present invention.
FIG. 5 shows the results of the immunohistochemical analysis of SNF1-4 subtypes.
FIG. 6 shows the results of the receiver operating characteristic curve analysis of the three markers used in immunohistochemical analysis.
FIG. 7 shows a result of a comparison between the proportion of predicted SNF subtypes based on immunohistochemical scoring of the present invention and the proportion of SNF subtypes in the luminal subtype multi-omics cohort.
FIG. 8 shows a technical roadmap of digital pathology typing.
FIG. 9 shows the confusion matrix of classification results in the validation set.
FIG. 10 shows a comparison between tissue segmentation results and original pathology images.
FIG. 11 shows a schematic diagram of the three-fold cross-validation used in the modeling process of the present invention.
FIG. 12 shows the ROC curve of each neural network model for discriminating the corresponding subtype in the validation set.
FIG. 13 shows the typical pathological morphology images of each subtype. Each subtype corresponds to 4 image patches, among which the upper two patches are the original images segmented from WSI, and the lower two patches are the original images overlaid with segmentation heatmaps.
FIGs. 14-22 shows a validation of key features of each digital pathology.
FIG. 23 shows a pathology image showing the survival status of SNF1-4 subtypes under conventional endocrine therapy.
FIG. 24 shows the relative survival rates of SNF1-4 subtypes under conventional endocrine therapy.
FIG. 25 shows a heatmap showing the expression of endocrine response modules of SNF1-4 subtypes.
FIG. 26 shows a comparison of flow cytometry results of SNF2 subtype and non-SNF2 subtype after immunotherapy, indicating an increase in CD3+ total T activation markers.
FIG. 27 shows a comparison of flow cytometry results of SNF2 subtype and non-SNF2 subtype after immunotherapy, indicating an increase in cytokines.
FIG. 28 shows the sensitivity of SNF1-4 subtypes to endocrine therapy in early and late HR⁺/HER2⁻breast cancer real-world cohorts.
FIG. 29 shows the PFS time analysis after first-line endocrine therapy for SNF3 subtype and non-SNF3 subtype.
FIG. 30 shows the sensitivity of SNF3 subtype and non-SNF3 subtype to endocrine therapy after long-term induction with 4-hydroxytamoxifen.
FIGs. 31 and 32 respectively show that the PDO growth ability of the SNF3 subtype and the non-SNF3 subtype is significantly inhibited by abemaciclib and olaparib.
FIG. 33 shows that the combination of abemaciclib and olaparib significantly reduces the survival rate of SNF3-PDO.
FIG. 34 shows the RNA-seq results (GSVA scores) of SNF1-4 subtype PDOs.
FIG. 35 shows the homologous recombination deficiency scores of SNF3 and non-SNF3 subtype tumors.
FIG. 36 shows the DRUG-seq results of SNF3 subtype tumors, indicating the changes in signaling pathways after combined administration.
FIG. 37 shows the receptor expression in the receptor tyrosine kinase pathway of SNF1-4 subtype.
FIG. 38 shows the IC₅₀ values of different tyrosine kinase inhibitors for SNF4 tumors.
FIG. 39 shows a pathology image showing the sensitivity of SNF4 tumors to different tyrosine kinase inhibitors.
FIG. 40 shows the changes in the PDGF-activated signaling pathway in SNF4 tumors after treatment with famitinib and sorafenib.
FIG. 41 shows the changes in SNF4 tumor cell viability after treatment with regorafenib and vandetanib.

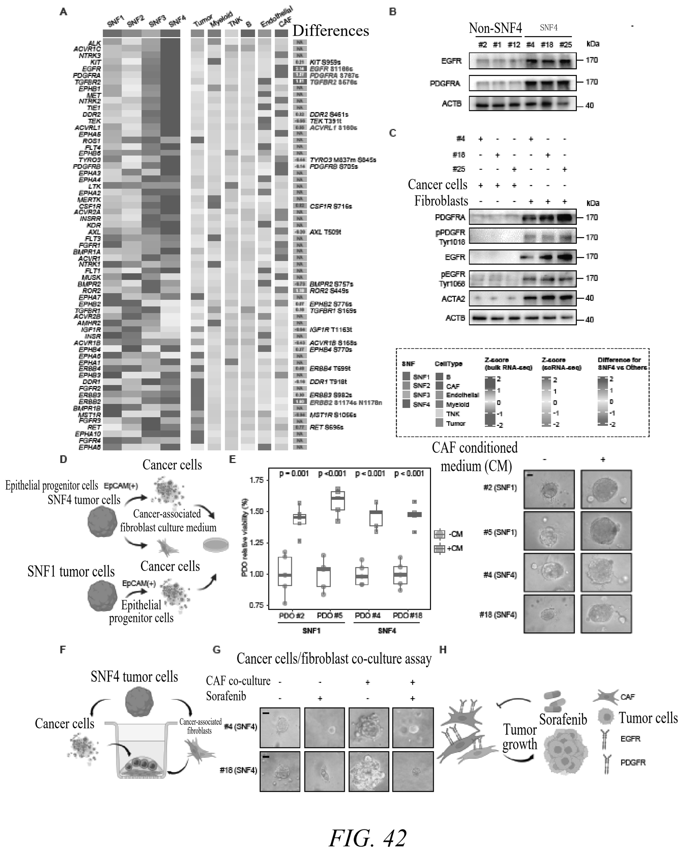

FIG. 42 shows that SNF4 subtype cancer-associated fibroblasts (CAF) can enhance tumor growth and are sensitive to sorafenib, wherein (A) is a heatmap showing Bulk RNA-seq expression (left) and scRNA-seq expression (middle) of the RKT list in the four SNF subtypes. The heatmap on the right shows the differences in phosphorylation abundance between SNF4 and other genes. Kinase genes listed in "GOMF_TRANSMEMBRANE_RECEPTOR_PROTEIN_KINASE_ACTIVITY" (obtained from http://www.gsea-msigdb.org/gsea/msigdb) as well as the previously published carefully curated protein kinase gene list are included. ⁵⁸ List of protein kinase genes. Phosphorylation sites enriched in SNF4 and detected with P < 0.05 are marked in purple. The P value is derived from a two-sided T test. NA: No phosphorylation sites were detected.

(B-C) show Western blot images of EGFR, PDGFRA, p-PDGFRA, and p-EGFR expression in SNF4 tumor or non-SNF4 tumor (B) and SNF4 tumor cells or SNF4 CAF (C). All Western blot experiments were repeated three times, with similar results.

(D-E) Tumor growth viability assay of SNF1 (n = 2) and SNF4 (n = 2) patient-derived cancer cells cultured in SNF4 tumor CAF conditioned medium (CM). Scale line: 100 micrometers. P(PDO#5) = 4.9e-05, P(PDO#4) = 4.1e-05, P(PDO#18) = 5.3e-05. P values are derived from two-sided Wilcoxon tests. The box plot shows the first and third quartiles as the lower and upper limits, the whiskers represent 1.5 times the IQR, and the center indicates the median.

(F-G) The efficacy of sorafenib (1 µM) on patient-derived cancer cells co-cultured with fibroblasts. Scale bar: 100 micrometers. Cancer cells are treated with sorafenib three times. One representative experiment is shown. The experiment is repeated three times, and the results are similar.

(H) Schematically showing how CAF enhances the growth of RTK-driven (SNF4) subtype tumors.

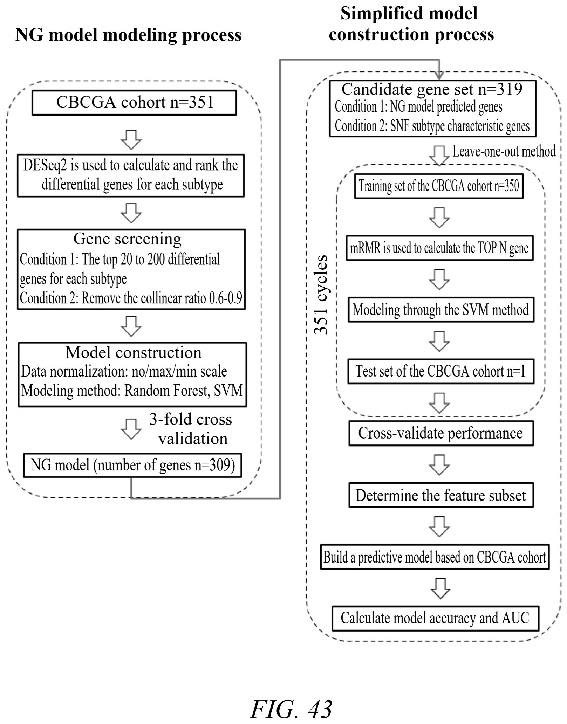

FIG. 43 shows the NG model modeling process (left panel) and the simplified model construction process (right panel).

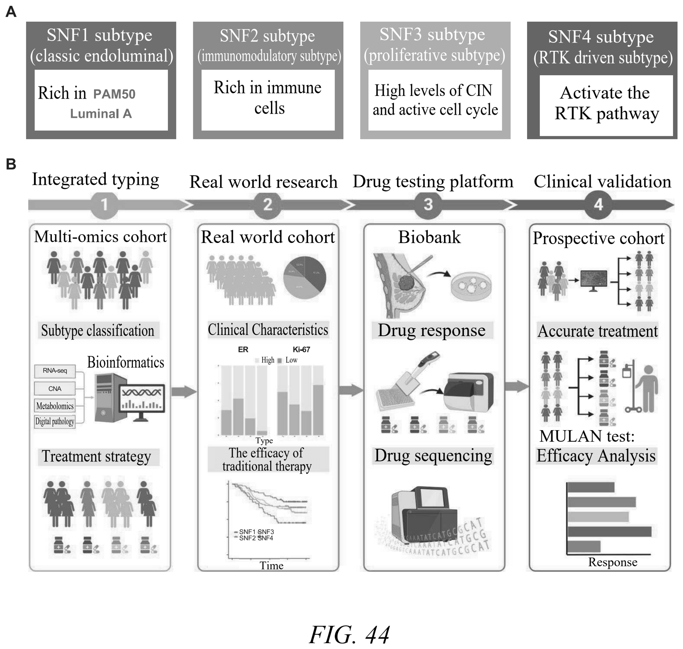

FIG. 44 shows the overall real-world study design. (A) Molecular characteristics of four subtypes of HR+/HER2- breast cancer. (B) Data from multi-omics cohorts, real-world studies, drug testing platforms, and prospective clinical studies are integrated to validate accurate treatment strategies targeting HR+/HER2- breast cancer subtypes. CIN, chromosomal instability; RTK, receptor tyrosine kinase; CNA, copy number alteration; ER, estrogen receptor; HR, hormone receptor; HER2, human epidermal growth factor receptor 2.

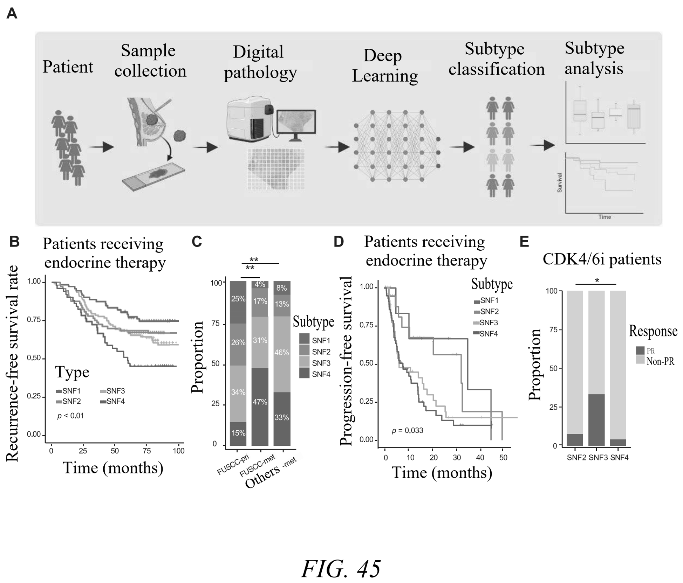

FIG. 45 shows the clinical characteristics and responses to conventional therapies of the HR+/HER2-breast cancer real-world cohort grouped by subtype. (A) Schematic diagram of the real-world cohort study design of HR+/HER2- breast cancer. (B) Recurrence-free survival rate of early HR+/HER2- breast cancer patients receiving endocrine therapy grouped by subtype. (C) A bar chart showing the subtype distribution of primary and metastatic HR+/HER2- breast cancer in the real-world cohort. The P value is calculated by chi-square test. (D) Progression-free survival of advanced HR+/HER2- breast cancer patients receiving fulvestrant backbone treatment grouped by subtype in the FUSCC real-world cohort. (E) According to RECIST v1.1, the efficacy of CDK4/6i on advanced HR+/HER2- breast cancer patients in the FUSCC real-world cohort. The P value is calculated by chi-square test. FUSCC, Fudan University Shanghai Cancer Center; PR, primary lesion; MET, metastatic lesion; CDK4/6i, CDK4/6 inhibitor; PR, partial response; HR, hormone receptor; HER2, human epidermal growth factor receptor 2; RECIST, Response Evaluation Criteria in Solid Tumors. Significance code: NS, no significance; *, P < 0.05; **, P < 0.01; ***, P < 0.001.

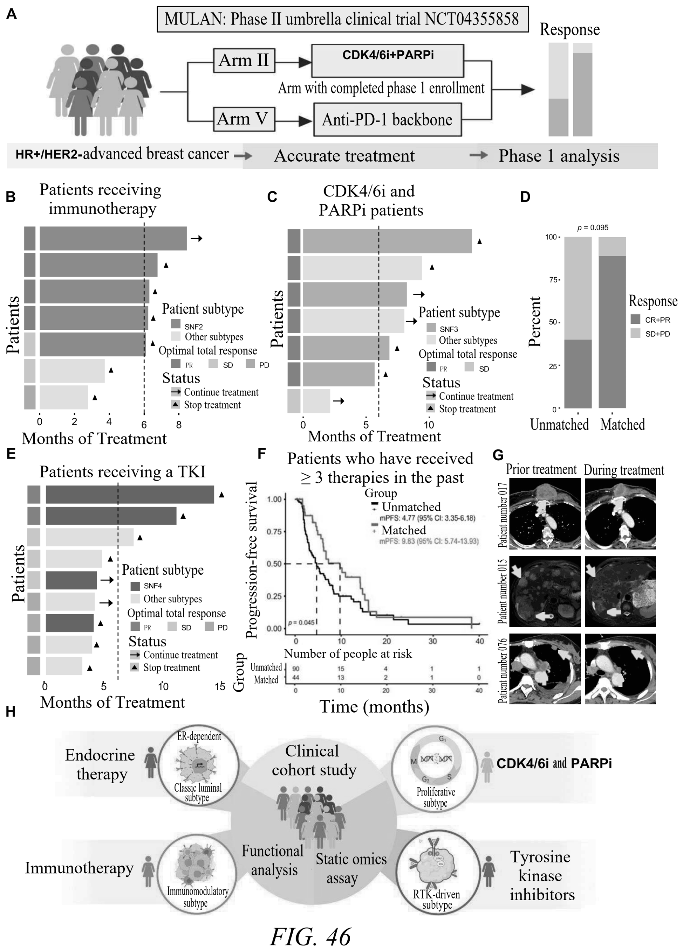

FIG. 46 shows that the prospective clinical cohort validated the subtype-directed accurate treatment strategy. (A) Schematic diagram of the first stage analysis of the MULAN study. (B) Duration of immunotherapy in the fifth group of evaluable patients in the MULAN cohort. The dashed line represents the situation at 6 months of treatment. (C) Durability of CDK4/6i combined with PARPi in the second group of evaluable patients in MULAN. The dashed line represents the data at 6 months of treatment. (D) The objective response rate of patients receiving subtype-directed accurate treatment strategy (matched group) and patients not receiving matched treatment (non-matched group) was evaluated in the MULAN cohort. (E) The TKI persistence of the evaluable patients in real-world clinical cohorts. The dashed line represents the data at 6 months of treatment. (F) Progression-free survival of matched and non-matched accurate treatment strategies in patients with advanced metastatic breast cancer who have previously received ≥ 3 lines of therapy. (G) Baseline and typical images of tumor regression during matched accurate treatment. (Top) Patients with the SNF2 subtype in the fifth group received immunotherapy; (Middle) Patients with the SNF3 subtype in the second group received CDK4/6i and PARPi treatment; (Bottom) Patients with the SNF4 subtype in the real-world cohort received TKI treatment. (8) Research Abstract. By integrating clinical cohort studies, omics analysis, and functional assays, the heterogeneity of treatment response for HR+/HER2- breast cancer is revealed, and a subtype-directed accurate treatment strategy is proposed. Through deep learning, patient subtypes are determined based on digital pathology. The best overall treatment response is defined according to the RECIST v1.1 criteria. CDK4/6i, CDK4/6 inhibitor; PARPi, PARP inhibitor; CR, complete response; PR, partial response; SD, stable disease; PD, progressive disease; TKI, tyrosine kinase inhibitor; mPFS, median progression-free survival; CI, confidence interval; RECIST, Response Evaluation Criteria in Solid Tumors; HR, hormone receptor; HER2, human epidermal growth factor receptor 2.

### Detailed Description of Embodiments

The systems, devices, and methods disclosed herein are described in detail by way of examples and with reference to the accompanying drawings. The examples discussed herein are merely exemplary and are provided to aid in the explanation of the apparatuses, devices, systems, and methods described herein. For any particular implementation of any of these devices, systems, or methods, any features or components shown in the drawings or discussed below shall not be regarded as mandatory unless specifically designated as mandatory.

In addition, for any method described, whether or not combined with a flowchart to describe the method, it should be understood that unless otherwise specified or required by the context, any explicit or implicit ordering of the steps implemented in the execution of the method does not imply that those steps must be performed in the presented order, but rather may be performed in a different order or in parallel.

As used herein, the term "exemplary" is used in the sense of "example," rather than "best" or "ideal". Furthermore, the term "a" and "an" herein does not denote a limitation on quantity, but rather indicates the presence of one or more of the referenced items.

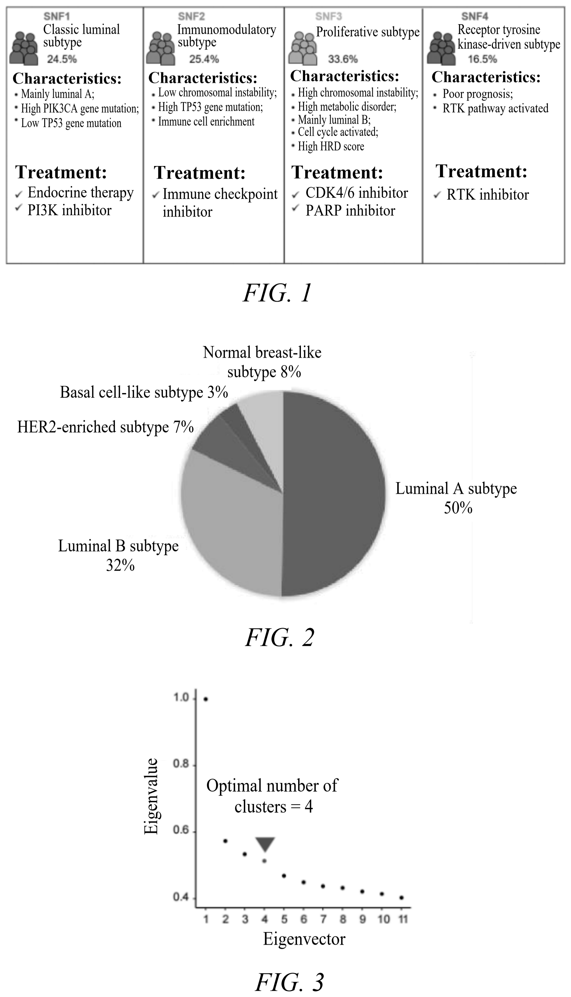

In order to explore the heterogeneity of HR-positive/HER2-negative breast cancer, optimize the traditional treatment strategy for HR⁺/HER2⁻ breast cancer, and construct an accurate treatment system for HR-positive/HER2-negative breast cancer, the present invention divides HR-positive/HER2-negative breast cancer into four subtypes: classic luminal subtype (SNF1), immunomodulatory subtype (SNF2), proliferative subtype (SNF3), and receptor tyrosine kinase (RTK)-driven subtype (SNF4) by utilizing multi-omics data including genomics, transcriptomics, proteomics, and/or metabolomics and by multi-omics integrated clustering through similarity network fusion (SNF) algorithm, and thus reveals characteristic genomic events of each subtype and, based on this, suggests potential treatment schemes for each subtype. 1) Classic luminal subtype: this subtype has favorable prognosis, with the main characteristics being predominantly composed of PAM50 LumA/B, high expression of hormone receptor-related pathways, and a high frequency of PIK3CA mutations; the use of PI3K inhibitors may be considered for subsequent treatment. 2) Immunomodulatory subtype: this subtype has high expression of immune checkpoint molecules and a high level of tumor-infiltrating lymphocytes, and a large amount of PAM50 basal cell-like subtype and HER2-overexpressing subtype are mixed, suggesting that immune checkpoint inhibitors may have better efficacy for the immunomodulatory subtype HR-positive/HER2-negative breast cancer. 3) Proliferative subtype: this subtype has activated cell cycle pathway, with accompanied CCND1 and MDM2 amplification as well as ATM loss and high expression of E2F1, E2F2, and E2F-targeted genes, indicating that CDK4/6 inhibitors combined with PARP inhibitors can be used for this subtype. 4) Receptor tyrosine kinase-driven subtype: this subtype has the worst prognosis, contains almost all PAM50 normal breast-like subtypes, while EGFR is highly expressed with accompanied activation of receptor tyrosine kinase-related pathways, indicating potential benefit from receptor tyrosine kinase inhibitor therapy (see FIG. 1).

The transcriptomic characteristics of each subtype can be summarized as follows:
SNF1: it is the PAM50 luminal A/B mixed subtype, enriched in hormone receptor-related pathways;
SNF2: it includes almost all PAM50 HER2-overexpressing subtype and basal cell-like subtype, contains a lower proportion of PAM50 luminal A/B subtypes, exhibits lower activation levels of hormone receptor-related pathways, is enriched with a large number of adaptive immune response pathways, shows lower estrogen receptor expression compared to other subtypes, has higher levels of tumor inflammation index and expression of several immune checkpoint biomarkers including programmed cell death protein-1 (PDCD1), and has significantly increased mRNA levels of several cytotoxic factors such as cluster of differentiation 8A (CD8A), granzyme A (GZMA), and perforin 1 (PRF1);
SNF3: it is mainly composed of the PAM50 luminal B subtype, has more activation of cell cycle pathways, higher mRNA expression of CND1, CDK1, and CDK2, higher mRNA expression of E2F1, E2F2, and E2F target genes, high mRNA expression of the G2/M cell cycle activation gene MDM2, low mRNA expression of the G2/M cell cycle inhibitory gene ATM, and enrichment of hormone receptor-related pathways;
SNF4: it contains a high proportion of PAM50 normal breast-like subtype, has relatively low activation level of hormone receptor-related pathways, enrichment of pathways related to stromal components, high enrichment of expression characteristics related to receptor protein kinases and fibroblasts, significant enrichment of the receptor tyrosine kinase pathway, and high expression of EGFR and several key receptor tyrosine kinases.

The metabolomic characteristics of each subtype can be summarized as follows:
SNF1: metabolomic features are not obvious, and no significantly upregulated or downregulated metabolic pathways are found;
SNF2: L-kynurenine and indoleamine 2,3-dioxygenase 1 (IDO1, the rate-limiting enzyme in the kynurenine metabolic pathway) levels are higher, and the abundance of phosphatidylserine is higher in SNF2 patients with poor prognosis;
SNF3: each metabolic pathway is more active compared with other subtypes, among which the abundance of amino acid- and nucleotide-related metabolites is relatively higher, the abundance of citric acid cycle-related metabolites is relatively lower, and the abundance of glutamine and L-asparagine as well as the expression quantity of amino acid transporter solute carrier 1A5 (SLC1A5) are increased;
SNF4: overall metabolism is weaker compared with other subtypes, presenting a state of metabolic "inertia".

The proteomic characteristics of each subtype can be summarized as follows:
SNF1: protein changes are overall similar to the transcriptome, see the relevant description in the transcriptome section;
SNF2: protein changes are overall similar to the transcriptome, see the relevant description in the transcriptome section, wherein typical events are such as significantly elevated protein levels of several cytotoxic factors including CD8A, GZMA, and PRF1;
SNF3: protein changes are overall similar to the transcriptome, see the relevant description in the transcriptome section, wherein typical events are such as higher protein levels of CCND1, CDK1, and CDK2;
SNF4: protein changes are overall similar to the transcriptome, see the relevant description in the transcriptome section.

The microenvironment characteristics of each subtype can be summarized as follows:
SNF1: microenvironment-related molecules are lowly expressed, tending toward cold tumor characteristics;
SNF2: multiple immune cell subpopulations such as adaptive immune cells are significantly increased, both iTIL and sTIL are higher, T cell receptor (T cell receptor α and T cell receptor β) diversity is higher, tending towards hot tumor characteristics;
SNF3: microenvironment-related molecules are lowly expressed, tending toward cold tumor characteristics;
SNF4: endothelial cells and fibroblasts are enriched, tending toward immune-excluded tumor characteristics.

The somatic mutation characteristics of each subtype can be summarized as follows:
SNF1: more than half of the tumors have phosphatidylinositol-3-kinase CA (PIK3CA) mutations, while the tumor protein P53 (TP53) is less mutated;
SNF2: it exhibits the highest TP53 mutation rate and possesses a lower copy number load;
SNF3: it has copy number amplification of cyclin D1 (CCND1)/fibroblast growth factor receptor 1 (FGFR1)/murine double minute 2 (MDM2), copy number loss of breast cancer 2 gene (BRCA2), copy number amplification of G2/M cell cycle activation gene MDM2, higher levels of copy number loss of G2/M cell cycle inhibitory gene - ataxia telangiectasia mutated gene (ATM), high chromosomal instability and whole genome doubling (WGD), significantly increased chromosomal instability (CIN) score and homologous recombination deficiency (HRD) score, higher frequency of simultaneous occurrence of CCND1 amplification and ATM loss in SNF3 recurrent metastatic tumors;
SNF4: somatic mutation features are not significant.

The morphological characteristics of each subtype (as shown in hematoxylin-eosin staining sections) can be summarized as follows:
SNF1: the morphology of normal breast glands is partially retained;
SNF2: immune cell infiltration is high;
SNF3: pleomorphic tumor cells are abundant;
SNF4: fibroblasts are enriched around tumor cells.

The clinicopathological characteristics of each subtype can be summarized as follows:
SNF1: the best prognosis;
SNF2: no obvious features;
SNF3: higher Ki-67 proliferation index, histological grading;
SNF4: the worst prognosis.

In the typing system and method for HR-positive/HER2-negative breast cancer of the present invention, the immunohistochemical scores obtained for the four subtypes are respectively:
classic luminal subtype: α-SMA < 20%, CD8 < 3%, Ki67 < 25%, the above three conditions are simultaneously satisfied;
immunomodulatory subtype: α-SMA < 20%, CD8 ≥ 3%, the above two conditions are simultaneously satisfied;
proliferative subtype: α-SMA < 20%, CD8 < 3%, Ki67 ≥ 25%, the above three conditions are simultaneously satisfied;
receptor tyrosine kinase-driven subtype: α-SMA ≥ 20%.

In other words, it is possible to determine which subtype a particular breast cancer belongs to based on the immunohistochemical characteristics described above. In practical operation, for convenience, the method of the present invention may be implemented in the following order:
if α-SMA ≥ 20%, the breast cancer is determined as receptor tyrosine kinase-driven subtype; or
if α-SMA < 20% and CD8 ≥ 3%, and the above two conditions are simultaneously satisfied, the breast cancer is determined as immunomodulatory subtype; or
if α-SMA < 20%, CD8 < 3%, and Ki67 ≥ 25%, and the above three conditions are simultaneously satisfied, the breast cancer is determined as proliferative subtype; or
if α-SMA < 20%, CD8 < 3%, and Ki67 < 25%, and the above three conditions are simultaneously satisfied, the breast cancer is determined as classic luminal subtype.

When implementing the above typing method for HR-positive/HER2-negative breast cancer, according to actual needs or based on the judgment of a clinician, the typing may be further confirmed according to other characteristics of each of the four subtypes, the other characteristics include one or more of the following characteristics: transcriptomics characteristics, metabolomics characteristics, proteomics characteristics, microenvironment characteristics, somatic mutation characteristics, morphological characteristics, and clinicopathological characteristics.

Although the typing of HR-positive/HER2-negative breast cancer can simultaneously reveal the biological characteristics of HR-positive/HER2-negative breast cancer and suggest subtype-specific therapeutic strategies, considering the time cost and economic cost, it is difficult to perform multi-omics sequencing for every HR-positive/HER2-negative breast cancer patient in actual clinical practice. Therefore, there is an urgent need to find a method of typing that can be used to recapitulate HR-positive/HER2-negative breast cancer patients with clinical simplicity, efficiency and relative accuracy.

The present invention provides a typing system for HR-positive/HER2-negative breast cancer, and the system comprises:
1) a whole-slide digital pathology image (WSI) data preprocessing module, wherein the module is configured to segment the whole-slide digital pathology images of tumor tissue samples of HR-positive/HER2-negative breast cancer into multiple enlarged image patches;
2) a first neural network module, wherein the first neural network module is a tissue type segmentation neural network module, and is configured to classify the image patches into different tissue types and assign corresponding tissue labels;
3) a second neural network module connected in series with the first neural network module, wherein the second neural network module is a subtype prediction neural network module, and is configured to perform the typing on the samples based on the image patches with the tissue labels;
   wherein the typing refers to dividing HR-positive/HER2-negative breast cancer into the following four subtypes: classic luminal subtype, immunomodulatory subtype, proliferative subtype, and receptor tyrosine kinase-driven subtype.

In some embodiments, the whole-slide digital pathology image data preprocessing module is configured to segment the whole-slide digital pathology image into image patches with a magnification of 10-40 times, preferably segmented into image patches of 256*256 pixels, 512*512 pixels, or 800*800 pixels at 20 times magnification.

The first neural network module is a tissue type segmentation neural network, and configured to classify the preprocessed image patches of the whole-slide digital pathology image into different tissue types and assign corresponding tissue labels. The tissue labels are preferably five tissue types: tumor, stroma, immune infiltration, normal gland, and necrosis or hemorrhage; the tissue labels are preferably tumor tissue label, stromal tissue label, immune infiltration tissue label, normal glandular tissue label, and necrosis or hemorrhage tissue label.

The second neural network module is a subtype prediction neural network, configured to determine the HR-positive/HER2-negative breast cancer subtype of the sample based on image patches with specific tissue labels. The specific tissue labels are preferably tumor tissue labels, stromal tissue labels, and/or immune infiltration tissue labels.

In a preferred embodiment of the present invention, the first neural network module and the second neural network module are constructed by artificial intelligence deep learning based on whole-slide digital pathology images of tumor tissue samples from patients with known HR-positive/HER2-negative breast cancer subtypes.

In some embodiments, the number of whole-slide digital pathology images of tumor pathological sections from patients with known HR-positive/HER2-negative breast cancer subtypes is 100 or more, preferably 200 or more, 220 or more, 230 or more, 240 or more, 200-300, 220-280, 230-270, 240-260, 242-250, or 244; preferably, each whole-slide digital pathology image of the tumor pathological section from patients with known HR-positive/HER2-negative breast cancer subtypes corresponds to one patient with known HR-positive/HER2-negative breast cancer subtype. Of course, the present invention can be accomplished without such one-to-one correspondence. Those skilled in the art, based on the disclosure of the present invention, can make some equivalent substitutions, such as multiple whole-slide digital pathology images corresponding to some patients.

In one aspect, the present invention provides a typing method or system for HR-positive/HER2-negative breast cancer, which divides HR-positive/HER2-negative breast cancer into the following four subtypes: classic luminal subtype (SNF1), immunomodulatory subtype (SNF2), proliferative subtype (SNF3), and receptor tyrosine kinase-driven subtype (SNF4), wherein:

The classic luminal subtype has one or more or all of the following characteristics: (1) at least 50% of the SNF1 high-expression genes in Table 1 or 4 exhibit enhanced expression in tumor samples relative to normal breast samples; (2) negative expression of α-SMA, negative expression of CD8, and negative expression of Ki67 in tumor samples; (3) normal ductal epithelial cells in the pathology image of the tumor sample with a proportion equal to or exceeding a predetermined threshold; (4) sensitive to endocrine therapy;
the immunomodulatory subtype has one or more or all of the following characteristics: (1) at least 50% of the SNF2 high-expression genes in Table 1 or 4 exhibit enhanced expression in tumor samples relative to normal breast samples; (2) negative expression of α-SMA and positive expression of CD8 in tumor samples; (3) immune cells in the pathology image of the tumor sample with a proportion equal to or exceeding a predetermined threshold; (4) sensitive to immunotherapy;
the proliferative subtype has one or more or all of the following characteristics: (1) at least 50% of the SNF3 high-expression genes in Table 1 or 4 exhibit enhanced expression in tumor samples relative to normal breast samples; (2) negative expression of α-SMA, negative expression of CD8, and positive expression of Ki67 in tumor samples; (3) pleomorphic tumor cells in the pathology image of the tumor sample with a proportion equal to or exceeding a predetermined threshold; (4) sensitive to cyclin-dependent kinase (CDK) 4/6 inhibitors, poly(adenosine diphosphate-ribose) polymerase (PARP) inhibitors, or a combination of CDK4/6 inhibitors and PARP inhibitors;
the receptor tyrosine kinase-driven subtype has one or more or all of the following characteristics: (1) at least 50% of the SNF4 high-expression genes in Table 1 or 4 exhibit enhanced expression in tumor samples relative to normal breast samples; (2) positive expression of α-SMA in tumor samples; (3) cancer-associated fibroblasts in the pathology image of the tumor sample with a proportion equal to or exceeding a predetermined threshold; (4) sensitive to tyrosine kinase inhibitors.

The present invention provides the embodiments described as follows, which may be carried out individually or in combination with the embodiments described elsewhere herein.

### Multi-omics typing

In one aspect, the present invention provides a multi-omics-based typing method for HR-positive/HER2-negative breast cancer, comprising: obtaining target multi-omics data of each patient in the target cancer patient group, the target multi-omics data comprises expression profile data, somatic copy number alteration data, and metabolome data; calculating the omics similarity network corresponding to each omics in the target multi-omics data; and clustering the omics similarity network by using the spectral clustering method, thereby obtaining the typing of the breast cancer.

In some embodiments, calculating the omics similarity network corresponding to each omics comprises normalizing the data of each omics, and calculating the squared Euclidean distance of each normalized data, then constructing the omics similarity network based on the squared Euclidean distance.

In some further embodiments, the data of each omics is normalized by using the "standardNormalization" function in the R package "SNFtools". In some further embodiments, the squared Euclidean distance of each normalized data is calculated by the "dist2" function in the R package "SNFtools". In some further embodiments, the "affinityMatrix" and "SNF" functions in the R package "SNFtools" are used to construct similarity networks. In some further embodiments, the following parameter settings are used in constructing the similarity network: K = 15, sigma = 0.5, t = 20. In some further embodiments, the "Spectrum" function (NN = 20) in the R package "Spectrum" is used to determine the optimal number of clusters.

In some further embodiments, spectral clustering is performed using similarity network fusion clustering (SNF) to obtain the typing of the breast cancer. In some further embodiments, spectral clustering is performed through the "spectralClustering" function in the R package "SNFtools" to obtain the typing of the breast cancer.

In some embodiments, the four SNF subtypes, namely classic luminal subtype, immunomodulatory subtype, proliferative subtype, and receptor tyrosine kinase-driven subtype, are obtained through the multi-omics-based typing method disclosed herein.

In one aspect, the present invention provides a subtype of HR-positive/HER2-negative breast cancer, which is obtained through the multi-omics-based typing method disclosed herein.

In the present invention, the HR-positive/HER2-negative breast cancer typing results obtained by the multi-omics-based typing method disclosed herein can serve as the gold standard for HR-positive/HER2-negative breast cancer typing. In some embodiments, the gold standard is used for the development and validation of other typing methods, for example, for developing, training, and validating the typing methods based on digital pathology images or based on gene expression disclosed herein.

The multi-omics-based typing of HR-positive/HER2-negative breast cancer disclosed herein can simultaneously reveal the biological characteristics of HR-positive/HER2-negative breast cancer and suggest subtype-specific therapeutic strategies, considering the time cost and economic cost, it is difficult to perform multi-omics analysis for every HR-positive/HER2-negative breast cancer patient in actual clinical practice. Therefore, the present invention further provides a typing method capable of clinically simple, efficient, and relatively accurate recapitulation of HR-positive/HER2-negative breast cancer patients, including a typing method based on pathology images or morphological features, a typing method based on biomarkers, and a typing method based on transcriptome/gene expression. These typing methods and the multi-omics-based typing methods both have high consistency.

### Digital pathology typing

In one aspect, the present invention provides a system and method for typing HR-positive/HER2-negative breast cancer based on digital pathology images.

In some technical solutions, the present invention provides a system for typing HR-positive/HER2-negative breast cancer based on digital pathology images, the system comprises:
1) digital pathology image (e.g., whole-slide digital pathology image) data preprocessing module, wherein the module is configured to segment the digital pathology images of tumor tissue samples of HR-positive/HER2-negative breast cancer into multiple image patches, for example, multiple enlarged image patches;
2) tissue type segmentation module, wherein the module is configured to identify the tissue type of the image patches and assign corresponding tissue labels;
3) a subtype prediction module connected in series with the tissue type segmentation module, wherein the subtype prediction module is configured to perform the typing of the sample based on the image patches with the tissue labels;
   wherein the typing refers to dividing HR-positive/HER2-negative breast cancer into the following four subtypes: classic luminal subtype, immunomodulatory subtype, proliferative subtype, and receptor tyrosine kinase-driven subtype.

In some technical solutions, the present invention provides a system for typing HR-positive/HER2-negative breast cancer, the system comprises:
1) digital pathology image (e.g., whole-slide digital pathology image) data preprocessing module, wherein the module is configured to segment the digital pathology images of tumor tissue samples of HR-positive/HER2-negative breast cancer into multiple image patches, for example, multiple enlarged image patches;
2) tissue type segmentation module, wherein the module is configured to classify the image patches into different tissue types and assign corresponding tissue labels;
3) a subtype prediction module connected in series with the tissue type segmentation module, wherein the subtype prediction module is configured to perform the typing of the sample based on the image patches with the tissue labels;
   wherein the typing refers to dividing HR-positive/HER2-negative breast cancer into the following four subtypes: classic luminal subtype, immunomodulatory subtype, proliferative subtype, and receptor tyrosine kinase-driven subtype.

In some embodiments, the present invention provides a system for typing HR-positive/HER2-negative breast cancer, the system comprises:
1) digital pathology image (e.g., whole-slide digital pathology image) data preprocessing module, wherein the module is configured to segment the digital pathology images of tumor tissue samples of HR-positive/HER2-negative breast cancer into multiple image patches, for example, multiple enlarged image patches;
2) a first neural network module, wherein the first neural network module is a tissue type segmentation neural network module, and is configured to classify the image patches into different tissue types or identify the tissue type of the image patches, and assign corresponding tissue labels;
3) a second neural network module connected in series with the first neural network module, wherein the second neural network module is a subtype prediction neural network module, and is configured to perform the typing on the samples based on the image patches with the tissue labels;
   wherein the typing refers to dividing HR-positive/HER2-negative breast cancer into the following four subtypes: classic luminal subtype, immunomodulatory subtype, proliferative subtype, and receptor tyrosine kinase-driven subtype.

In one aspect, the present invention provides a computer-implemented method for typing HR-positive/HER2-negative breast cancer, the method comprises: receiving digital pathology images of tumor tissue samples corresponding to HR-positive/HER2-negative breast cancer, and segmenting them into multiple image patches, for example, multiple enlarged image patches; applying the tissue type segmentation module to the image patches to classify the image patches into different tissue types or to identify the tissue type of the image patches, and assign corresponding tissue labels; applying the subtype prediction module to the image patches with the tissue labels to perform the typing of the sample; wherein the typing refers to dividing HR-positive/HER2-negative breast cancer into the following four subtypes: classic luminal subtype, immunomodulatory subtype, proliferative subtype, and receptor tyrosine kinase-driven subtype.

In some embodiments, the tissue type segmentation module and the subtype prediction module are each independent machine learning modules, such as deep learning modules, for example CNN, CNN trained using MIL, recurrent neural network (RNN), long short-term memory RNN (LSTM), gated recurrent unit RNN (GRU), graph convolutional network, support vector machine, and/or random forest modules.

In some embodiments, the tissue type segmentation module or the first neural network module has been generated by processing multiple training digital pathology images (e.g., whole-slide digital pathology images) to identify image patches with tissue types selected from at least one of tumor, stroma, immune infiltration, normal gland, and necrosis or hemorrhage, and to assign corresponding tissue labels. In some embodiments, the tissue type segmentation module or the first neural network module identifies image patches with the tissue type as tumor. In some embodiments, the tissue type segmentation module or the first neural network module inputs the image patches with the corresponding tissue labels into the subtype prediction module or the second neural network module, and the subtype prediction module or the second neural network module performs the typing of the sample based on the image patches with the tissue labels. In some embodiments, the subtype prediction module or the second neural network module has been generated by processing multiple training image patches (e.g., image patches with tissue labels) to type HR-positive/HER2-negative breast cancer samples based on image patches with tissue labels.

In some embodiments, the machine learning module is constructed through machine learning, such as artificial intelligence deep learning, based on digital pathology images (for example, whole-slide digital pathology images) of tumor tissue samples from patients with known HR-positive/HER2-negative breast cancer subtypes. In some embodiments, the breast cancer subtype of the patients with known HR-positive/HER2-negative breast cancer subtype is determined by the typing method described in the present invention, for example, determined by the multi-omics-based typing method of the present invention.

In some embodiments, the first neural network module and the second neural network module are constructed through machine learning, such as artificial intelligence deep learning, based on digital pathology images (for example, whole-slide digital pathology images) of tumor tissue samples from patients with known HR-positive/HER2-negative breast cancer subtypes. In some embodiments, the breast cancer subtype of the patients with known HR-positive/HER2-negative breast cancer subtype is determined by the typing method described in the present invention, for example, determined by the multi-omics-based typing method of the present invention.

According to one or more embodiments, the machine learning system can be trained in different manners. For example, the training of the machine learning system can be performed by any one or any combination of supervised training, semi-supervised training, unsupervised training classifier training, hybrid training, and/or uncertainty estimation. The type of training used may depend on the data volume, data type, and/or data quality.

The training techniques discussed in present invention can also be conducted in stages, wherein images with better annotation are initially used for training, which allows the use of images with fewer annotations and less supervision for more effective subsequent training.

Training can begin using the most comprehensively annotated images relative to all potentially usable training images. For example, training can begin using supervised learning. The first set of images can be received or determined together with the associated annotations. Each image may have annotated and/or masked regions and may include the information which, for example, identifies regions of interest and tissue types. The first set of images can be provided to a training algorithm, such as CNN, which can determine the correlation between the first set of images and their associated annotations.

After training with the first set of images is completed, a second set of images with fewer annotations than the first group (for example, with partial annotations) can be received or determined. In one embodiment, the annotation may only indicate that the image has a region of interest, but may not specify which cancer subtype it may belong to. The second set of images can be trained using a training algorithm (for example, multiple instance learning) different from that of the first group. The first set of training data can be used to partially train the system and can make the second training round more effective in generating accurate algorithms.

In this manner, training can be conducted based on the quality and type of training images, using any number of algorithms at any number of stages. These techniques can be used in cases where multiple sets of training images are received, and these sets of training images may have different quality, annotation levels, and/or annotation types.

In some embodiments, the number of digital pathology images of tumor pathological sections (such as whole-slide digital pathology images) from patients with known HR-positive/HER2-negative breast cancer subtypes is 100 or more, preferably 200 or more, 220 or more, 230 or more, 240 or more, 200-300, 220-280, 230-270, 240-260, 242-250, or 244; preferably, each digital pathology image of the tumor pathological section (for example, whole-slide digital pathology image) from patients with known HR-positive/HER2-negative breast cancer subtypes corresponds to one patient with known HR-positive/HER2-negative breast cancer subtype. Of course, the present invention can be accomplished without such one-to-one correspondence. Those skilled in the art, based on the disclosure of the present invention, can make some equivalent substitutions, such as multiple whole-slide digital pathology images corresponding to some patients.

In some embodiments, images used for training may originate from real sources (e.g., human patients) or may originate from synthetic sources (e.g., graphic rendering engines, 3D models, etc.). Example sources of digital pathology images may include: (a) digitized slides stained with various stain method (such as, but not limited to, H&E, etc.); and/or (b) digitized tissue samples from imaging devices. For example, digital pathology images may include digitized images of microscope slides containing specimens. Dye-based staining systems have been developed, including periodic acid-Schiff reaction, Masson trichrome, Nissl and methylene blue, as well as hematoxylin and eosin (H&E). For medical diagnosis, H&E is a widely used dye-based method, in which hematoxylin stains the cell nuclei blue, eosin stains the cytoplasm and extracellular matrix pink, and other tissue regions exhibit variations of these colors.

In some embodiments, the artificial intelligence deep learning employs image patches with tissue labels and their corresponding HR-positive/HER2-negative breast cancer subtypes to train and validate machine learning models (for example, convolutional neural network (CNN) models), such that the machine learning model is capable of outputting image patch prediction scores for image patches with specific tissue labels identified or segmented from digital pathology images (for example, whole-slide digital pathology images) of HR-positive/HER2-negative breast cancer patients, wherein the prediction score for each patient is the mean of the image patch prediction scores of the image patches with specific tissue labels included in the analysis for that patient; wherein the digital pathology typing of each patient is the HR-positive/HER2-negative breast cancer subtype corresponding to the highest prediction score.

In some embodiments, the image patches with specific tissue labels included in the analysis for the patient are randomly selected image patches with specific tissue labels from the patient, with a quantity of more than 100, preferably more than 200, more than 220, more than 230, more than 240, 200-300, 220-280, 230-270, 240-260, 242-258, or 250.

In some embodiments, the machine learning model (for example, convolutional neural network (CNN) model) is trained and/or validated using a three-fold cross-validation algorithm. After three-fold cross-validation, the prediction scores of the three best models were normalized and averaged to generate the prediction scores of each HR-positive/HER2-negative breast cancer subtype for each patient. The digital pathology typing of each patient is the HR-positive/HER2-negative breast cancer subtype corresponding to the highest prediction score.

In some embodiments, the artificial intelligence deep learning trains and/or validates the convolutional neural network model by employing a nonlinear classifier of an artificial intelligence support vector machine (SVM) and a one-versus-many method, using image patches with tumor tissue labels and their corresponding samples of HR-positive/HER2-negative breast cancer subtypes.

In some embodiments, convolutional neural networks are used to extract deep features, and support vector machines provide binary classification results (for example, SNF1-non-SNF1), and finally a one-versus-many method (SNF1-SNF2-SNF3-SNF4) is used to determine which of the four subtypes the patient belongs to.

In some embodiments, the tissue type segmentation neural network model is obtained by neural network modeling using the ResNet-18 network architecture.

In some embodiments, the tissue types are tumor, stroma, immune infiltration, normal gland, and necrosis or hemorrhage. In some embodiments, the tissue labels are tumor, stroma, immune infiltration, normal gland, and necrosis or hemorrhage.

In some embodiments, the tissue types are tumor, stroma, and immune infiltration. In some embodiments, the tissue labels are tumor, stroma, and immune infiltration.

In some embodiments, the tissue types are tumor and stroma. In some embodiments, the tissue labels are tumor and stroma. In some embodiments, the tissue type is tumor, and the tissue label is tumor. In some embodiments, SNF1, SNF3, and SNF4 types utilize "tumor" image patches for subtype prediction, whereas SNF2 type utilizes "stromal" image patches for subtype prediction. In some embodiments, the SNF1, SNF3, SNF2, and SNF4 types all employ "tumor" image patches for subtype prediction. In some embodiments, SNF1, SNF3, SNF2, and SNF4 types utilize only "tumor" image patches for subtype prediction.

In some embodiments, during the modeling stage, the determination threshold is established using a Youden index as a criterion by performing ROC analysis between the model output scores and the true results, and this threshold can be applied to the determination of subsequent typing.

In some embodiments, the magnified image patch is an image patch magnified 10-40 times. In some embodiments, the magnified image patch is an image patch of 256*256 pixels, 512*512 pixels, or 800*800 pixels at 20 times magnification.

In some embodiments, the number of the pathology slide images (for example, whole-slide pathology images) is more than 100.

In some embodiments, the artificial intelligence deep learning comprises training and validating a convolutional neural network model using image patches with tissue labels and their corresponding HR-positive/HER2-negative breast cancer subtypes.

In some embodiments, the artificial intelligence deep learning comprises training and validating the convolutional neural network model using a three-fold cross-validation algorithm.

In some embodiments, the artificial intelligence deep learning comprises training and validating the convolutional neural network model by employing a nonlinear classifier of an artificial intelligence support vector machine and a one-versus-many method.

In some embodiments, a tissue type segmentation neural network model is obtained by neural network modeling using the ResNet-18 network architecture.

In one aspect, the typing system described in the present invention further includes a scanning device such as a scanner, which is configured to scan pathology slide images. In one aspect, the typing system described in the present invention further comprises a microscopic device such as a microscope, which is configured to observe and/or acquire pathology slide images.

The present invention further provides a method for typing HR-positive/HER2-negative breast cancer using the above-mentioned typing system for HR-positive/HER2-negative breast cancer.

In one aspect, the present invention further provides a chip, wherein the chip comprises a processor configured to invoke and execute a computer program from a memory, such that a device equipped with the chip performs the typing method of the present invention.

The present invention further provides a computer-readable storage medium having a computer program stored thereon, and when the computer program is executed by a processor, the typing method of the present invention is implemented.

In some embodiments, the typing method of the present invention comprises: receiving digital pathology images of tumor tissue samples corresponding to HR-positive/HER2-negative breast cancer, and segmenting them into multiple image patches, for example, multiple enlarged image patches; applying the tissue type segmentation module to the image patches to classify the image patches into different tissue types or to identify the tissue type of the image patches, and assign corresponding tissue labels; applying the subtype prediction module to the image patches with the tissue labels to perform the typing of the sample; wherein the typing refers to dividing HR-positive/HER2-negative breast cancer into the following four subtypes: classic luminal subtype, immunomodulatory subtype, proliferative subtype, and receptor tyrosine kinase-driven subtype.

### Typing method based on morphological features of pathology images

In one aspect, the present invention provides an in vitro method for determining subtypes of HR-positive/HER2-negative breast cancer based on morphological features, comprising:
(a) provide pathology images of tumor samples from patients with HR-positive/HER2-negative breast cancer; and
(b) determining the morphological features of the pathology images, including:
(b1) determining the proportion of normal ductal epithelial cells to all cells in the pathology images;
(b2) determining the proportion of immune cells to all cells in the pathology images;
(b3) determining the proportion of pleomorphic tumor cells to all cells in the pathology images; and/or
(b4) determining the proportion of cancer-associated fibroblasts to all cells in the pathology images.

The higher the proportion of normal ductal epithelial cells to all cells indicates the greater the retention of normal breast ducts. In some embodiments, when the proportion of normal ductal epithelial cells is equal to or exceeds a predetermined threshold, the HR-positive/HER2-negative breast cancer is determined to be classic luminal subtype. In some embodiments, when the proportion of immune cells is equal to or exceeds a predetermined threshold, the HR-positive/HER2-negative breast cancer is determined to be immunomodulatory subtype. In some embodiments, when the proportion of pleomorphic tumor cells is equal to or exceeds a predetermined threshold, the HR-positive/HER2-negative breast cancer is determined to be proliferative subtype. In some embodiments, when the proportion of cancer-associated fibroblasts is equal to or exceeds a predetermined threshold, the HR-positive/HER2-negative breast cancer is determined to be receptor tyrosine kinase-driven subtype.

Those skilled in the art will understand that the predetermined thresholds described herein will vary depending on the assay methodology employed (e.g., manual interpretation, artificial intelligence deep learning, etc.), detection reagents such as staining reagents, detection instruments such as microscopes and scanners, and other factors. Those skilled in the art will be able to determine an appropriate predetermined threshold based on the teachings of the present invention. As used herein, the term "predetermined threshold" may refer to an average threshold calculated from a plurality of samples (abbreviated as: threshold (cut-off)), wherein the plurality of samples is obtained from a plurality of subjects, particularly subjects with known HR-positive/HER2-negative breast cancer subtypes. In order to obtain the threshold, the plurality of subjects may include subjects with tumors of different subtypes, for example, subjects with the four SNF subtypes of breast cancer as described in the present invention. In some embodiments, the predetermined threshold is an average threshold obtained by calculating the tumor samples of known HR-positive/HER2-negative breast cancer subtypes.

In some embodiments, the optimal threshold point can be selected by the Youden index. The confusion matrix corresponding to the optimal threshold point on the ROC curve is the basis for calculating indicators such as sensitivity, specificity, and accuracy. The Youden index, also known as the correctness index, refers to the sum of sensitivity and specificity minus 1: Youden index = Sensitivity + Specificity - 1. The Youden index ranges between 0 and 1, representing the overall ability of the classification model to identify true patients and non-patients. The larger the Youden index, the better the performance of the classification model.

In some embodiments, whether the proportion is equal to or exceeds a predetermined threshold is determined by manual interpretation or by a subtype prediction module described herein, such as a neural network module (for example, the subtype prediction neural network module described herein).

In some embodiments, the predetermined threshold for the proportion of normal ductal epithelial cells to all cells is 8%, 9%, 10%, 11%, 12%, 15%, or higher, for example 20%. In some embodiments, the predetermined threshold for the proportion of immune cells to all cells is 2%, 3%, or higher, for example, 4% or 5%. In some embodiments, the predetermined threshold for the proportion of pleomorphic tumor cells to all cells is 20%, 25%, or higher, for example, 30%. In some embodiments, the predetermined threshold for the proportion of cancer-associated fibroblasts to all cells is 15%, 20%, or higher, for example, 25%. In some embodiments, the predetermined threshold for the proportion of normal ductal epithelial cells to all cells is 10%, the predetermined threshold for the proportion of immune cells to all cells is 3%, the predetermined threshold for the proportion of pleomorphic tumor cells to all cells is 25%, and the predetermined threshold for the proportion of cancer-associated fibroblasts to all cells is 20%. In some embodiments, the predetermined threshold for the proportion of normal ductal epithelial cells to all cells is 8%, the predetermined threshold for the proportion of immune cells to all cells is 4%, the predetermined threshold for the proportion of pleomorphic tumor cells to all cells is 20%, and the predetermined threshold for the proportion of cancer-associated fibroblasts to all cells is 25%.

In some embodiments, the in vitro method for determining subtypes of HR-positive/HER2-negative breast cancer based on morphological features of the present invention may be performed through the typing system/method described in other parts of the present invention.

In some embodiments, the predetermined threshold or predetermined expression threshold described in the present invention may be determined by the Youden index.

### Biomarker-based typing method

In one aspect, the present invention provides a set of genes for typing HR-positive/HER2-negative breast cancer, comprising the α-SMA gene, CD8 gene, and Ki67 gene. In some embodiments, HR-positive/HER2-negative breast cancer typing includes four subtypes: classic luminal subtype, immunomodulatory subtype, proliferative subtype, and receptor tyrosine kinase-driven subtype. In some embodiments, the typing of HR-positive/HER2-negative breast cancer is based on determining the expression levels of the aforementioned genes in HR-positive/HER2-negative breast cancer samples, such as protein expression levels or RNA transcript expression levels. In some embodiments, the protein expression level can be determined by immunohistochemical staining.

In some embodiments, if the expression level of α-SMA reaches or exceeds the predetermined expression threshold T_{α-SMA} for α-SMA in HR-positive/HER2-negative breast cancer tumor samples, then the HR-positive/HER2-negative breast cancer belongs to the receptor tyrosine kinase-driven subtype; if the expression level of α-SMA is lower than the predetermined expression threshold T_{α-SMA} and the expression level of CD8 reaches or exceeds a predetermined expression threshold T_{CD8} for CD8 in an HR-positive/HER2-negative breast cancer tumor sample, then the HR-positive/HER2-negative breast cancer belongs to the immunomodulatory subtype; if the expression level of α-SMA is lower than the predetermined expression threshold T_{α-SMA}, the expression level of CD8 is lower than the predetermined expression threshold T_{CD8}, and the expression level of Ki67 reaches or exceeds the predetermined expression threshold T_{Ki67} for Ki67 in an HR-positive/HER2-negative breast cancer tumor sample, then the HR-positive/HER2-negative breast cancer belongs to the proliferative subtype; if the expression level of α-SMA is lower than the predetermined expression threshold T_{α-SMA}, the expression level of CD8 is lower than the predetermined expression threshold T_{CD8}, and the expression level of Ki67 is lower than the predetermined expression threshold T_{Ki67} in an HR-positive/HER2-negative breast cancer tumor sample, then the HR-positive/HER2-negative breast cancer belongs to the classic luminal subtype.

Those skilled in the art will understand that the predetermined expression thresholds described in the present invention will vary depending on the factors such as detection methodologies employed (for example, immunohistochemistry, PCR, chemiluminescence, etc.), detection reagents, and/or instruments. Those skilled in the art can determine an appropriate predetermined expression threshold based on the teachings of the present invention. As used herein, the term "predetermined expression threshold" may refer to an average threshold calculated from a plurality of samples (abbreviated as: threshold (cut-off)), wherein the plurality of samples is obtained from a plurality of subjects, particularly subjects suffering from cancer. In order to obtain the threshold, the plurality of subjects may include subjects with tumors of different subtypes, for example, subjects with the four SNF subtypes of breast cancer as described in the present invention. The threshold may represent the amount or concentration of RNA transcripts or, after immunohistochemical staining, the percentage of positive tumor cells among all tumor cells. In one embodiment, the threshold is given as a CT (cycle threshold) value (see below). In one embodiment, the threshold is given as the percentage of positive tumor cells among all tumor cells.

As used herein, the expression "reaching or exceeding a predetermined expression threshold" includes expression levels that are greater than or equal to the set expression threshold. The expression level of "reaching or exceeding a predetermined expression threshold" may also be referred to as "expression-positive," whereas the expression level of "below a predetermined expression threshold" may also be referred to as "expression-negative". In some embodiments, the expression threshold of α-SMA is 15%, 20%, or higher, for example, 25%. In some embodiments, the expression threshold of CD8 is 2%, 3%, or higher, for example, 4% or 5%. In some embodiments, the expression threshold of Ki67 is 20%, 25%, or higher, for example, 30%. In some embodiments, the expression threshold of α-SMA is 20%, the expression threshold of CD8 is 3%, and the expression threshold of Ki67 is 25%.

In some embodiments, if α-SMA expression is positive, then the HR-positive/HER2-negative breast cancer belongs to the receptor tyrosine kinase-driven subtype; if α-SMA expression is negative and CD8 expression is positive, then the HR-positive/HER2-negative breast cancer belongs to the immunomodulatory subtype; if α-SMA expression is negative, CD8 expression is negative, and Ki67 expression is positive, then the HR-positive/HER2-negative breast cancer belongs to the proliferative subtype; if α-SMA expression is negative, CD8 expression is negative, and Ki67 expression is negative, then the HR-positive/HER2-negative breast cancer belongs to the classic luminal subtype. Accordingly, in some embodiments, the receptor tyrosine kinase-driven subtype HR-positive/HER2-negative breast cancer is characterized by positive expression of α-SMA; the immunomodulatory subtype HR-positive/HER2-negative breast cancer is characterized by negative expression of α-SMA and positive expression of CD8; the proliferative subtype HR-positive/HER2-negative breast cancer is characterized by negative expression of α-SMA, negative expression of CD8, and positive expression of Ki67; and/or, the classic luminal subtype HR-positive/HER2-negative breast cancer is characterized by negative expression of α-SMA, negative expression of CD8, and negative expression of Ki67.

In one aspect, the present invention provides an in vitro method for determining the subtype of HR-positive/HER2-negative breast cancer tumor, comprising: (a) determining the expression level of α-SMA in the tumor sample; (b) determining the expression level of CD8 in the tumor sample; and/or (c) determining the expression level of Ki67 in the tumor sample. In some embodiments, step (a), step (b), and step (c) may be performed in any order, or simultaneously. In some further embodiments, step (a) is performed before step (b) and/or step (c). In some further embodiments, step (b) is performed before step (c).

In some embodiments, the method further comprises determining whether the expression levels of α-SMA, CD8, and/or Ki67 reach, exceed, or are below their respective predetermined expression thresholds. In some further embodiments, when the expression level of α-SMA reaches or exceeds a predetermined expression threshold T_{α-SMA} for α-SMA in the tumor sample, the HR-positive/HER2-negative breast cancer is determined to be receptor tyrosine kinase-driven subtype. In some further embodiments, when the expression level of α-SMA is below a predetermined expression threshold T_{α-SMA} and the expression level of CD8 reaches or exceeds a predetermined expression threshold T_{CD8} for CD8 in the tumor sample, the HR-positive/HER2-negative breast cancer is determined to be immunomodulatory subtype. In some further embodiments, when the expression level of α-SMA is below a predetermined expression threshold T_{α-SMA}, the expression level of CD8 is below a predetermined expression threshold T_{CD8}, and the expression level of Ki67 reaches or exceeds a predetermined expression threshold T_{Ki67} for Ki67 in the tumor sample, the HR-positive/HER2-negative breast cancer is determined to be proliferative subtype. In some further embodiments, when the expression level of α-SMA is below a predetermined expression threshold T_{α-SMA}, the expression level of CD8 is below a predetermined expression threshold T_{CD8}, and the expression level of Ki67 is below a predetermined expression threshold T_{Ki67} in the tumor sample, the HR-positive/HER2-negative breast cancer is determined to be classic luminal subtype.

In some embodiments, the in vitro method for determining the subtype of tumors in patients with HR-positive/HER2-negative breast cancer disclosed herein may be carried out by the typing system/method for HR-positive/HER2-negative breast cancer described in other parts of the present invention.

In one aspect, the present invention provides a kit useful for typing HR-positive/HER2-negative breast cancer, comprising reagents for detecting the expression levels of α-SMA, CD8, and/or Ki67. In some embodiments, reagents for detecting the expression levels of α-SMA, CD8, and/or Ki67 may be packaged separately or together in any combination.

In one aspect, the present invention provides the use of a reagent for detecting the expression levels of α-SMA, CD8, and/or Ki67 in the preparation of a diagnostic agent for typing HR-positive/HER2-negative breast cancer.

In one aspect, the present invention provides the use of α-SMA, CD8, and/or Ki67 as markers for typing HR-positive/HER2-negative breast cancer. In another aspect, the present invention provides the use of α-SMA, CD8, and/or Ki67 in the preparation of markers for typing HR-positive/HER2-negative breast cancer or in the preparation of reagents for detecting the expression levels of markers for typing HR-positive/HER2-negative breast cancer.

In one aspect, the present invention provides a method for identifying a gene useful for typing HR-positive/HER2-negative breast cancer, comprising the steps of: (a) performing mRNA expression profile difference analysis among subtypes of HR-positive/HER2-negative breast cancer to obtain candidate genes with statistically significant expression differences among subtypes. In some further embodiments, the method further comprises step (b) performing correlation analysis between mRNA expression levels and protein expression levels using mRNA expression data of the mRNA expression profile and protein expression data, so as to further screen candidate genes. In some further embodiments, the top 10, 9, 8, 7, 6, 5, 4, 3, 2, or 1 genes with the greatest expression differences among subtypes are selected as candidate genes. In some further embodiments, the top 10, 9, 8, 7, 6, 5, 4, 3, or 2 genes with the greatest expression differences among subtypes and whose mRNA expression levels are correlated with protein expression levels are selected as candidate genes.

### Typing method based on classifier gene expression

In one aspect, the present invention provides a method for classifying subtypes of HR-positive/HER2-negative breast cancer, comprising: obtaining a gene expression profile of a target test sample; and applying a machine learning classifier to the gene expression profile to classify the target test sample into one of the subtypes: classic luminal subtype (SNF1), immunomodulatory subtype (SNF2), proliferative subtype (SNF3), and receptor tyrosine kinase-driven subtype (SNF4).

In some embodiments, obtaining a gene expression profile of a target test sample includes detecting the expression levels of classifier genes. In some embodiments, the classifier genes comprise or only comprise at least one of the high expression genes listed in Table 1 or 4, for example, comprise or only comprise at least one of SNF1 high expression genes, at least one of SNF2 high expression genes, at least one of SNF3 high expression genes, and/or at least one of SNF4 high expression genes listed in Table 1 or 4. In some embodiments, the classifier genes comprise or only comprise at least two, at least 8, at least 16, at least 32, at least 48, at least 64, at least 100, at least 128, at least 150, at least 200, at least 240, at least 280, or at least 300 of high expression genes listed in Table 1 or 4, for example, comprise or only comprise at least two, at least 8, at least 16, at least 32, at least 48, at least 64, at least 88, or all of the SNF1 high expression genes, at least two, at least 8, at least 16, at least 32, at least 48, at least 64, at least 80, or all of the SNF2 high expression genes, at least two, at least 8, at least 16, at least 32, at least 48, at least 64, at least 88, or all of the SNF3 high expression genes, and/or at least two, at least 8, at least 16, at least 32, at least 48, or all of the SNF4 high expression genes listed in Table 1 or 4. In some embodiments, the classifier genes comprise or only comprise at least 10, at least 20, at least 25, at least 30, at least 35, or at least 35 of SNF1 high expression genes, at least 10, at least 20, at least 25, at least 30, at least 35, or at least 35 of SNF2 high expression genes, at least 10, at least 20, at least 25, at least 30, at least 35, or at least 35 of SNF3 high expression genes, and/or at least 10, at least 20, at least 25, at least 30, at least 35, or at least 35 of SNF4 high expression genes listed in Table 1 or 4. In some embodiments, the classifier genes comprise or only comprise at least 25 of SNF1 high expression genes, at least 25 of SNF2 high expression genes, at least 25 of SNF3 high expression genes, and/or at least 25 of SNF4 high expression genes listed in Table 1 or 4. In some embodiments, the classifier genes comprise or only comprise at least 30 of SNF1 high expression genes, at least 30 of SNF2 high expression genes, at least 30 of SNF3 high expression genes, and/or at least 30 of SNF4 high expression genes listed in Table 1 or 4. In some embodiments, the classifier genes comprise or only comprise at least 30 of SNF1 high expression genes, at least 25 of SNF2 high expression genes, at least 30 of SNF3 high expression genes, and/or at least 20 of SNF4 high expression genes listed in Table 1 or 4. In some embodiments, the classifier genes comprise or only comprise at least 30 of SNF1 high expression genes, at least 25 of SNF2 high expression genes, at least 30 of SNF3 high expression genes, and/or at least 15 of SNF4 high expression genes listed in Table 1 or 4.

In some embodiments, the classifier genes comprise or are selected from SNF1 classifier genes, SNF2 classifier genes, SNF3 classifier genes, and SNF4 classifier genes. In some cases, the SNF1 classifier genes comprise or only comprise at least two, at least 8, at least 16, at least 32, at least 48, at least 64, at least 88, or all of the SNF1 high expression genes listed in Table 1 or 4. In some cases, the SNF2 classifier genes comprise or only comprise at least two, at least 8, at least 16, at least 32, at least 48, at least 64, at least 80, or all of the SNF2 high expression genes listed in Table 1 or 4. In some cases, the SNF3 classifier genes comprise or only comprise at least two, at least 8, at least 16, at least 32, at least 48, at least 64, at least 88, or all of the SNF3 high expression genes listed in Table 1 or 4. In some cases, the SNF4 classifier genes comprise or only comprise at least two, at least 8, at least 16, at least 32, at least 48, or all of the SNF4 high expression genes listed in Table 1 or 4.

In some embodiments, the gene expression profile is based on the expression of SNF1 high expression genes, SNF2 high expression genes, SNF3 high expression genes, and SNF4 high expression genes, for example, the expression of a portion, substantially all, or all genes listed in Table 1 or 4. In some embodiments, the gene expression profile is based on at least 5%, 10%, 15%, 20%, 25%, 30%, 35%, 40%, 45%, 50%, 60%, 70%, 80%, 90%, 95%, 96%, 97%, 98%, 99%, or 100% of the SNF1 high expression genes, at least 5%, 10%, 15%, 20%, 25%, 30%, 35%, 40%, 45%, 50%, 60%, 70%, 80%, 90%, 95%, 96%, 97%, 98%, 99%, or 100% of the SNF2 high expression genes, at least 5%, 10%, 15%, 20%, 25%, 30%, 35%, 40%, 45%, 50%, 60%, 70%, 80%, 90%, 95%, 96%, 97%, 98%, 99%, or 100% of the SNF3 high expression genes, and/or at least 5%, 10%, 15%, 20%, 25%, 30%, 35%, 40%, 45%, 50%, 60%, 70%, 80%, 90%, 95%, 96%, 97%, 98%, 99%, or 100% of the SNF4 high expression genes in Table 1 or 4.

In some embodiments, the classifier genes are subtype-specific high expression genes, such as the SNF1 high expression genes, SNF2 high expression genes, SNF3 high expression genes, or SNF4 high expression genes listed in Table 1 or 4. As used herein, the term "high expression gene" refers to a gene whose expression in one SNF subtype of HR-positive/HER2-negative breast cancer is statistically significantly enhanced compared to that in other SNF subtypes. In some cases, expression is enhanced by at least 10%, at least 20%, at least 30%, at least 40%, 50%, at least 60%, at least 70%, at least 80%, 90%, at least 100%, at least 150%, at least 200%, at least 300%, at least 500%, or higher, for example 1000%, etc.

In some embodiments, the machine learning classifier has been generated by processing gene expression profile training sets of multiple patients with known HR-positive/HER2-negative breast cancer subtype to predict the SNF subtype of HR-positive/HER2-negative breast cancer. In some embodiments, the candidate SNF1 high expression gene set, SNF2 high expression gene set, SNF3 high expression gene set, and SNF4 high expression gene set useful for developing the classifier can be obtained by the following method: differential expression analysis among subtypes is performed using the transcriptome data of patients with known HR-positive/HER2-negative breast cancer subtype (for example, differential expression analysis by RNA-seq) to obtain the top m (for example, top 100, top 80, or top 50) highly expressed genes for each subtype; optionally, the candidate gene sets of each subtype are reduced by collinearity elimination (for example, removing genes with correlation coefficients higher than 0.75), thereby obtaining the candidate gene sets of each subtype. In some embodiments, m is an integer less than or equal to 500, for example, 400, 300, 200, 150, 100, 90, 80, 70, 60, 50, 40, 30, 20, or 10. In some embodiments, the candidate genes obtained through differential expression analysis among subtypes may be the same or different, for example, the top 100 highly expressed genes of the SNF1 and SNF2 subtypes and the top 80 highly expressed genes of the SNF3 subtype and the SNF3 subtype.

In some embodiments, the classifier is a random forest classifier or a support vector machine classifier. In some embodiments, the classifier may employ a generalized linear model (GLM) or a decision tree; the decision tree can be, for example, Recursive Partitioning and Regression Trees, Gradient Boosting (GBDT), eXtreme Gradient Boosting (XGBoost), LightGBM, and the like. In some embodiments, ROC analysis is used to measure the accuracy of the developed classifier.

In some embodiments, the classifier genes for SNF1 typing comprise or only comprise the top 5 to top 80 genes in predictive relevance ranking among the SNF1 genes in Table 4 or Table 5, for example, the top 80, top 75, top 70, top 65, top 60, top 55, top 50, top 45, top 40, top 35, top 30, top 25, top 20, top 15, top 10, or top 5 genes.

In some embodiments, the classifier genes for SNF2 typing comprise or only comprise the top 5 to top 80 genes in predictive relevance ranking among the SNF2 genes in Table 4 or Table 5, for example, the top 80, top 75, top 70, top 65, top 60, top 55, top 50, top 45, top 40, top 35, top 30, top 25, top 20, top 15, top 10, or top 5 genes.

In some embodiments, the classifier genes for SNF3 typing comprise or only comprise the top 5 to top 80 genes in predictive relevance ranking among the SNF3 genes in Table 4 or Table 5, for example, the top 80, top 75, top 70, top 65, top 60, top 55, top 50, top 45, top 40, top 35, top 30, top 25, top 20, top 15, top 10, or top 5 genes.

In some embodiments, the classifier genes for SNF4 typing comprise or only comprise the top 5 to top 50 genes in predictive relevance ranking among the SNF1 genes in Table 4 or Table 5, for example, the top 45, top 40, top 35, top 30, top 25, top 20, top 15, top 10, or top 5 genes.

In some embodiments, the present invention provides a method for determining whether an HR-positive/HER2-negative breast cancer tissue sample belongs to the SNF1 subtype, comprising: obtaining the gene expression profile of the SNF1 subtype classifier genes of the target test sample; and applying the machine learning classifier to the gene expression profile to determine whether the HR-positive/HER2-negative breast cancer tissue sample belongs to the SNF1 subtype. In some embodiments, the present invention provides a method for determining whether an HR-positive/HER2-negative breast cancer tissue sample belongs to the SNF2 subtype, comprising: obtaining the gene expression profile of the SNF2 subtype classifier genes of the target test sample; and applying the machine learning classifier to the gene expression profile to determine whether the HR-positive/HER2-negative breast cancer tissue sample belongs to the SNF2 subtype. In some embodiments, the present invention provides a method for determining whether an HR-positive/HER2-negative breast cancer tissue sample belongs to the SNF3 subtype, comprising: obtaining the gene expression profile of the SNF3 subtype classifier genes of the target test sample; and applying the machine learning classifier to the gene expression profile to determine whether the HR-positive/HER2-negative breast cancer tissue sample belongs to the SNF3 subtype. In some embodiments, the present invention provides a method for determining whether an HR-positive/HER2-negative breast cancer tissue sample belongs to the SNF4 subtype, comprising: obtaining the gene expression profile of the SNF4 subtype classifier genes of the target test sample; and applying the machine learning classifier to the gene expression profile to determine whether the HR-positive/HER2-negative breast cancer tissue sample belongs to the SNF4 subtype.

In another aspect, the present invention provides a method for classifying subtypes of HR-positive/HER2-negative breast cancer in a test sample, comprising: a) obtaining the gene expression profiles for each of the classic luminal subtype (SNF1), immunomodulatory subtype (SNF2), proliferative subtype (SNF3), and receptor tyrosine kinase-driven subtype (SNF4), wherein the gene expression profiles are based on the expression of a portion, substantially all, or all genes listed in Table 1 or 4; b) obtaining the gene expression profile of the test sample, wherein the gene expression profile of the test sample is based on the expression of the same genes in Table 1 or 4 used in step a); c) comparing the gene expression profile of the test sample with the gene expression profiles in step a); d) assigning to the test sample one subtype among the classic luminal subtype (SNF1), immunomodulatory subtype (SNF2), proliferative subtype (SNF3), and receptor tyrosine kinase-driven subtype (SNF4) that has the most similar gene expression profile to the test sample.

In some embodiments, 1, 2, or 3 of steps a), c), and d) may be performed by the machine learning classifier described in the present invention.

In some embodiments, the comparison in step c) is performed by comparing the expression heat maps of steps a) and b).

In some embodiments, the gene expression profiles in steps a) and b) are based on at least 5%, 10%, 15%, 20%, 25%, 30%, 35%, 40%, 45%, 50%, 60%, 70%, 80%, 90%, 95%, 96%, 97%, 98%, 99%, or 100% of the genes listed in Table 1 or 4. In some embodiments, in the gene expression profiles of steps a) and b), at least 5%, 10%, 15%, 20%, 25%, 30%, 35%, 40%, 45%, 50%, 60%, 70%, 80%, 90%, 95%, 96%, 97%, 98%, 99%, or 100% of the genes of SNF1 high expression genes, SNF2 high expression genes, SNF3 high expression genes, and SNF4 high expression genes in Table 1 or 4 are expressed, respectively. In some embodiments, the gene expression profiles in steps a) and b) are based on at least 80%, 90%, 95%, 96%, 97%, 98%, 99%, or 100% of the SNF1 high expression genes, at least 80%, 90%, 95%, 96%, 97%, 98%, 99%, or 100% of the SNF2 high expression genes, at least 80%, 90%, 95%, 96%, 97%, 98%, 99%, or 100% of the SNF3 high expression genes, and at least 80%, 90%, 95%, 96%, 97%, 98%, 99%, or 100% of the SNF4 high expression genes in Table 1 or 4. In some embodiments, the gene expression profiles in steps a) and b) are based on at least 90% of the SNF1 high expression genes, at least 90% of the SNF2 high expression genes, at least 90% of the SNF3 high expression genes, and at least 90% of the SNF4 high expression genes in Table 1 or 4. In some embodiments, the gene expression profiles in steps a) and b) are based on at least 95% of the SNF1 high expression genes, at least 95% of the SNF2 high expression genes, at least 95% of the SNF3 high expression genes, and at least 95% of the SNF4 high expression genes in Table 1 or 4. In some embodiments, the gene expression profiles in steps a) and b) are based on at least 99% of the SNF1 high expression genes, at least 99% of the SNF2 high expression genes, at least 99% of the SNF3 high expression genes, and at least 99% of the SNF4 high expression genes in Table 1 or 4.

In yet another aspect, the present invention provides a method for classifying subtypes of HR-positive/HER2-negative breast cancer in a test sample, comprising: a) obtaining gene expression profiles of a plurality of training samples with known HR-positive/HER2-negative breast cancer subtypes, wherein each of classic luminal subtype (SNF1), immunomodulatory subtype (SNF2), proliferative subtype (SNF3), and receptor tyrosine kinase-driven subtype (SNF4) is present in the plurality of breast cancer samples, and wherein the gene expression profiles of the training samples are based on the expression of a portion, substantially all, or all genes listed in Table 1 or 4; b) constructing a centroid for each HR-positive/HER2-negative breast cancer subtype in the training set using supervised or unsupervised algorithms; c) obtaining the gene expression profile of the test sample, wherein the gene expression profile of the test sample is based on the expression of the same genes in Table 1 or 4 used in step a); d) comparing the gene expression profile of the test sample with the centroid constructed in step b); e) calculating the distance from the gene expression profile of the test sample to each centroid; f) assigning one subtype among classic luminal subtype (SNF1), immunomodulatory subtype (SNF2), proliferative subtype (SNF3), and receptor tyrosine kinase-driven subtype (SNF4) to the test sample according to the nearest centroid.

In some embodiments, the gene expression profiles in steps a) and c) are based on at least 5%, 10%, 15%, 20%, 25%, 30%, 35%, 40%, 45%, 50%, 60%, 70%, 80%, 90%, 95%, 96%, 97%, 98%, 99%, or 100% of the genes listed in Table 1 or 4.

In some embodiments, the gene expression profiles in steps a) and c) are based on at least 5%, 10%, 15%, 20%, 25%, 30%, 35%, 40%, 45%, 50%, 60%, 70%, 80%, 90%, 95%, 96%, 97%, 98%, 99%, or 100% of the genes listed in Table 1 or 4. In some embodiments, in the gene expression profiles of steps a) and c), at least 5%, 10%, 15%, 20%, 25%, 30%, 35%, 40%, 45%, 50%, 60%, 70%, 80%, 90%, 95%, 96%, 97%, 98%, 99%, or 100% of the genes of SNF1 high expression genes, SNF2 high expression genes, SNF3 high expression genes, and SNF4 high expression genes in Table 1 or 4 are expressed, respectively. In some embodiments, the gene expression profiles in steps a) and c) are based on at least 5%, 10%, 15%, 20%, 25%, 30%, 35%, 40%, 45%, 50%, 60%, 70%, 80%, 90%, 95%, 96%, 97%, 98%, 99%, or 100% of the SNF1 high expression genes, SNF2 high expression genes, SNF3 high expression genes, and SNF4 high expression genes in Table 1 or 4, respectively. In some embodiments, the gene expression profiles in steps a) and c) are based on at least 80%, 90%, 95%, 96%, 97%, 98%, 99%, or 100% of the SNF1 high expression genes, at least 80%, 90%, 95%, 96%, 97%, 98%, 99%, or 100% of the SNF2 high expression genes, at least 80%, 90%, 95%, 96%, 97%, 98%, 99%, or 100% of the SNF3 high expression genes, and at least 80%, 90%, 95%, 96%, 97%, 98%, 99%, or 100% of the SNF4 high expression genes in Table 1 or 4. In some embodiments, the gene expression profiles in steps a) and c) are based on at least 90% of the SNF1 high expression genes, at least 90% of the SNF2 high expression genes, at least 90% of the SNF3 high expression genes, and at least 90% of the SNF4 high expression genes in Table 1 or 4. In some embodiments, the gene expression profiles in steps a) and c) are based on at least 95% of the SNF1 high expression genes, at least 95% of the SNF2 high expression genes, at least 95% of the SNF3 high expression genes, and at least 95% of the SNF4 high expression genes in Table 1 or 4. In some embodiments, the gene expression profiles in steps a) and c) are based on at least 99% of the SNF1 high expression genes, at least 99% of the SNF2 high expression genes, at least 99% of the SNF3 high expression genes, and at least 99% of the SNF4 high expression genes in Table 1 or 4.

In some embodiments, the SNF1 subtypes of the present invention have the following characteristics: in tumor samples, at least 5%, 10%, 15%, 20%, 25%, 30%, 35%, 40%, 45%, 50%, 60%, 70%, 80%, 90%, 95%, 96%, 97%, 98%, 99%, or 100% of the SNF1 high expression genes in Table 1 or 4 exhibit enhanced expression relative to normal breast samples.

In some embodiments, the SNF2 subtypes of the present invention have the following characteristics: in tumor samples, at least 5%, 10%, 15%, 20%, 25%, 30%, 35%, 40%, 45%, 50%, 60%, 70%, 80%, 90%, 95%, 96%, 97%, 98%, 99%, or 100% of the SNF2 high expression genes in Table 1 or 4 exhibit enhanced expression relative to normal breast samples.

In some embodiments, the SNF3 subtypes of the present invention have the following characteristics: in tumor samples, at least 5%, 10%, 15%, 20%, 25%, 30%, 35%, 40%, 45%, 50%, 60%, 70%, 80%, 90%, 95%, 96%, 97%, 98%, 99%, or 100% of the SNF3 high expression genes in Table 1 or 4 exhibit enhanced expression relative to normal breast samples.

In some embodiments, the SNF4 subtypes of the present invention have the following characteristics: in tumor samples, at least 5%, 10%, 15%, 20%, 25%, 30%, 35%, 40%, 45%, 50%, 60%, 70%, 80%, 90%, 95%, 96%, 97%, 98%, 99%, or 100% of the SNF4 high expression genes in Table 1 or 4 exhibit enhanced expression relative to normal breast samples.

In some embodiments, enhanced expression can be expression enhanced by at least 10%, at least 20%, at least 30%, at least 40%, 50%, at least 60%, at least 70%, at least 80%, 90%, at least 100%, at least 150%, at least 200%, at least 300%, at least 500%, or higher, for example 1000%, etc.

In another aspect, the present invention provides a method for determining the subtype of an HR-positive/HER2-negative breast cancer tissue sample obtained from a patient, the method comprises detecting the expression level of at least one classifier gene (for example, at least one highly expressed gene in Table 1 or 4), wherein the detection of the expression level of the classifier gene specifically identifies the HR-positive/HER2-negative breast cancer tissue sample as classic luminal subtype (SNF1), immunomodulatory subtype (SNF2), proliferative subtype (SNF3), or receptor tyrosine kinase-driven subtype (SNF4). In some embodiments, the method further comprises comparing the detected expression level of the at least one classifier gene with the expression of the at least one classifier gene in at least one sample training set, wherein the at least one sample training set comprises expression data of the at least one classifier gene from a reference HR-positive/HER2-negative breast cancer SNF1 sample, expression data of the at least one classifier gene from a reference HR-positive/HER2-negative breast cancer SNF2 sample, expression data of the at least one classifier gene from a reference HR-positive/HER2-negative breast cancer SNF3 sample, expression data of the at least one classifier gene from a reference HR-positive/HER2-negative breast cancer SNF4 sample, or combinations thereof; and classifying the sample into SNF1, SNF2, SNF3, or SNF4 subtype based on the results of the comparison step. In some cases, the comparison step includes applying a machine learning classifier or statistical algorithm, which comprises determining the correlation between expression data obtained from the sample and expression data from the at least one training set; and classifying the sample into SNF1, SNF2, SNF3, or SNF4 subtype based on the results of a machine learning classifier or statistical algorithm.

In some cases, the expression levels of classifier genes are detected at the nucleic acid level. In some cases, the nucleic acid level is RNA or cDNA. In some cases, detection of expression levels comprises performing quantitative real-time reverse transcription polymerase chain reaction (qRT-PCR), RNAseq, microarray, gene chip, nCounter gene expression assay, serial analysis of gene expression (SAGE), rapid analysis of gene expression (RAGE), nuclease protection assay, Northern blotting, or any other equivalent gene expression detection techniques. In some cases, the expression levels of the classifier genes in Table 1 or 4 are detected by performing RNA-seq. In some cases, the expression levels of the classifier genes in Table 1 or 4 are detected by performing qRT-PCR. In some cases, the detection of expression levels comprises using at least one pair of oligonucleotide primers specific to at least one classifier gene from Table 1 or 4. In some cases, the sample is a formalin-fixed paraffin-embedded (FFPE) breast cancer tumor tissue sample obtained from a patient. In some cases, the sample is a fresh or frozen tissue sample. In some cases, the at least one classifier gene comprises a plurality of classifier genes. In some cases, the plurality of classifier genes comprise at least two classifier genes, at least 8 classifier genes, at least 16 classifier genes, at least 24 classifier genes, at least 32 classifier genes, at least 40 classifier genes, or at least 48 classifier genes, at least 60 classifier genes, at least 80 classifier genes, at least 100 classifier genes, at least 120 classifier genes, at least 150 classifier genes, at least 180 classifier genes, at least 200 classifier genes, at least 240 classifier genes, at least 270 classifier genes, or at least 300 classifier genes from Table 1 or 4. In some cases, the at least one classifier gene comprises all classifier genes from Table 1 or 4.

In the present invention, the identification of HR-positive/HER2-negative breast cancer subtypes can be improved by applying algorithms designed to normalize and/or improve the reliability of gene expression data. In some embodiments of the present invention, due to a large number of individual data points to be processed, data analysis utilizes a computer or other device, machine, or apparatus to apply various algorithms described herein. "Machine learning algorithm" refers to a computation-based prediction method, which those skilled in the art also call a "classifier," used to characterize one or more gene expression profiles, for example, in order to determine HR-positive/HER2-negative breast cancer subtypes. In one embodiment, the biomarker levels determined by, for example, microarray-based hybridization assay, sequencing assay, nano-string assay, or the like are subjected to an algorithm to classify the expression profiles. Supervised learning typically involves "training" a classifier to identify the differences among SNF subtypes, and then "testing" the accuracy of the classifier on an independent test set. Therefore, for new unknown samples, the classifier can be used to predict, for example, the category to which the sample belongs (e.g., each subtype of SNF1-4).

In one embodiment, the method for analyzing biomarker level data comprises using a pre-classifier algorithm. For example, the algorithm may use specific molecular fingerprints to pre-classify samples according to their composition, and then apply correction/normalization factors. Then the data/information can be input into the final classification algorithm, which will integrate the information to assist the final typing.

In one embodiment, the method for analyzing biomarker level data further comprises using a classifier algorithm as provided herein. In one embodiment of the present invention, diagonal linear discriminant analysis, k-nearest neighbor algorithm, support vector machine (SVM) algorithm, linear support vector machine, random forest algorithm, or probability model-based methods, or combinations thereof are provided for classification of microarray data. In some embodiments, predictive markers for distinguishing sample subtypes are selected based on the statistical significance of differences in biomarker levels between categories of interest.

**Table 1. High expression genes of four SNF subtypes**

| **SNF1 high expression gene** | | **SNF2 high expression gene** | | **SNF3 high expression gene** | | **SNF4 high expression gene** | |
|---|---|---|---|---|---|---|---|
| **Gene Name** | **Entrez ID** | **Gene Name** | **Entrez ID** | **Gene Name** | **Entrez ID** | **Gene Name** | **Entrez ID** |
| LACRT | 90070 | GABRQ | 55879 | ATP12A | 479 | SPANXA1 | 30014 |
| MYBPC1 | 4604 | DCD | 117159 | FAM25A | 643161 | PGA3 | 643834 |
| MYO3B | 140469 | DKK1 | 22943 | LIN28A | 79727 | FAM205A | 259308 |
| CCDC175 | 729665 | NKX1-2 | 390010 | TCL1B | 9623 | PCDH11X | 27328 |
| SLC4A4 | 8671 | FABP7 | 2173 | CHRNA9 | 55584 | SLC15A5 | 729025 |
| SLCO1B7 | 338821 | CASP14 | 23581 | SST | 6750 | DES | 1674 |
| HCRTR2 | 3062 | UGT1A4 | 54657 | NEUROD4 | 58158 | MS4A6E | 245802 |
| DGAT2L6 | 347516 | PLA2G4E | 123745 | CACNG2 | 10369 | FGFBP1 | 9982 |
| KLHL1 | 57626 | VGLL1 | 51442 | IFITM5 | 387733 | ACTC1 | 70 |
| AC004080.3 | NA | ORM2 | 5005 | KRTAP4-1 | 85285 | SPHKAP | 80309 |
| NKX3-1 | 4824 | MAGEA4 | 4103 | CGA | 1081 | NPY2R | 4887 |
| NTNG1 | 22854 | MAGEA10 | 4109 | CDK5R2 | 8941 | CAV3 | 859 |
| TRIM72 | 493829 | MYPN | 84665 | GPR139 | 124274 | PKHD1L1 | 93035 |
| GAD1 | 2571 | LY6D | 8581 | LYZL2 | 119180 | CDH12 | 1010 |
| NEUROD1 | 4760 | FAM9A | 171482 | GDPD2 | 54857 | DLK1 | 8788 |
| PIP | 5304 | SLC15A1 | 6564 | KRT33A | 3883 | ADH4 | 127 |
| HOXB8 | 3218 | A2ML1 | 144568 | ZDHHC22 | 283576 | DSG1 | 1828 |
| EVX1 | 2128 | GLYATL2 | 219970 | SVOP | 55530 | GABRA4 | 2557 |
| POTEC | 388468 | AC233755.1 | NA | ELAVL3 | 1995 | CYP1A1 | 1543 |
| KRTAP3-3 | 85293 | AMY1A | 276 | GSTA3 | 2940 | KCNJ16 | 3773 |
| CDH18 | 1016 | AC233755.2 | NA | COL2A1 | 1280 | DPYS | 1807 |
| NLRP13 | 126204 | FCRL4 | 83417 | LRTM2 | 654429 | ADGRG4 | 139378 |
| VIPR2 | 7434 | IL21 | 59067 | SLC5A7 | 60482 | SYT4 | 6860 |
| LRRC31 | 79782 | NR2E1 | 7101 | SMIM32 | 389332 | MYOC | 4653 |
| CLDN25 | 644672 | KRT4 | 3851 | ANK1 | 286 | OPRPN | 58503 |
| DLX2 | 1746 | ZPBP2 | 124626 | PABPC1 L2 A | 340529 | OSTN | 344901 |
| LTF | 4057 | LBP | 3929 | TRH | 7200 | AC073082.1 | NA |
| RASSF10 | 644943 | AC010463.1 | NA | ZG16 | 653808 | SLC7A14 | 57709 |
| TPH1 | 7166 | STRA8 | 346673 | NDST4 | 64579 | ANGPT4 | 51378 |
| NEK10 | 152110 | AWAT2 | 158835 | VCX | 26609 | DDX3Y | 8653 |
| CFAP65 | 255101 | C1QL2 | 165257 | ZIC3 | 7547 | POTEM | 641455 |
| CACNA2D3 | 55799 | SPATA3 | 130560 | CELF3 | 11189 | SERTM1 | 400120 |
| GRIA2 | 2891 | FGG | 2266 | ASCL1 | 429 | TRIM43B | 653192 |
| WFDC5 | 149708 | SORCS3 | 22986 | CSMD3 | 114788 | ABRA | 137735 |
| ETNPPL | 64850 | PSAPL1 | 768239 | MCCD1 | 401250 | ARHGAP36 | 158763 |
| CHGB | 1114 | ACE2 | 59272 | LRRC53 | 1053788 03 | PTX3 | 5806 |
| MUC5B | 727897 | SCN1A | 6323 | DPYSL5 | 56896 | LHCGR | 3973 |
| CYP4X1 | 260293 | COL6A5 | 256076 | PTH2 | 113091 | KCNA4 | 3739 |
| PRR20E | 729250 | LHX5 | 64211 | TMEM179 | 388021 | FTMT | 94033 |
| DLX1 | 1745 | SCEL | 8796 | MYT1 | 4661 | CLCA4 | 22802 |
| SP5 | 389058 | S100A8 | 6279 | PCSK2 | 5126 | IL13RA2 | 3598 |
| RAB41 | 347517 | CTSE | 1510 | RTBDN | 83546 | ANGPTL7 | 10218 |
| NAT1 | 9 | OR56A3 | 390083 | KCNH7 | 90134 | CYP3A5 | 1577 |
| NDNF | 79625 | PRKAG3 | 53632 | SLC22A24 | 283238 | CA10 | 56934 |
| SCGB3A1 | 92304 | AKR1B15 | 441282 | SPINK13 | 153218 | SLC36A2 | 153201 |
| NEUROG2 | 63973 | SULT1C3 | 442038 | DUXB | 1000334 11 | MRGPRX2 | 117194 |
| IQCJ | 654502 | DSPP | 1834 | PRTN3 | 5657 | SHOX | 6473 |
| ZP4 | 57829 | HTR3A | 3359 | GPR37L1 | 9283 | PRL | 5617 |
| SCUBE2 | 57758 | SEMG2 | 6407 | TEX13B | 56156 | C2orf40 | NA |
| AL451007.3 | NA | ZP2 | 7783 | UGT2A1 | 10941 | SAA1 | 6288 |
| PEBP4 | 157310 | WFDC12 | 128488 | SSTR5 | 6755 | | |
| SPAG6 | 9576 | AC141272.1 | NA | GFY | 1005070 03 | | |
| PLA2G3 | 50487 | PAX7 | 5081 | CALHM1 | 255022 | | |
| NPY1R | 4886 | PLEKHG7 | 440107 | NKX2-5 | 1482 | | |
| GALNT5 | 11227 | IGSF23 | 147710 | IGDCC3 | 9543 | | |
| ZNF385B | 151126 | MSGN1 | 343930 | IP6K3 | 117283 | | |
| GABRR3 | 200959 | GSDMC | 56169 | TMPRSS9 | 360200 | | |
| KLK11 | 11012 | ERVV-1 | 147664 | AMBP | 259 | | |
| SLC28A3 | 64078 | ACOD1 | 730249 | ELFN2 | 114794 | | |
| KCNQ5 | 56479 | GNAT3 | 346562 | PRLH | 51052 | | |
| CYP4A22 | 284541 | C8B | 732 | ACTL6B | 51412 | | |
| FABP1 | 2168 | CXCL9 | 4283 | NAT8 | 9027 | | |
| CFAP157 | 286207 | ARSF | 416 | WFDC13 | 164237 | | |
| IL1RAPL1 | 11141 | XIRP1 | 165904 | CST9 | 128822 | | |
| UCP1 | 7350 | HOXD12 | 3238 | HMX3 | 340784 | | |
| SYT9 | 143425 | IGF2BP3 | 10643 | SPDYC | 387778 | | |
| ESRRG | 2104 | MUC2 | 4583 | NEURLA1 | 9148 | | |
| PYDC1 | 260434 | MAGEA6 | 4105 | MYOG | 4656 | | |
| GHRH | 2691 | KIR3DX1 | 90011 | MARCH4 | NA | | |
| DNASE2B | 58511 | IL12RB2 | 3595 | SLC8A2 | 6543 | | |
| ANKRD34C | 390616 | GABBR2 | 9568 | SLC22A9 | 114571 | | |
| ZNF648 | 127665 | ISL2 | 64843 | TH | 7054 | | |
| SHROOM1 | 134549 | IFNG | 3458 | SLC4A10 | 57282 | | |
| RTP3 | 83597 | IL22RA2 | 116379 | FCRLB | 127943 | | |
| SCGB1D2 | 10647 | S100A7A | 338324 | DHRS2 | 10202 | | |
| SERPINB10 | 5273 | OR2T34 | 127068 | TAS2R38 | 5726 | | |
| CYP2A7 | 1549 | KLHDC7B | 113730 | KRTAP10-1 | 386677 | | |
| SLC5A1 | 6523 | OLIG3 | 167826 | DSCAM | 1826 | | |
| PPP4R4 | 57718 | CLCA2 | 9635 | CABP7 | 164633 | | |
| FYB2 | 199920 | IVL | 3713 | NR0B2 | 8431 | | |
| ZBTB18 | 10472 | CP | 1356 | KCNJ18 | 1001344 44 | | |
| CLIC6 | 54102 | UGT2B10 | 7365 | KCNG4 | 93107 | | |
| HOXB6 | 3216 | | | AC092329. 3 | NA | | |
| IRX2 | 153572 | | | HS3ST6 | 64711 | | |
| CDH7 | 1005 | | | HSFX3 | 1019289 17 | | |
| ANKRD30A | 91074 | | | GDF1 | 2657 | | |
| FMO5 | 2330 | | | FAM196A | NA | | |
| WNT11 | 7481 | | | PRAME | 23532 | | |
| | | | | TEX19 | 400629 | | |

### Treatment method

In one aspect, the present invention provides a method for treating classic luminal subtype breast cancer, comprising administering endocrine therapy to a patient, for example, administering an estrogen receptor antagonist. In some embodiments, the characteristic of classic luminal subtype breast cancer is that the expression level of α-SMA in the tumor sample is lower than the predetermined expression threshold T_{α-SMA}, the expression level of CD8 is lower than the predetermined expression threshold T_{CD8}, and the expression level of Ki67 is lower than the predetermined expression threshold T_{Ki67}. In other embodiments, the characteristic of classic luminal subtype breast cancer is that the pathology images of the tumor samples have partially preserved morphology of normal breast ducts (i.e., retaining a proportion of normal breast ducts equal to or exceeding a predetermined threshold, or having a proportion of normal ductal epithelial cells in the pathology images equal to or exceeding the predetermined threshold). In other embodiments, the characteristics of classic luminal subtype breast cancer are that

in one aspect, the present invention provides a method for treating immunomodulatory subtype breast cancer, comprising administering immunotherapy to a patient, for example, administering an immune checkpoint inhibitor. In some embodiments, the immunomodulatory subtype breast cancer is characterized in that the expression level of α-SMA in the tumor sample is lower than a predetermined expression threshold T_{α-SMA}, and the expression level of CD8 reaches or exceeds a predetermined expression threshold T_{CD8}. In other embodiments, the immunomodulatory subtype breast cancer is characterized by having a proportion of immune cell infiltration in the pathology images of tumor samples exceeding a predetermined threshold.

In one aspect, the present invention provides a method for treating proliferative subtype breast cancer, comprising administering to a patient a CDK4/6 inhibitor, a PARP inhibitor, or a combination of a CDK4/6 inhibitor and a PARP inhibitor. In some embodiments, the proliferative subtype breast cancer is characterized in that the expression level of α-SMA in the tumor sample is lower than the predetermined expression threshold T_{α-SMA}, the expression level of CD8 is lower than the predetermined expression threshold T_{CD8}, and the expression level of Ki67 reaches or exceeds the predetermined expression threshold T_{Ki67} of Ki67. In other embodiments, the proliferative subtype breast cancer is characterized by having an abundance of pleomorphic tumor cells in the pathology image of the tumor sample exceeding a predetermined threshold.

In one aspect, the present invention provides a method for treating receptor tyrosine kinase-driven subtype breast cancer, comprising administering a tyrosine kinase inhibitor to a patient. In some embodiments, the receptor tyrosine kinase-driven subtype breast cancer is characterized in that the expression level of α-SMA in the tumor sample reaches or exceeds a predetermined expression threshold T_{α-SMA}. In other embodiments, the receptor tyrosine kinase-driven subtype breast cancer is characterized by having a proportion of cancer-associated fibroblasts in the pathology images of tumor samples exceeding a predetermined threshold.

In one aspect, the present invention provides a method for treating HR-positive/HER2-negative breast cancer, comprising:
(a) determining the subtype of HR-positive/HER2-negative breast cancer through the typing system and/or method of the present invention; and
(b) if the HR-positive/HER2-negative breast cancer is determined to be the classic luminal subtype, endocrine therapy is administered to the patient; if the HR-positive/HER2-negative breast cancer is determined to be immunomodulatory subtype, immunotherapy is administered to the patient; if the HR-positive/HER2-negative breast cancer is determined to be proliferative subtype, a CDK4/6 inhibitor, or a PARP inhibitor, or a combination of a CDK4/6 inhibitor and a PARP inhibitor is administered to the patient; alternatively, if the HR-positive/HER2-negative breast cancer is determined to be receptor tyrosine kinase-driven subtype, a tyrosine kinase inhibitor is administered to the patient.

In some embodiments, the immune checkpoint inhibitor is selected from inhibitors of the following immune checkpoints: PD-1, PD-L1, CTLA-4, TIGIT, NKG2A, B7-H3, VISTA, LAG3, TIM-3, 2B4, BTLA, LAIR1, CD160, and/or TGFRβ. In some embodiments, inhibition can be performed at the DNA, RNA, or protein level. In some embodiments, inhibitory nucleic acids (e.g., dsRNA, siRNA, or shRNA) can be used to inhibit the expression of inhibitory molecules. In other embodiments, the inhibitor of the inhibitory signal is a polypeptide, for example, a soluble ligand (e.g., PD-1-lg or CTLA-4Ig) or an antibody or antigen-binding fragment thereof that binds to the inhibitory molecule; for example, antibodies or fragments thereof (also referred to herein as "antibody molecules") that bind to PD-1, PD-L1, CTLA-4, TIGIT, NKG2A, B7-H3, VISTA, LAG3, TIM-3, 2B4, BTLA, LAIR1, CD160, and/or TGFRβ, or combinations thereof.

In some embodiments, the tyrosine kinase inhibitor is a receptor tyrosine kinase inhibitor. In some further embodiments, the tyrosine kinase inhibitor is selected from one or more of the tyrosine kinase inhibitors targeting the following kinases: ALK, ACVR1C, NTRK3, KIT, EGFR, PDGFR (especially PRGFRA or PDGFRB), TGFBR2, EPHB1, MET, NTRK2, TIE1, DDR2, TEK, ACVRL1, EPHA5, ROS1, FLT4, EPHB6, TYRO3, EPHA3, EPHA4, LTK, EPHA2, MERTK, CSF1R, ACVR2A, INSRR, KDR, AXL, FLT3, FGFR1, BMPR1A, ACVR1, NTRK1, and FLT1. In some embodiments, the tyrosine kinase inhibitor is selected from tyrosine kinase inhibitors targeting the kinases shown in the following table. In some embodiments, the tyrosine kinase inhibitor is selected from the drugs shown in the following table.

| Targeted kinase | Representative drugs |
|---|---|
| ALK | Alectinib, Crizotinib, Brigatinib, Lorlatinib, Ceritinib |
| Bcr-Abl | Bosutinib, dasatinib, nilotinib, ponatinib, imatinib |
| BTK | Acalabrutinib, Ibrutinib, Zarubrutinib |
| C-Met | Crizotinib, cabozantinib |
| Epidermal growth factor receptor | Erlotinib, afatinib, gefitinib, dacomitinib, osimertinib, neratinib |
| JAKs | Ruxolitinib, Baricitinib, Tofacitinib |
| PDGFR | Lenvatinib, nintedanib, ponatinib, regorafenib, imatinib |
| RET | Lenvatinib, regorafenib, sunitinib, vandetanib |
| SRC | Dasatinib, bosutinib, ponatinib |
| Vascular endothelial growth factor receptor | Axitinib, lenvatinib, regorafenib, pazopanib, nintedanib esylate, sorafenib, sunitinib |
| Epidermal growth factor receptor | Nintedanib, Erdafitinib |
| c-Kit | Pexidartinib, avapritinib |
| FLT3 | Glumetinib, sunitinib |

In some further embodiments, the tyrosine kinase inhibitor is selected from the group comprising: EGFR inhibitor, Her3 inhibitor, IGFR inhibitor, and Met inhibitor. For example, tyrosine kinase inhibitors include but are not limited to erlotinib hydrochloride and linifanib (N-[4-(3-amino-1H-indazol-4-yl)phenyl]-N'-(2-fluoro-5-methylphenyl)urea, also known as ABT869, available for purchase from Genentech). Sunitinib malate and Bosutinib (4-[(2,4-dichloro-5-methoxyphenyl)amino]-6-methoxy-7-[3-(4-methylpiperazin-1-yl)propoxy]quinoline-3-carbonitrile, also known as SKI-606, and described in U.S. Patent No. 6,780,996); Dasatinib, pazopanib, sorafenib, vandetanib (ZD6474); and imatinib or imatinib mesylate.

In some further embodiments, epidermal growth factor receptor (EGFR) inhibitors include but are not limited to erlotinib hydrochloride; gefitinib N-[4-[(3-chloro-4-fluorophenyl)amino]-7-[[(3"S")-tetrahydro-3-furanyl]oxy]-6-quinazolinyl]-4(dimethylamino)-2-butenamide); vandetanib; lapatinib (3R,4R)-4-amino-1-((4-((3-methoxyphenyl)amino)pyrrolo[2,1-f][1,2,4]triazin-5-yl)methyl)piperidin-3-ol (BMS690514); canertinib dihydrochloride (CI-1033); 6-[4-[(4-ethyl-1-piperazinyl)methyl]phenyl]-N-[(1R)-1-phenylethyl]-7H-pyrrolo[2,3-d]pyrimidin-4-amine (AEE788, CAS 497839-62-0); mubritinib (TAK165); pelitinib (EKB569); afatinib, neratinib (HKI-272); (3S)-3-morpholinylmethyl N-[4-[[1-[(3-fluorophenyl)methyl]-1H-indazol-5-yl]amino]-5-methylpyrrolo[2,1-f][1,2,4]triazin-6-yl]-carbamate (BMS599626); N-(3,4-dichloro-2-fluorophenyl)-6-methoxy-7-[[(3aα,5β,6aα)-octahydro-2-methylcyclopenta[c]pyrrol-5-yl]methoxy]-4-quinazolinamine (XL647, CAS 781613-23-8); and 4-[4-[[(1R)-1-phenylethyl]amino]-7H-pyrrolo[2,3-d]pyrimidin-6-yl]-phenol (PKI166, CAS 187724-61-4).

In some further embodiments, the EGFR antibody includes but is not limited to cetuximab; panitumumab; matuzumab (EMD-72000); nimotuzumab (hR3); zalutumumab; TheraCIM h-R3; MDX0447(CAS 339151-96-1); and ch806 (mAb-806, CAS 946414-09-1).

Other inhibitors include but are not limited to neratinib (HKI-272, (2E)-N-[4-[[3-chloro-4-[(pyridin-2-yl)methoxy]phenyl]amino]-3-cyano-7-ethoxyquinolin-6-yl]-4-(dimethylamino)but-2-enamide, and described in PCT publication WO 05/028443); lapatinib or lapatinib ditosylate (3R,4R)-4-amino-1-((4-((3-methoxyphenyl)amino)pyrrolo[2,1-f][1,2,4]triazin-5-yl)methyl)piperidin-3-ol (BMS690514); (2E)-N-[4-[(3-chloro-4-fluorophenyl)amino]-7-[[(3S)-tetrahydro-3-furanyl]oxy]-6-quinazolinyl]-4-(dimethylamino)-2-butenamide (BIBW-2992, CAS 850140-72-6); (3S)-3-morpholinylmethyl N-[4-[[1-[(3-fluorophenyl)methyl]-1H-indazol-5-yl]amino]-5-methylpyrrolo[2,1-f][1,2,4]triazine-6-yl]-carbamate (BMS 599626, CAS 714971-09-2); canertinib dihydrochloride (PD183805 or CI-1033); and N-(3,4-dichloro-2-fluorophenyl)-6-methoxy-7-[[(3aα,5β,6aα)-octahydro-2-methylcyclopenta[c]pyrrol-5-yl]methoxy]-4-quinazolinamine (XL647, CAS 781613-23-8).

In some further embodiments, the HER3 inhibitor includes but is not limited to LJM716, MM-121, AMG-888, RG7116, REGN-1400, AV-203, MP-RM-1, MM-111, and MEHD-7945A.

In some further embodiments, the MET inhibitor includes but is not limited to cabozantinib (XL184, CAS 849217-68-1); foretinib (GSK1363089, formerly known as XL880, CAS 849217-64-7); tivantinib (ARQ197, CAS 1000873-98-2); 1-(2-hydroxy-2-methylpropyl)-N-(5-(7-methoxyquinolin-4-yloxy)pyridin-2-yl)-5-methyl-3-oxo-2-phenyl-2,3-dihydro-1H-pyrazole-4-carboxamide (AMG458); crizotinib (PF-02341066); (3Z)-5-(2,3-dihydro-1H-indol-1-ylsulfonyl)-3-({3,5-dimethyl-4-[(4-methylpiperazin-1-yl)carbonyl]-1H-pyrrol-2-yl}methylene)-1,3-dihydro-2H-indol-2-one (SU11271); (3Z)-N-(3-chlorophenyl)-3-({3,5-dimethyl-4-[(4-methylpiperazin-1-yl)carbonyl]-1H-pyrrol-2-yl}methylene)-N-methyl-2-oxoindoline-5-sulfonamide (SU11274); (3Z)-N-(3-chlorophenyl)-3-{[3,5-dimethyl-4-(3-morpholin-4-ylpropyl)-1H-pyrrol-2-yl]methylene}-N-methyl-2-oxoindoline-5-sulfonamide (SU11606); 6-[difluoro[6-(1-methyl-1H-pyrazol-4-yl)-1,2,4-triazolo[4,3-b]pyridazin-3-yl]methyl]-quinoline (JNJ38877605, CAS 943540-75-8); 2-[4-[1-(quinolin-6-ylmethyl)-1H-[1,2,3]triazolo[4,5-b]pyrazin-6-yl]-1H-pyrazol-1-yl]ethanol (PF04217903, CAS 956905-27-4); N-((2R)-1,4-dioxan-2-ylmethyl)-N-methyl-N'-[3-(1-methyl-1H-pyrazol-4-yl)-5-oxo-5H-benzo[4,5]cyclohepta[1,2-b]pyridin-7-yl]sulfonamide (MK2461, CAS 917879-39-1); 6-[[6-(1-methyl-1H-pyrazol-4-yl)-1,2,4-triazolo[4,3-b]pyridazin3-yl]thio]-quinoline (SGX523, CAS 1022150-57-7); and (3Z)-5-[[(2,6-dichlorophenyl)methyl]sulfonyl]-3-[[3,5-dimethyl-4-[[(2R)-2-(1-pyrrolidinylmethyl)-1-pyrrolidinyl]carbonyl]-1H-pyrrol-2-yl]methylene]-1,3-dihydro-2H-indol-2-one (PHA665752, CAS 477575-56-7).

In some further embodiments, the IGFR inhibitor includes but is not limited to BMS-754807, XL-228, OSI-906, GSK0904529A, A-928605, AXL1717, KW-2450, MK0646, AMG479, IMCA12, MEDI-573, and BI836845. See, for example, review of Yee, JNCI, 104; 975 (2012).

In some embodiments, CDK4/6 inhibitors include but are not limited to palbociclib (CAS registry number: 571190-30-2, also known as PD-0332991, or 6-acetyl-8-cyclopentyl-5-methyl-2-{[5-(1-piperazinyl)-2-pyridyl]amino}pyrido[2,3-d]pyrimidin-7(8H)-one, ribociclib (CAS registry number: 1211441-98-3). (CAS registry number: 1211441-98-3, also known as LEE011 or 7-cyclopentyl-N,N-dimethyl-2-((5-(piperazin-1-yl)pyridin-2-yl)amino)-7H-pyrrolo[2,3-d]pyrimidine-6-carboxamide), abemaciclib (CAS registration number: 1231929-97-7), also known as LY835219 or N-[5-[(4-ethyl-1-piperazinyl)methyl]-2-pyridinyl]-5-fluoro-4-[4-fluoro-2-methyl-1-(1-methylethyl)-1H-benzimidazol-6-yl]-2-pyrimidinamine), dalpiciclib (Aerukang^{®}), trilaciclib (Cosela^{®}), or the compounds disclosed in International Application Publication No. WO 2010/020675, U.S. Patent Nos. 8,415,355 and 8,685,980 (the entire contents of which are incorporated herein by reference).

In some embodiments, the PARP inhibitor may be an inhibitor of PARP1, PARP2, PARP3, PARP4, PARP5, or PARP6. In some embodiments, the PARP inhibitor may be, for example, olaparib, rucaparib, niraparib, talazoparib, fluzoparib, pamiparib, and/or veliparib.

### Kit

A kit for practicing the method of the present invention may be further provided. The "kit" may encompass any article (e.g., packaging or container) comprising at least one reagent (e.g., antibody, nucleic acid probe, or primer, etc.) for specifically detecting the expression of the biomarker or classifier gene of the present invention. The kit can be promoted, distributed, or sold as a unit for performing the method of the present invention. Additionally, the kit may include packaging instructions describing the kit and its method of use.

In one embodiment, a kit for practicing the method of the present invention is provided. Such kits are compatible with manual and automated immunocytochemical techniques (for example, cell staining). These kits comprise at least one antibody against a biomarker or classifier gene of interest, chemicals for detecting antibodies bound to the biomarker, counterstains, and optionally a bluing agent (to facilitate identification of positively stained cells). Any chemical for detecting antigen-antibody binding can be used in the practice of the present invention.

### Examples

The following illustrates specific embodiments of the present invention through experiments.
(1) Samples, clinical data, and multi-platform integrated analysis of cancer HR⁺/HER2⁻ breast cancer patients

A large-scale multi-omics cohort of Chinese HR⁺/HER2⁻ breast cancer comprising 579 patients was established (CBCGA cohort). Among them, 512 patients have whole-exome sequencing (WES) data of primary tumor tissue and paired blood samples, 527 patients have somatic copy number alteration (SCNA) data, 569 patients have RNA sequencing data, 182 patients have mass spectrometry-based quantitative proteome data based on TMT (Tandem Mass Tag), 384 patients have metabolome data, 421 patients have digital pathology data, and 9 patients have single-cell RNA-Seq data based on 10X Genomics.

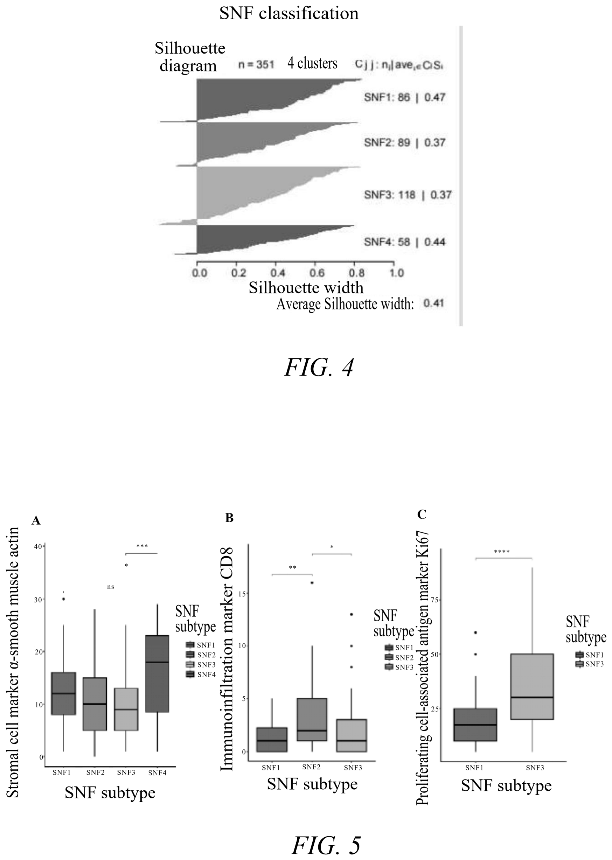

In order to study the intrinsic biology of this disease from different dimensions, similarity network fusion (SNF) was used to perform multi-omics clustering of somatic copy number alteration, mRNA, and metabolite abundance in 351 patients with overlapping datasets (referred to herein as the "CBCGA cohort of 351 samples" or the "multi-omics gold standard cohort"). The distribution of PAM50 subtypes is consistent with the literature report (Cancer Genome Atlas, N. Comprehensive molecular portraits of human breast tumors. Nature 490, 61-70 (2012)) (FIG. 2), indicating the accuracy and reliability of the multi-omics data. The eigenvalues (y-axis) of the eigenvectors (x-axis) are plotted from the Data Graph Laplacian matrix. The largest intrinsic gap is between the third and fifth eigenvectors, indicating that the optimal number of clusters for SNF clustering analysis is 4, namely SNF1-4 (FIG. 3 and FIG. 4). Thus, the SNF classifier integrating RNA expression, somatic copy number alteration, and metabolite abundance data can achieve statistical robustness and complete delineation of molecular heterogeneity in HR⁺/HER2⁻ breast cancer.

The present invention studied the different characteristics of each SNF subtype. Regarding the association between SNF subtypes and PAM50 subtypes, SNF1 is a PAM50 luminal A/B mixed subtype, whereas SNF3 mainly includes the PAM50 luminal B subtype. SNF2 comprises almost all PAM50 HER2-overexpressing subtype and basal cell-like subtype, while SNF4 contains a high proportion of PAM50 normal breast-like subtype.

SNF1 and SNF3 tumors are almost entirely composed of PAM50 luminal A/B subtypes. More than half of the tumors in SNF1 carry PIK3CA mutations (approximately 51%), whereas tumors with TP53 mutations are fewer (approximately 11%). SNF3 tumors have more CCND1/FGFR1/MDM2 copy number increases and more cell cycle pathway activation. High chromosomal instability and homologous recombination deficiency scores were also observed in SNF3 tumors, indicating high genomic instability. In addition, SNF1 and SNF3 tumors are enriched in hormone receptor pathways.

SNF2 and SNF4 tumors contain fewer PAM50 luminal A/B subtypes and less hormone receptor pathway activation. SNF2 is enriched in adaptive immune response pathways. In SNF4 tumors, expression markers related to receptor protein kinases and extracellular matrix structural components are highly enriched.

In the present invention, the typing results obtained based on the multi-omics data of 351 samples from the aforementioned CBCGA cohort are used as the SNF typing gold standard herein, which can serve as the known HR⁺/HER2⁻ breast cancer typing for training and validating the Al digital pathology typing method and the classifier gene expression-based typing method, etc. described in the present invention.

### (2) Metabolomic landscape of HR⁺/HER2⁻ breast cancer

Next, the differences in metabolic reprogramming characteristics among the four SNF subtypes were explored. First, it was found that the expression distance was significantly greater than the distance in normal samples. Euclidean distance was applied to study the overall differences in metabolic gene expression between SNF subtypes and their corresponding normal tissues. The results showed that the SNF3 subtype exhibited the highest level of metabolic dysregulation, whereas SNF4 was the subtype with the lowest metabolic dysregulation. Subsequently, network analysis was conducted to explore subtype-specific metabolic genes, polar metabolites, and lipids, and to specifically elucidate the metabolic characteristics among subtypes. The results showed that most dysregulated pathways were upregulated in the SNF3 subtype, but downregulated in SNF4. Furthermore, phospholipid metabolism was used to characterize the SNF1 and SNF2 subtypes, and genes involved in phospholipid synthesis such as PIP5K1A (East, M.P., Laitinen, T. & Asquith, C.R.M. PIP5K1A: a potential target for cancers with KRAS or TP53 mutations. Nat Rev Drug Discov 19, 436 (2020).) and PIK3CG (Semba, S. et al. Down-regulation of PIK3CG, a catalytic subunit of phosphatidylinositol 3-OH kinase, by CpG hypermethylation in human colorectal carcinoma. Clin Cancer Res 8, 3824-31 (2002)) were identified as highly expressed.

Further, a global analysis was conducted to study the association between polar metabolites and lipids and genomic events, aiming to analyze the association between genomic variations and metabolic reprogramming. The relationship between metabolic genes, genomic mutations, and copy number alterations in each subtype of the SNF cluster and the abundance of polar metabolites and lipid subclasses was studied, with a focus on 19 known cancer-related genes with high-frequency mutations in HR⁺/HER2⁻breast cancer, and a linear regression model (controlling for confounding factors) was applied to examine the relationship between somatic mutations and metabolite abundance. It was found that TP53 mutation is positively correlated with the abundance of nucleotides such as deoxyinosine and oxidized prostaglandin. Regarding copy number alteration, it was found that the copy number values of chromosomal regions containing specific breast cancer oncogenes (such as TRPS1 and ADORA1) (8q23.3 or 1q32.1) are positively or negatively correlated with variant metabolites (nucleotides or amino acids). In addition, the correlation between the expression of cell cycle-related genes and DNA synthesis-related metabolites was also observed. For example, it was observed that AURKA mRNA expression is positively correlated with deoxyinosine abundance, and CCND3 mRNA expression is negatively correlated with dUMP abundance.

### (3) Different clinical characteristics of the four SNF subtypes

Through immunohistochemical (IHC) staining analysis, it was found that SNF3 tumors have significantly higher Ki67 index and tumor grading, whereas SNF2 tumors have lower ER expression. There is no significant difference in HER2 status among the SNF subtypes. Interestingly, the distant metastasis-free survival rate and recurrence-free survival rate of SNF4 are significantly lower than those of the other three subtypes. Especially for PAM50 luminal A patients, the prognosis of SNF4 is far inferior to that of other subtypes. Univariate and multivariate Cox analyses indicate that the SNF4 subtype is an independent prognostic indicator.

### (4) Upregulation of cell cycle signaling pathway in SNF3 subtype

Application of CDK4/6 inhibitors can significantly improve the survival rate of HR⁺/HER2⁻ patients. However, there still exist many non-responders to this indicator, especially in the Asian population (Mayer, E.L. et al. Palbociclib with adjuvant endocrine therapy in early breast cancer (PALLAS): interim analysis of a multicentre, open-label, randomised, phase 3 study. Lancet Oncol 22, 212-222 (2021); Johnston, S.R.D. et al. Abemaciclib Combined With Endocrine Therapy for the Adjuvant Treatment of HR+, HER2-, Node-Positive, High-Risk, Early Breast Cancer (monarchE). J Clin Oncol 38, 3987-3998 (2020).). Genomic analysis revealed an overall increase in the frequency of copy number alteration of cell cycle genes in SNF3 samples. Then the relationship between these copy number alterations and RNA or protein expression was analyzed. An observed significant increase in the RNA and protein expression of oncogenes (such as CCND1), and a decrease in the expression of tumor suppressor genes (such as RB1), indicates cis-activation of cell cycle signaling driven by genomic variation of SNF3. Specifically, cis-effects between copy number alteration and RNA expression of CCND1 were observed in both SNF3 and other tumors, whereas cis-effects between copy number alteration and protein level were observed only in SNF3 tumors. Similarly, gene set enrichment analysis (GSEA) indicated that the cell cycle pathway was activated in SNF3, and the mRNA and protein abundance of other cell cycle genes also increased, for example, phosphorylated retinoblastoma protein (phospho-Rb) was significantly elevated.

Two regulatory factors of G2/M, namely the G2/M activator MDM2 and the G2/M inhibitor ATM, were also observed to cis-regulate cell cycle signaling in SNF3. In addition, the homologous recombination deficiency (HRD) and chromosomal instability (CIN) scores of SNF3 tumors are significantly higher.

### (5) Microenvironment landscape of HR⁺/HER2⁻ breast cancer

The tumor microenvironment (TME) participates in tumor development and influences treatment selection (Maman, S. & Witz, I.P. A history of exploring cancer in context. Nat Rev Cancer 18, 359-376 (2018)). Therefore, a comprehensive analysis of the microenvironment characteristics of the four SNF subtypes was conducted.

The tumor microenvironment characteristics of SNF2 and SNF4 tumors were further analyzed by single-cell RNA sequencing (scRNA-Seq). A total of 59,479 cells from 9 tumors were analyzed, and 11 different cell clusters were identified, including B cells, cancer-associated fibroblasts (CAF), CD4+ T cells, CD8+ T cells, ISG (interferon-stimulated) T cells, NK cells, tumor cells, endothelial cells, myeloid cells, non-tumor epithelial cells, and proliferative cells. A higher proportion of CD8+ T cells was observed in SNF2 samples, and a higher proportion of CAF was observed in SNF4 samples, which validated the deconvolution results of the bulk RNA-Seq data. In addition, the functional differences of immune cells in patients of different subtypes were studied through single-cell sequencing. Compared with non-SNF2 patients, CD8+ T cells from SNF2 patients are enriched in the interferon response pathway, indicating activation of the immune response. The cytotoxicity score, GZMK, and GNLY levels of CD8+ T cells in SNF2 patients are higher, indicating that CD8+ T lymphocytes exhibit stronger cytotoxicity in these patients.

Further, immunohistochemical experiments were conducted to verify the tumor microenvironment characteristics of the four SNF subtypes. The score of stromal cell marker α-smooth muscle actin (α-SMA) was significantly higher in SNF4; the immune infiltration marker CD8 is significantly higher in SNF2; the score of the proliferation cell-associated antigen marker Ki67 in SNF3 is higher than that in SNF1 (FIGs. 5A, 5B, 5C).

The method steps of the above immunohistochemical experiment are as follows:
the paraffin-embedded tissue sections were dewaxed at 60°C for 20 minutes, washed in xylene, and treated with a series of graded alcohols. For hematoxylin-eosin staining, the slides were stained with Mayer hematoxylin and 0.1% sodium bicarbonate, followed by counterstaining with eosin Y solution. For immunohistochemical analysis, the slides were heated in physiological saline-sodium citrate buffer at 95-100°C. After cooling, the slides were blocked at room temperature with blocking solution (a solution of 2% goat serum, 2% bovine serum albumin, and 0.05% Tween in PBS) and incubated with primary antibodies diluted in the blocking solution at 4°C. Endogenous peroxidase activity was quenched with 0.3% H₂O₂. The slide was incubated at room temperature with a horseradish peroxidase-conjugated secondary antibody, developed with 3,3'-diaminobenzidine substrate, counterstained with hematoxylin, and dehydrated with a series of graded alcohols. The positive static density is measured by a computerized imaging system, which is composed of a charge-coupled device camera connected to a microscope. The percentage of positively stained cells (0-100%) was independently assessed by two pathologists.

Based on the results of immunohistochemistry experiments, a novel HR-positive/HER2-negative breast cancer SNF typing system can be summarized, wherein the immunohistochemical scores obtained for the aforementioned four subtypes are respectively:
SNF1: α-SMA < 20%, CD8 < 3%, Ki67 < 25%, the above three conditions are simultaneously satisfied;
SNF2: α-SMA < 20%, CD8 ≥ 3%, the above two conditions are simultaneously satisfied;
SNF3: α-SMA < 20%, CD8 < 3%, Ki67 ≥ 25%, the above three conditions are simultaneously satisfied;
SNF4: α-SMA ≥ 20%.

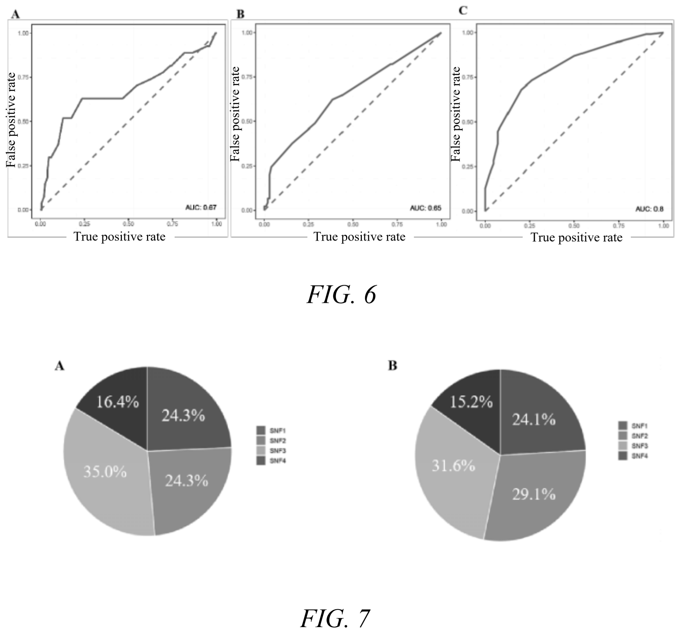

To verify the discriminative efficacy of the aforementioned markers, receiver operating characteristic (ROC) curve analyses were conducted for the three markers respectively, with results shown in FIGs. 6A, 6B, and 6C. According to the area under the ROC curve (AUC), the above typing method can be determined as an appropriate model. Additionally, the proportion of predicted SNF typing based on immunohistochemical score described above was compared with the proportion of SNF typing in the luminal subtype multi-omics cohort of the present invention, and the results are shown in FIG. 7. From the results, it can be seen that the above predicted SNF typing based on immunohistochemical score can reproduce the SNF typing of HR-positive/HER2-negative breast cancer multi-omics cohorts.

### (6) Tumor-associated fibroblasts (CAF) derived from SNF4 subtype tumors can promote tumor growth

Since patients with SNF4 subtype have the worst prognosis among HR⁺/HER2⁻ breast cancer patients, it is necessary to explore its underlying mechanism. GSEA was performed using RNA-Seq data, revealing that the receptor tyrosine kinase pathway, including several important receptor tyrosine kinases, was significantly enriched in SNF4 patients. The receptor tyrosine kinase pathway is one of the downstream pathways promoting the progression of HR⁺/HER2⁻ breast cancer (Burstein, H.J. Systemic Therapy for Estrogen Receptor-Positive, HER2-Negative Breast Cancer. N Engl J Med 383, 2557-2570 (2020)). Receptor tyrosine kinases can be highly expressed in tumor or stromal cells (Du, Z. & Lovly, C.M. Mechanisms of receptor tyrosine kinase activation in cancer. Mol Cancer 17, 58 (2018); Ostman, A. PDGF receptors in tumor stroma: Biological effects and associations with prognosis and response to treatment. Adv Drug Deliv Rev 121, 117-123 (2017).). In view of SNF4 tumors having a higher abundance of CAF, receptor tyrosine kinase may exert its function through CAF. Through comprehensive analysis of RNA-Seq, scRNA-Seq, and phosphoproteomics (CPTAC (Clinical Proteomic Tumor Analysis Consortium) breast cancer cohort), it was observed that two receptor tyrosine kinases highly expressed in SNF4 (namely EGFR and PDGFRA) were mainly expressed and highly phosphorylated in CAF. Through Western blotting, it was verified that PDGFRA and EGFR were highly expressed and highly phosphorylated in SNF4 tumor samples, especially in CAF derived from SNF4 tumors.

CAF can promote tumor growth (Gui, Y. et al. Metastatic Breast Carcinoma-Associated Fibroblasts Have Enhanced Protumorigenic Properties Related to Increased IGF2 Expression. Clin Cancer Res 25, 7229-7242 (2019); Bertero, T. et al. Tumor-Stroma Mechanics Coordinate Amino Acid Availability to Sustain Tumor Growth and Malignancy. Cell Metab 29, 124-140.e10 (2019); Jungwirth, U. et al. Impairment of a distinct cancer-associated fibroblast population limits tumor growth and metastasis. Nat Commun 12, 3516 (2021).). To determine the effect of CAF on tumor growth of HR⁺/HER2⁻ breast cancer, cancer cells were isolated from SNF1 (less invasive subtype) and SNF4 (more invasive subtype) tumors, and cultured with conditioned medium from CAF isolated from SNF4 tumors. In addition, it was observed that conditioned medium from SNF4-derived CAF can promote the proliferation of tumor cells derived from SNF1 and SNF4 tumors, indicating that CAF abundance is the main mechanism of invasiveness in the SNF4 subtype.

It has been reported that phosphorylated EGFR and PDGFRA can activate the downstream MAPK signaling pathway (Perrone, F. et al. PDGFRA, PDGFRB, EGFR, and downstream signaling activation in malignant peripheral nerve sheath tumor. Neuro Oncol 11, 725-36 (2009)). Accordingly, in the CPTAC cohort, higher phosphorylation levels of key molecules in the MAPK pathway such as RAF (RAF1) and ERK1/2 (MAPK1/3) were observed in SNF4 samples.

### (7) Establishment of an accurate typing system for HR-positive/HER2-negative breast cancer based on digital pathology and artificial intelligence

### 1) Digital pathology image collection, quality control, and preprocessing (WSI image data preprocessing module)

Pathological HE-stained sections of tumor masses from HR-positive/HER2-negative breast cancer patients treated at the Department of Pathology, Fudan University Shanghai Cancer Center (FUSCC) between 2009 and 2016 were retrieved. The sections were scanned using the Nanozoomer S210 digital section scanner, and quality control was performed on the obtained WSI. The exclusion criteria were as follows: a. section contamination, fragmentation, or severe folding; b. carcinoma in situ with micro-infiltrating sample; c. samples that have undergone neoadjuvant therapy prior to surgery. A total of 244 quality-controlled samples were ultimately included for subsequent analysis. Each sample corresponds to one WSI, thus 244 quality-controlled samples correspond to 244 WSIs.

The region of interest (ROI) containing the majority of invasive carcinoma components was delineated using the ImageScope tool. Using the openslide library in matlab software, each WSI was segmented into image patches (square-shaped) with sizes of 256*256 pixels at 20× magnification in a sliding window manner, excluding background image patches. The pixel size of each WSI differs, resulting in a different total number of image patches; however, during subsequent modeling, an equal number of image patches are extracted from each WSI to ensure that all WSIs participate in the modeling on an average basis.

### 2) Construction of tissue type segmentation neural network model (first neural network module)

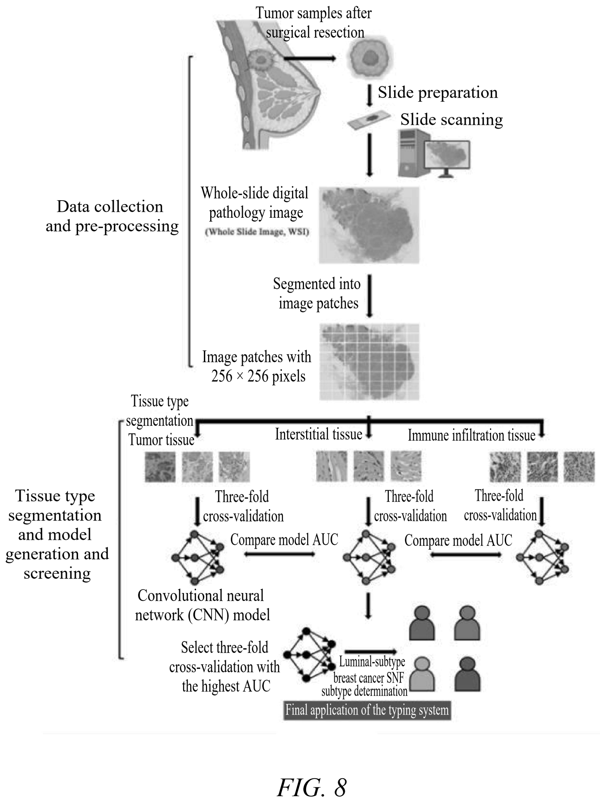

Under the guidance of pathologists, manual annotation of 20 WSIs of tissue types was completed, delineating five tissue types: tumor, stroma, lymphocytic infiltration (also known as immune infiltration), normal breast duct (also known as normal gland), and necrosis or hemorrhage. The delineated regions were segmented into image patches with classification labels, namely the annotated image patches shown in FIG. 8. These 20 WSIs do not originate from the 244 quality-controlled samples described in 1), but rather originate from an additional 20 breast cancer patients, with one WSI corresponding to each patient.

15 WSI-generated image patches were randomly selected from the above 20 WSIs for tissue type segmentation neural network model modeling. A total of 126,967 image patches generated from these 15 images were randomly divided into a training set and a validation set. Among them, the training set contains 100,134 image patches, and the validation set contains 26,833 image patches.

The ResNet-18 network architecture was selected for neural network modeling to obtain a tissue type segmentation neural network model. The hyperparameter settings are as follows: loss function, cross-entropy loss function; the size of each batch of data, 256; learning rate, 0.001; optimizer, ADAM. This modeling process can be performed as follows: manually annotated N whole-slide digital pathology images of breast cancer patients were used, 5 tissue labels are delineated thereon, namely tumor tissue label, stromal tissue label, immune infiltration tissue label, normal glandular tissue label, and necrosis or hemorrhage tissue label; the N whole-slide digital pathology images of the breast cancer patients were cut into M image patches at a size of 256*256 pixels, 512*512 pixels, or 800*800 pixels under 20× magnification, obtaining image patches with tissue labels for training and validating the tissue type segmentation neural network model, wherein N > 10, preferably N > 14, 14 < N < 21, N = 15, N = 20; M > 100000, preferably M > 120000, M > 126000, M > 126900, M > 126960, 120000 < M < 130000, M = 126967.

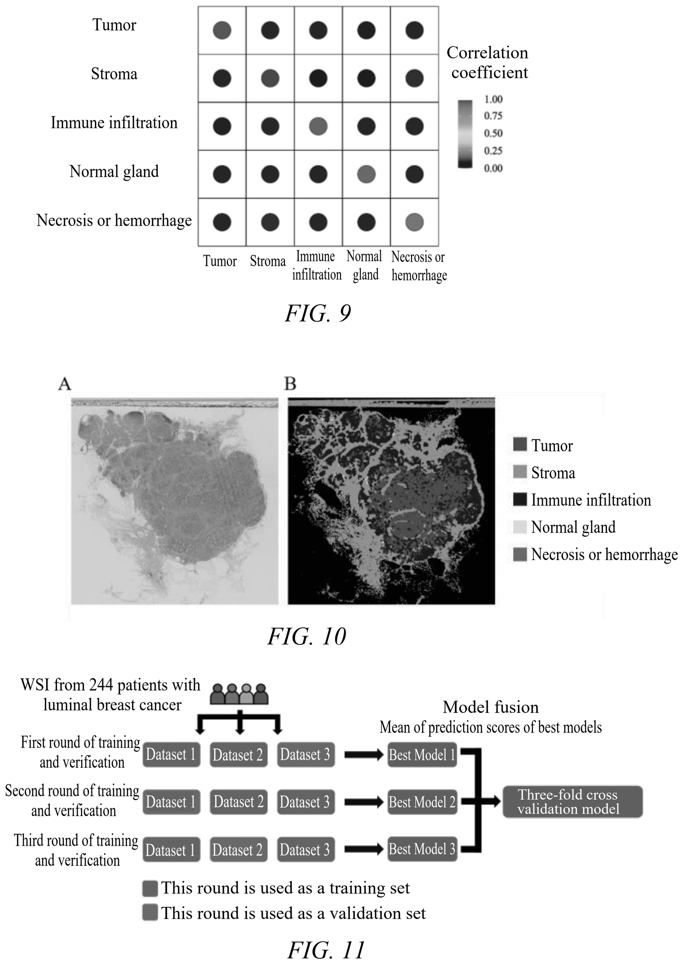

In the validation set, the discriminative performance of the tissue type segmentation neural network model for each tissue type was evaluated by confusion matrix (FIG. 9).

Integrating the prediction results of the tissue type segmentation neural network model for all image patches of the same WSI can generate a tissue type segmentation heatmap of the WSI, which, by comparison with the original WSI image, visually demonstrates the efficacy of the tissue type segmentation neural network model (FIG. 10).

Subsequently, the tissue type segmentation neural network model was applied to identify all image patches in the subsequent experiments, and image patches with tumor tissue labels, stromal tissue labels, immune infiltration tissue labels were selected for subsequent HR-positive/HER2-negative breast cancer typing analysis. The specific tissue labels in the present invention referred to tumor tissue labels, stromal tissue labels, and/or immune infiltration tissue labels.

### 3) Typing is achieved based on tissue type segmentation neural network model (second neural network module)

Using image patches with different tissue labels (such as tumor tissue, stromal tissue, immune infiltration tissue) for each patient, a three-fold cross-validation algorithm was employed to train and validate the CNN model, ultimately achieving the identification of SNF typing for each patient. Three-fold cross-validation was conducted to minimize the impact of training/validation set partitioning on the predictive performance of the model.

Specifically, the 244 WSIs, which have completed preprocessing and tissue type segmentation in the early stage as described in sections 1) and 2), were divided into three datasets, ensuring that the distribution of SNF subtypes remained consistent across the datasets. In each round, image patches from two datasets were selected for training the CNN model, and image patches from the remaining one dataset (i.e., the validation set) were used to validate the CNN model, with a total of three rounds of training conducted (FIG. 11). 500 image patches with specific labels were randomly extracted from the WSI of each patient and annotated with SNF subtypes. In the training set, image patches with specific labels and SNF annotations were input to the CNN model for deep learning. After extensive deep learning on image patches with specific labels and SNF annotations, the CNN model is capable of developing CNN models for identifying each SNF subtype using a one-to-many strategy.

During the training process, each image patch obtained a prediction score generated by the CNN model (ranging from 0 to 1, with the modeling process reproducible by setting a random seed, see Artificial intelligence for multimodal data integration in oncology, Cancer Cell 40, October 10, 2022, 1095-1110). The predicted score for each patient was defined as the mean of the scores of all image patches of the patient, and receiver operating characteristic (ROC) curve analysis was performed. The model corresponding to the maximum AUC was determined as the best model based on the area under the ROC curve (AUC) in the validation set after 50 rounds of cumulative training of the CNN model. Other hyperparameter settings are as follows: loss function, cross-entropy loss function; the size of each batch of data, 256; learning rate, 0.001; optimizer, ADAM.

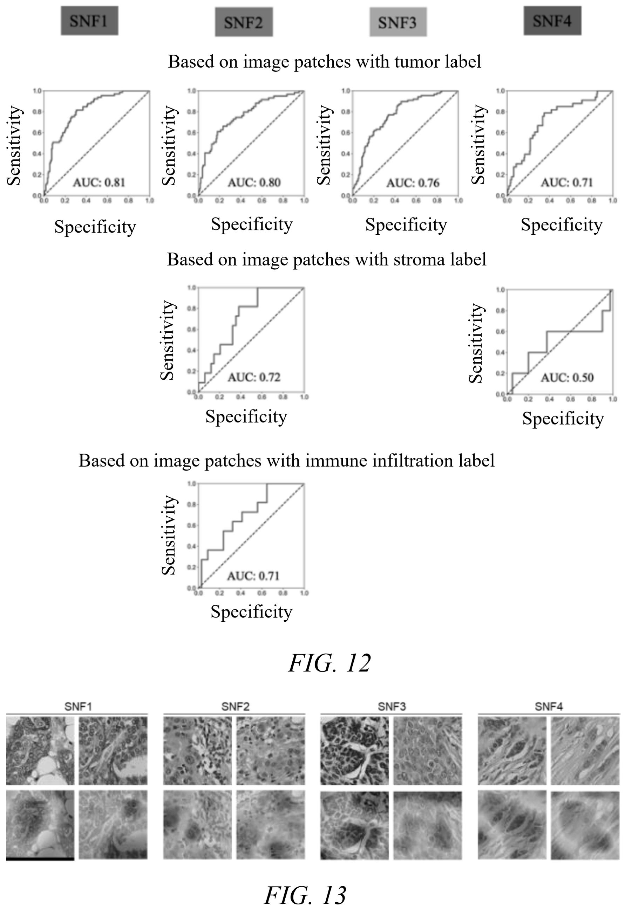

Consistent with the foregoing, three-fold cross-validation was used to divide patients into three sets on average, one dataset was selected each time as the validation set to validate the CNN model performance, three optimal models were generated through three rounds of training and validation, and the prediction scores of the three optimal models were normalized and averaged. Comparing CNN models trained based on image patches with different tissue labels, the accuracy of the prediction scores was evaluated by ROC analysis, and the CNN model with the highest AUC was selected for the discrimination of SNF subtypes (FIG. 12), ultimately generating the prediction scores of each SNF subtype for each patient (Table 2). The digital pathology typing of each patient was the SNF subtype corresponding to the highest prediction score.

Pathological characteristics of each SNF subtype were inferred by the pathological assessment CNN model (FIG. 13). For each SNF subtype, representative image patches with the highest predicted scores in patients were visually examined. The image patches of the SNF1 subtype partially retained the morphology of normal breast. Characteristic image patches of the SNF2 subtype exhibited high immune cell infiltration. Representative image patches of the SNF3 subtype exhibited densely distributed tumor cells. The SNF4 subtype image patch exhibited the invasion of tumor cells into the stroma.

The computer hardware device used for constructing the typing model through deep learning algorithms was configured as: the CPU model was I9-10900K, the memory was 32G, the hard disk was 4TB, and the graphics card was RTX-3090-24G.

The software used included Matlab R2021a (Mathworks Inc., USA), python, and the Pycharm integrated development environment. The required libraries were as follows: joblib==1.0.0, numpy==1.19.1, opencv-python==4.3.0.36, openslide-python==1.1.2, pytorch==1.9.1, sklearn==0.0, tensorboard==2.4.1, tensorboardx==2.1, torchvision==0.10.1. Their general functions were: Matlab R2021a was used to generate image patches from WSI; Pycharm was python code editor and integrated development environment; Joblib was used to enhance running speed; Numpy was python basic library; Opencv-python openslide-python was used for WSI processing and image patch invocation; Pytorch was used for deep learning modeling; Tensorboard was used to monitor and reproduce the modeling process; Sklearn was used to generate ROC curve.

Table 2 provides the pathological prediction scores for SNF1-4 subtypes in patients.

**Table 2. Pathological prediction scores for SNF1-4 subtypes in 244 patients respectively**

| Case code | SNF1 | SNF2 | SNF3 | SNF4 |
|---|---|---|---|---|
| AEUJ | -0.4765391 | -0.0695886 | 0.19830622 | 1.47150776 |
| AGNS | -0.525219 | -1.6313348 | 2.06235369 | -0.0061663 |
| AOKS | -0.6259552 | 1.37151399 | 0.3957521 | -1.5010336 |
| AYKR | -0.4906833 | 0.73089957 | -0.4242718 | 0.54461497 |
| BSMK | 0.21244299 | 0.1192041 | -1.0131742 | 1.77286737 |
| CLAV | -0.4383179 | -1.2705736 | 1.89856569 | -0.7298027 |
| COUB | -0.6302244 | 1.74049037 | -1.1181509 | -0.2352458 |
| CWNB | -0.161857 | 0.8503421 | -1.252632 | -0.0291987 |
| DIYJ | -0.3283052 | 0.36382769 | -0.3693143 | 0.4268295 |
| DWOJ | -0.572737 | 1.12247119 | 0.21788764 | 0.02897848 |
| EBSP | -0.3248237 | -0.9998514 | 0.57469194 | 0.27098736 |
| EFWC | 0.14039488 | 0.04881098 | -1.2318873 | 0.82157828 |
| EFXZ | -0.6369115 | 1.39733648 | -0.9972971 | -1.4589224 |
| EISM | -0.5527605 | 1.2018377 | -1.1543844 | 1.09721691 |
| FKGM | 0.11246128 | -0.017279 | 1.24834296 | 0.23682681 |
| FWMQ | -0.6287592 | 1.56020188 | 0.41552756 | 0.37898679 |
| FZPI | -0.241399 | 0.31279999 | 0.94961101 | 0.69818022 |
| GBNO | -0.5945818 | 0.82211164 | 2.89177619 | -0.6731525 |
| GQCV | 0.00122085 | 0.18195522 | 0.98941449 | 0.36342639 |
| GTNE | -0.6371012 | 0.08240804 | 1.76368311 | -0.2839681 |
| GYQK | -0.3183042 | -1.8889725 | -0.0684058 | 1.90433778 |
| HAGF | 1.46205645 | -1.0903271 | -0.9810086 | 0.77306843 |
| HDGE | -0.10044 | 0.63296788 | -0.4015693 | 0.25825845 |
| ICKO | -0.4752479 | -0.525266 | 0.88062096 | -0.3267107 |
| INMG | 1.29364668 | -0.4554268 | -0.9861986 | 0.34021896 |
| IOJB | -0.3622152 | -0.8729126 | -0.1737904 | 0.12036517 |
| IYVQ | -0.5082098 | 1.44057163 | -0.3217412 | -1.115048 |
| JLWB | -0.3278596 | -0.6998819 | 1.50901845 | 0.55179419 |
| JTUY | -0.6049808 | 0.92178308 | 0.48020586 | 1.09065366 |
| JXLE | -0.5903016 | 0.72731422 | -0.0048373 | -1.7496559 |
| KBPY | -0.3047384 | 1.60439044 | -2.2109697 | -2.7878339 |
| KELR | -0.4667773 | 1.34787857 | 0.09718122 | 0.83734076 |
| KJEL | 0.0703041 | -0.2102935 | -0.2280809 | -1.5854858 |
| KQIS | 3.53593155 | -0.5427612 | -1.6059454 | 1.51878667 |
| KZEF | 1.81939773 | -0.0225858 | -1.2357943 | 1.33473907 |
| LRZM | 3.65432577 | -1.5899164 | -1.1257368 | -0.2697331 |
| LWBT | 0.18576934 | -0.2287221 | -0.6563491 | -0.2836634 |
| NBKZ | 0.30205409 | -0.1112936 | 0.64543472 | -1.0373198 |
| NVIY | -0.2886775 | 0.23987359 | 0.75880928 | -0.3511403 |
| NXPA | -0.6173321 | 0.84564606 | 0.17938358 | -1.2771536 |
| ODTH | -0.449534 | 0.70363879 | -0.7773198 | -1.0472438 |
| PEYS | -0.4720184 | 0.23888043 | -0.552505 | -0.1198171 |
| PHGA | -0.6266921 | 0.21827394 | -0.2024227 | 0.85509844 |
| PHRN | -0.5611502 | -1.1151516 | 1.31376544 | 1.08443487 |
| PJGM | -0.6146139 | 0.45082402 | 0.25070291 | 0.27914623 |
| PQOW | -0.3582541 | -0.1120192 | 1.10786933 | -0.0960143 |
| PYLA | 4.47509226 | -1.2923933 | -1.5214452 | -0.0808133 |
| QLJI | -0.6006747 | -1.7429917 | 0.68460219 | 1.08899255 |
| QNAS | -0.5799081 | -0.1705365 | 0.08571749 | 0.71336108 |
| RESN | -0.5757774 | 1.32821674 | -0.1899109 | -1.8778149 |
| RFIP | -0.5566818 | 0.92773128 | 0.42604251 | -0.6820681 |
| RNCI | -0.3152282 | -0.5513061 | -0.3403116 | 0.26129155 |
| SAEI | 0.00424272 | 0.23007724 | -0.7725064 | 0.24372528 |
| SHAX | -0.6324077 | 0.80291507 | 1.13003042 | -2.5963255 |
| SHDF | -0.3579264 | -0.8330029 | 0.03218076 | 0.28480012 |
| SPTV | 1.69676543 | 0.58319093 | -0.1643044 | -0.8122764 |
| TJQM | 1.62702977 | -1.0036797 | -0.8002143 | 0.12797343 |
| TLKM | -0.2110697 | 0.53921215 | 0.37998768 | -0.0076258 |
| TOGY | -0.0558914 | 0.76765889 | -1.3722899 | -1.0904982 |
| TRCB | -0.6204585 | -1.5531244 | 0.96317824 | -0.0518538 |
| UCXS | -0.4917479 | -0.3380006 | 0.02250697 | 0.25994951 |
| UVIJ | -0.5060768 | -0.656425 | -0.769646 | 0.75659612 |
| VAMK | -0.079538 | 0.9214664 | -0.4432589 | 0.77580198 |
| VBIT | 0.13494015 | 0.08408926 | -0.4381828 | -0.1834681 |
| VJLB | -0.640022 | -0.0739124 | -0.55529 | -0.1319596 |
| VLQF | -0.4674466 | 1.08086416 | 0.16702874 | -1.979386 |
| VNGP | 1.00339984 | -0.8794778 | -0.8287702 | -0.3875443 |
| VQFJ | 1.39880437 | -0.9568689 | 0.86048611 | 1.32787871 |
| VQKJ | -0.5353118 | 1.02402726 | 0.23549936 | 0.91236493 |
| WDRH | -0.5504774 | 1.20880152 | 0.25153077 | -0.7241873 |
| WHVO | -0.6107224 | -1.2995184 | 1.70894905 | 0.89789597 |
| WPVM | -0.6225623 | 1.01214819 | -0.7032191 | -0.2322657 |
| WYSF | -0.0840034 | -2.0645629 | -0.4355859 | 0.934495 |
| XPVH | -0.4370746 | -1.6051849 | -0.1145949 | 2.09861704 |
| XQJY | -0.219938 | -0.3092395 | -0.8081362 | -1.0650178 |
| XRDZ | -0.3808106 | -2.3222766 | 2.20897216 | -0.6141783 |
| YGXJ | -0.236346 | 0.71981604 | 0.84924799 | -1.25169 |
| YIGX | -0.3430018 | 0.91539903 | 1.49969948 | -0.8120586 |
| YJDI | 2.15981061 | -2.2633861 | -1.5779673 | 0.14036887 |
| ZPWX | 1.23829418 | -0.0012534 | -1.0691421 | 0.03667825 |
| ZRXI | -0.4268601 | -0.0729953 | 0.81126352 | 1.87004824 |
| ZUCD | 0.52310037 | 0.02042613 | -1.1472647 | -0.2457406 |
| AJEH | -0.7451359 | -1.3597138 | 0.46630926 | 0.27427489 |
| BLHR | 0.38516893 | -1.4321897 | 1.38701061 | 0.25902254 |
| BLIP | 0.93628563 | -0.3845633 | -0.1423983 | 0.80826427 |
| BTJD | 0.68356954 | 1.77100468 | -1.1779273 | 1.05921527 |
| CKPD | 0.34837564 | -0.856736 | 0.90495888 | 1.28680342 |
| COUE | -1.5525928 | 0.59876174 | 0.32992086 | -1.4336663 |
| DHJT | 1.77093946 | -0.6476419 | -0.7800399 | 1.05496759 |
| DWHR | 0.79770213 | 0.54939386 | 0.33003039 | 0.3786526 |
| EVNI | -1.0815481 | 0.55592933 | 0.91432792 | 0.29938561 |
| FLZQ | -0.7553339 | 0.65486718 | 1.01516089 | -1.6310351 |
| FSDZ | 1.00857266 | -0.6538257 | 0.57213822 | 1.19826016 |
| GNTB | -0.4994909 | 0.94914159 | 0.3864989 | 0.20304647 |
| GXLH | 1.00489662 | 0.82320901 | -1.5626797 | -1.5560582 |
| HAIV | -1.7676289 | 1.3877904 | 1.41007494 | -0.8249249 |
| HRPE | 1.81279368 | -0.5753921 | -1.4962026 | -1.0446529 |
| HYCV | 0.68820819 | -0.3287687 | -1.5931726 | -0.1327863 |
| IEQH | -1.9180593 | 1.90397936 | 1.85529211 | -0.3815816 |
| IHKT | -1.0179414 | -0.5829205 | 0.34456474 | 0.75556 |
| IMOC | -1.5632293 | -0.8009757 | -0.5391807 | -0.552348 |
| IWLO | 0.81610097 | -0.325411 | 0.18818975 | -1.0591451 |
| IZVH | 0.61387238 | -0.9278967 | -1.5041119 | 1.19685242 |
| JFLK | 1.25093157 | -0.8374756 | -0.4806803 | 0.65232261 |
| JQHV | -0.4178111 | -0.8973933 | -0.3023575 | 0.84269314 |
| JYON | -0.8496024 | -0.2731921 | 0.61038981 | 0.62179467 |
| KCIF | 1.23407799 | 0.4049769 | -1.4223231 | 1.18968818 |
| KJNZ | 0.61536801 | -1.3240574 | -0.3476119 | 1.26488484 |
| KLFB | -0.433741 | -1.1053743 | 1.0948605 | 0.05540592 |
| LCUV | -1.1593298 | 1.03353693 | 0.95653544 | 0.23546582 |
| LDZQ | -0.2609668 | -0.7627854 | 2.1636707 | 0.71376478 |
| LRAX | -0.8109872 | -0.058276 | 1.01707735 | -1.3256136 |
| MIEY | 1.02123043 | 0.23351619 | -1.5664888 | -1.0053466 |
| MKAF | 1.74879332 | -0.7871722 | -1.02992 | 0.80550465 |
| MPGH | -1.1848836 | 0.73200755 | -0.4798178 | -1.2594219 |
| NIDW | 0.13397948 | -1.747857 | 0.12937658 | 1.48481536 |
| NMLV | 0.71158971 | -0.5442022 | 0.33477153 | 0.41909018 |
| NUVQ | 0.82022706 | -1.0679202 | -0.7294067 | 1.32224995 |
| ODBT | -1.9802106 | -0.0207015 | 1.27830825 | 0.87768302 |
| ODFP | -1.6173771 | -0.5954724 | 1.79503907 | -0.158726 |
| ODPJ | -1.3295055 | 0.59615678 | 0.60311156 | -0.7851722 |
| OPBQ | 1.60057828 | -0.7180338 | -1.6730754 | -0.5523864 |
| ORNF | -0.8437327 | -1.6830902 | -0.1026688 | 0.25209839 |
| OWVK | 0.48459506 | -0.9385743 | -0.8336319 | 0.9555088 |
| PANK | 0.21119809 | -0.6436702 | 0.85804307 | -0.2970459 |
| PHEJ | 0.50873488 | 0.24413438 | -0.7291146 | -0.1136171 |
| PMVK | -0.5608673 | 0.12186147 | 0.5906778 | 0.23386167 |
| PNML | 0.96583783 | 0.33242475 | 0.47645798 | -0.9509547 |
| QKAY | -0.5279805 | 0.62321221 | 0.89721551 | -0.535923 |
| QLHP | 0.54704139 | -1.2878443 | 1.92597369 | -2.0559553 |
| QNVB | 0.14162845 | -0.201931 | 0.28706475 | -0.7147493 |
| QTNC | 0.65988349 | 2.10802007 | -0.0234788 | 0.27997697 |
| QXTH | -0.5495529 | 1.8446484 | -1.0361244 | -0.1165141 |
| RANB | -0.5832575 | -0.5659063 | 0.18992393 | 1.0529534 |
| SDJA | -0.4844049 | -0.8612161 | 1.4338499 | 0.48039606 |
| SMFR | 0.90253519 | -0.8034342 | -0.2996192 | 1.21284933 |
| SZQL | -0.8229683 | 1.76585835 | 0.43638736 | -0.7629393 |
| TBSH | 0.76616327 | 0.18877919 | -1.8099253 | 0.8134245 |
| TMYF | 0.21592894 | -1.0997355 | -0.6306304 | 0.67742056 |
| TVMK | -0.5130106 | 0.39296564 | -0.6623913 | 0.3065978 |
| UKIA | 1.31962245 | -0.0236389 | -0.6494333 | 0.94745054 |
| UKMR | -1.706493 | 0.85027055 | 1.69219772 | -0.0721212 |
| USQH | 1.26701692 | -1.0676952 | -0.9866606 | 1.25944711 |
| VGIE | 0.63149305 | 1.84306187 | -1.0213613 | -1.9359003 |
| VGTE | -1.16892 | 0.14343807 | -1.2915121 | 0.14638754 |
| VHFI | -1.1863314 | -1.007165 | -0.3031369 | 0.46197683 |
| VHXK | -1.7123557 | 0.89539684 | 1.50899516 | -0.8165835 |
| WDYR | 1.63813836 | 0.35750246 | -1.6172154 | -1.3742599 |
| WKNS | 0.65177476 | 1.92697392 | -0.2669384 | -1.8870702 |
| WUVL | 0.69543705 | 0.42645271 | 0.22771733 | -0.2882066 |
| WVAK | 0.40959853 | 0.80077723 | 0.61990311 | 0.73064971 |
| WYJE | 0.19538715 | -0.7300254 | -0.4518734 | 1.39060613 |
| XFWO | -1.4774494 | 1.53143973 | -0.0694367 | -2.0715653 |
| XIDN | -0.2814169 | 1.3191592 | 0.41433852 | -1.5127279 |
| XJYK | -0.143976 | 1.43215837 | 0.06616734 | -2.1848029 |
| XOBF | 0.28531379 | -0.4878259 | -0.1478669 | 0.22027124 |
| XSJM | 0.87215257 | -0.8328887 | -1.1095249 | 1.43341307 |
| XYIN | 0.56753719 | -0.3671386 | -0.6022099 | -1.2365817 |
| YFGZ | -0.5056162 | -0.6290846 | 0.58760185 | 0.08144739 |
| YGNX | -0.6312602 | -0.4828241 | 0.78754564 | 0.81271357 |
| ZIAO | 1.25554438 | -0.7580066 | -1.3932509 | 1.26777514 |
| ZLPB | -1.0179903 | 0.81668214 | 0.88547373 | -0.3342623 |
| ZROW | 0.76960261 | 2.59491364 | -1.6811584 | -1.6598408 |
| ZXLK | -0.3024676 | -0.6667595 | 0.57340553 | 0.34759231 |
| ACWQ | 0.98630027 | -0.7328971 | 1.27029469 | 1.76451362 |
| AEXF | -0.0793219 | 1.62450645 | -0.6563535 | -1.3735963 |
| BFJP | 0.95447061 | -0.7296741 | -0.4743427 | 1.12930621 |
| BMRK | -1.1556793 | 0.35953455 | 1.14763641 | -0.9571985 |
| BTWO | -0.6246753 | -0.6553005 | -0.0738531 | -1.1675956 |
| COSG | -0.6232809 | -0.3873281 | 0.19296302 | -0.853733 |
| CSTL | 0.55782757 | -1.2191839 | -0.1958419 | -0.5486666 |
| DIAX | 1.64178796 | -0.7868106 | -0.9191321 | 1.89456915 |
| DICU | 1.16104704 | 0.13136137 | -0.6433183 | -0.9290338 |
| DZPE | 0.06090906 | -0.7067192 | 0.99868459 | 0.97332152 |
| FENY | -0.6042457 | -0.8578808 | 0.24348485 | 0.22098982 |
| FNIX | 0.92854468 | 0.6298643 | -1.2759396 | -0.7590777 |
| FQCZ | -1.0480247 | -0.4066514 | 1.02355952 | 0.01172748 |
| GBEX | -0.1195768 | 1.077337 | 0.0179255 | -0.7258616 |
| GSPW | 1.11983253 | -0.8406131 | -0.5101004 | 1.45728232 |
| HOSW | 1.12050969 | -0.9167788 | -1.1420356 | 0.45382506 |
| ILFV | -0.5447801 | -0.2837389 | 0.74013928 | 1.20218458 |
| ITBW | -1.8877481 | 0.35665744 | 0.33980863 | -1.2814037 |
| JFAQ | -0.949821 | 2.48785867 | -0.6456986 | -1.5530428 |
| JLDQ | -1.2827269 | 1.3249382 | 1.55172044 | 0.01748075 |
| KBGM | 0.84875565 | 0.88413531 | -0.3976072 | -0.3680065 |
| LASO | 1.01571588 | -0.7195552 | -0.0939689 | 2.25076261 |
| LTQA | 0.5729284 | -1.1768277 | 0.37297217 | 0.6783492 |
| LXRO | 0.30547901 | -0.7706728 | -1.4058745 | -1.4856605 |
| MNDC | -0.1623637 | 1.27937693 | -0.8674427 | 0.35456595 |
| MNXT | 0.12956615 | 0.70375794 | -1.6722837 | -0.8364534 |
| MTQA | 1.1126045 | 0.01872398 | 0.10586761 | 0.82848744 |
| MVRN | -0.4877226 | 0.98057153 | 0.30304074 | -1.0814347 |
| MWDY | 0.33142928 | -1.1346844 | -0.2896051 | -0.7749594 |
| NADU | -0.0751484 | -1.2124439 | 0.67848769 | -0.292635 |
| NBPH | 1.48011622 | -0.449247 | -1.613744 | -1.0601302 |
| NREC | -1.3302666 | 1.62447258 | -1.2714421 | -0.844317 |
| NVIW | -0.3246691 | 0.44342139 | 0.92516084 | -0.824417 |
| NXYB | 0.33734327 | -0.1937569 | -0.2957903 | 1.10553494 |
| OCLQ | 0.37331377 | 0.50580398 | 0.48843153 | 1.03612674 |
| ODEJ | -0.4584675 | -0.6387318 | 1.00297524 | 0.34504199 |
| OPYS | -0.2023195 | -1.2996749 | -0.6013182 | 2.36948082 |
| PBLW | -2.0319967 | 1.52809713 | 1.12703417 | -1.6260585 |
| PGDY | 1.41056936 | 0.8312559 | -1.7824577 | -1.5685215 |
| PIJK | 0.36399083 | -0.2876306 | -0.2534742 | 0.34474177 |
| PISZ | -0.8590514 | -0.4795041 | 0.43767283 | -0.2087001 |
| POKT | -1.0546903 | -0.1652438 | 0.72513426 | 0.00798293 |
| PTLW | 0.79139237 | 2.52833575 | -1.3558831 | -0.3015997 |
| PVOG | 0.83365031 | -0.475494 | -0.9817185 | 0.56049043 |
| PVYR | -0.4404815 | -0.3023829 | 0.52273735 | -0.6709966 |
| QAGJ | 0.19134643 | -0.4799982 | -0.296785 | 1.04801197 |
| QLHW | -1.5177062 | 0.00441067 | 1.31346681 | 1.50934834 |
| QNXH | -0.16717 | 0.43929593 | -0.9051677 | -0.7146469 |
| QPZH | 1.432975 | -0.470695 | -1.5622337 | -1.4483082 |
| QUMP | -1.1864399 | -0.82394 | 1.74782942 | -0.3603417 |
| QWJP | -0.8774191 | -0.6651406 | 1.24782291 | 0.87680132 |
| TMAR | -0.1806853 | -0.0987802 | 1.33059295 | -0.4103612 |
| TOCM | -0.6590945 | 2.36154015 | -0.637237 | -0.5988138 |
| TOYN | 0.78840389 | 0.24696643 | 0.79192616 | -1.1913014 |
| TSWP | 1.67453646 | -1.006217 | -1.0395462 | 0.49154067 |
| TXRL | 1.48896266 | -0.9433819 | -1.5417043 | 1.3957068 |
| UBSN | -0.0102419 | 0.88429164 | -0.2075083 | 0.84142134 |
| UDMQ | -1.0067405 | -0.9130691 | 0.78198489 | -0.1501556 |
| UONM | 0.99682397 | -0.61643 | 1.58010247 | -0.6512228 |
| UPLH | 0.99611908 | -1.0635222 | 0.54420279 | -0.7746487 |
| USVE | -0.3255066 | 1.30278089 | 0.61376147 | -0.4974968 |
| UWIR | -1.0706215 | 0.4683186 | 0.44194841 | 0.67556811 |
| VEBY | 0.97424825 | -1.0004504 | -0.0320589 | 0.03848689 |
| VPHD | -0.084803 | -0.7614422 | 2.12408226 | 1.36539899 |
| VQMZ | -1.7743813 | 0.64169149 | -0.453123 | -1.3688234 |
| VSNM | -1.8741149 | 1.2789644 | 0.17238973 | -0.9907748 |
| VWTH | -1.2957447 | -0.4283718 | 1.60861491 | 0.37050531 |
| WAJL | 0.9007206 | -1.1960741 | -1.0455425 | -1.0752109 |
| WEPS | -1.8036126 | -0.8667206 | 0.9144315 | 1.88106526 |
| WFGA | -0.2562591 | -1.1463048 | 1.19707894 | 0.62647743 |
| WRNK | -0.0336756 | 0.97558239 | 0.53480552 | -0.1161141 |
| XKIR | -1.1285189 | -0.6168111 | 1.76273594 | 0.041937 |
| XMIT | -1.4113071 | -0.1281043 | -1.0047852 | 0.39344681 |
| XULO | -0.3201372 | 2.95133284 | -1.5102669 | -0.0157752 |
| YENU | 1.64881852 | 1.6614031 | -1.3830836 | -0.4491305 |
| YPXV | 1.0532847 | -0.564587 | -1.1512002 | -0.3191267 |
| ZCHX | 1.30242843 | 0.21223392 | -0.6949595 | 0.06993262 |
| ZPNU | -0.7240537 | -0.7377767 | 1.33079384 | 0.28484111 |
| ZVCO | 1.42311622 | -0.7862204 | -1.3768114 | 2.01524794 |
| ZYTB | 0.74542296 | 0.39464123 | 0.00693666 | 0.29284903 |

### 4) Digital pathology typing was performed in the validation cohort

In the validation cohort of 166 HR-positive/HER2-negative breast cancer patients at Fudan University Shanghai Cancer Center, it was demonstrated that the present digital pathology typing model can reproduce the key features of multi-omics typing.

Paraffin-embedded postoperative tissue sections were obtained from 166 HR-positive/HER2-negative breast cancer patients treated at Fudan University Shanghai Cancer Center between 2009 and 2016, independent of the aforementioned cohort (244 cases). Genomic data were obtained by high-throughput sequencing for all 166 HR-positive/HER2-negative breast cancer patients.

The region of interest (ROI) containing the majority of invasive carcinoma components was delineated using the ImageScope tool. Using the openslide library in matlab software, each WSI was segmented into image patches with sizes of 256*256 pixels in a sliding window manner (square-shaped), excluding background image patches.

The predictive score of each subtype was calculated in these 166 new cases by three-fold cross-validation. These prediction scores indicated the likelihood that the sample corresponds to the respective SNF subtype. Thus, each patient respectively obtained four final predicted scores for the four SNF subtypes, among which the SNF subtype corresponding to the highest score was the inferred SNF subtype of the patient.

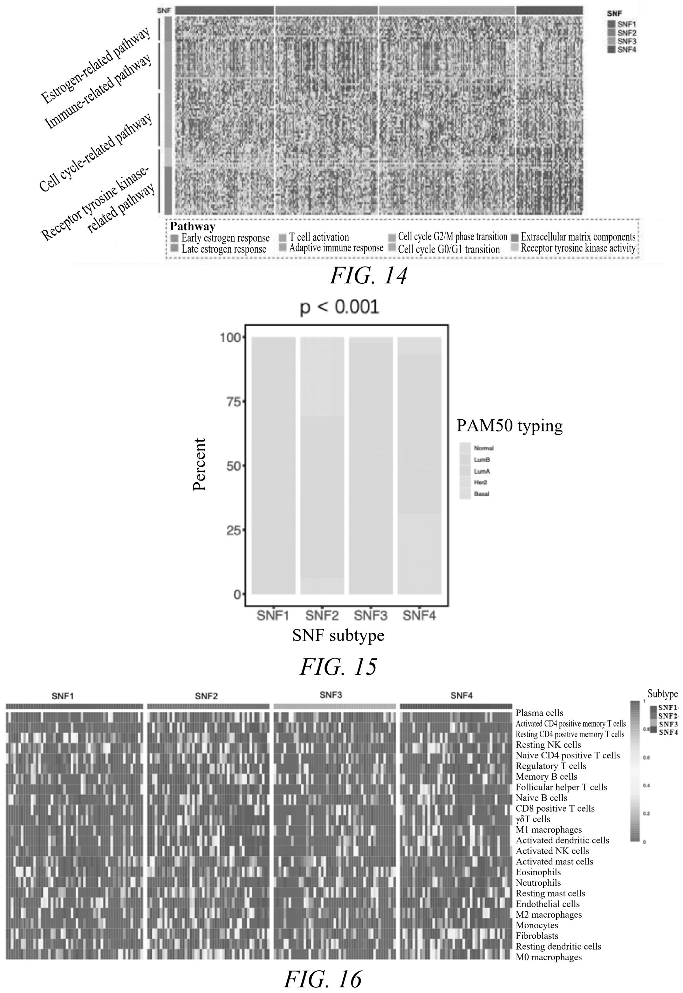

Fresh tumor tissues were previously collected postoperatively from 244 patients with HR-positive/HER2-negative breast cancer, RNA was extracted and subjected to next-generation sequencing, thereby obtaining the patients' transcriptome data (reflecting the expression quantity of each gene), and pathway enrichment analysis was performed using the transcriptome data, ultimately obtaining enrichment scores for each pathway per single sample. Subsequently, the obtained digital pathology subtype was validated for its biological characteristics at the transcriptome level, with results shown in FIG. 14. Each row on the left side of the heatmap in FIG. 14 represents a pathway, arranged in the order of SNF1/SNF2/SNF3/SNF4 characteristic pathways. Each column represents the enrichment analysis results of each pathway for a sample. The color intensity of each cell depends on the enrichment score. The results indicated that digital pathology typing could reproduce the key transcriptomic features of the four subtypes of HR-positive/HER2-negative breast cancer. For example, patients of the SNF1 subtype (blue) exhibited higher expression of estrogen-related pathways. SNF2 patients (green) exhibited highly activated immune pathways represented by adaptive immunity. Patients of the SNF3 subtype (yellow) exhibited high expression of the cell cycle pathway. The receptor tyrosine kinase pathway in SNF4 patients was upregulated higher than in other subtypes.

The correspondence between digital pathology SNF typing and PAM50 typing was consistent with the typing results based on multi-omics sequencing, namely, SNF1 type was mainly PAM50 luminal A/B, most PAM50 basal cell-like breast cancer and HER2-overexpressing breast cancer belonged to SNF2, and almost all normal breast types belonged to SNF4 (FIG. 15).

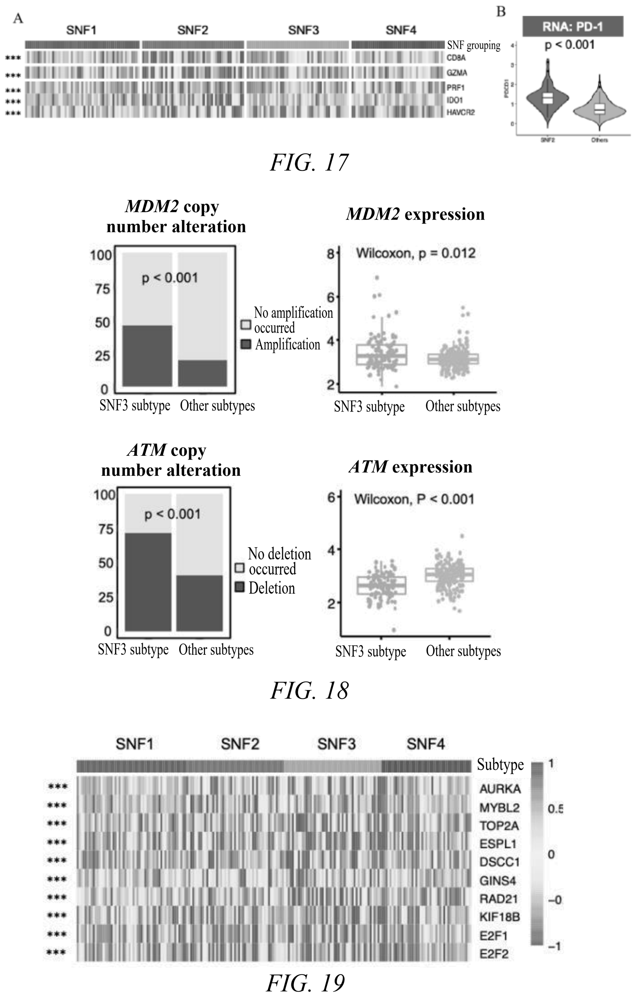

Typing studies based on multi-omics sequencing have found that tumors of SNF2 patients exhibited a highly activated immune microenvironment accompanied by high expression of immune checkpoint molecules. FIG. 16 analyzes the microenvironment of digital pathology SNF2 patients through transcriptome analysis, revealing that T cells and other immune activations in these patients were in an activated state. FIG. 13 shows a typical pathology section of a digital pathology SNF2 patient, whose tumor contained infiltrating lymphocytes such as CD8-positive T cells and activated NK cells (cells with smaller nuclei (dark blue) in the SNF2 image patch of FIG. 13). In FIG. 17, immune checkpoint molecules such as PD-1 and IDO1 are highly expressed in digital pathology SNF2, and simultaneously CD8A and GZMA are also highly expressed in SNF2, which is consistent with the previously identified characteristics of SNF2.

Using the Oncoscan kit, the DNA copy number alteration of each gene in the previously collected tumor tissue was determined, thereby detecting the amplification and deletion of each gene. As shown in FIG. 18, ATM deletion and MDM2 amplification in SNF3 are significantly more frequent than in other subtypes, which is consistent with the previously identified characteristics of SNF3.

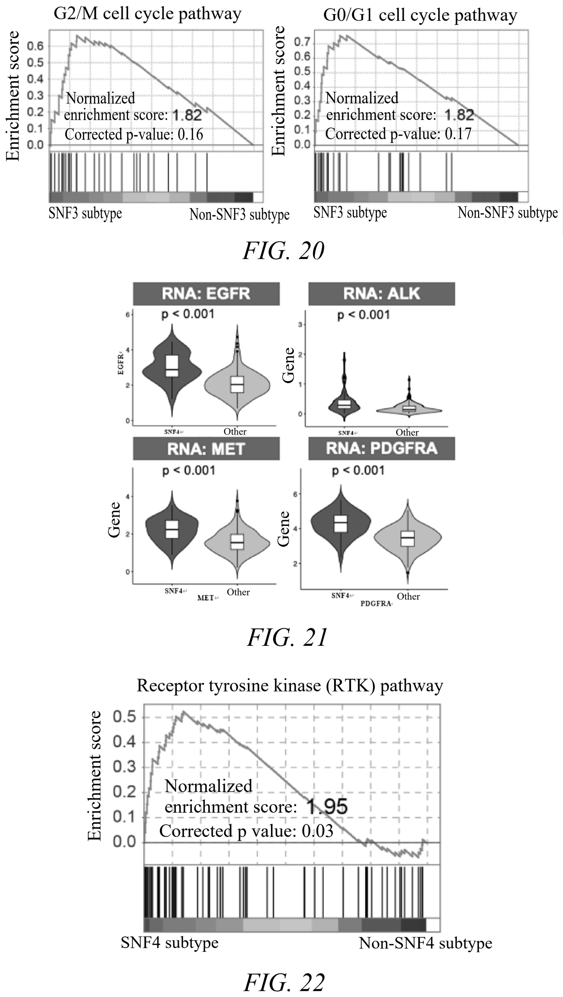

Typing studies based on multi-omics sequencing have confirmed that the upregulation of cell cycle-related pathways such as G0/G1 and G2/M is one of the hallmark features of SNF3 patients. As shown in FIG. 20, SNF3 patients exhibit higher enrichment scores in cell cycle-related pathways such as G0/G1 and G2/M (NES > 1.8), indicating a high enrichment of the aforementioned pathways, which is consistent with the previously identified characteristics of SNF3.

Preliminary studies indicated that SNF3 patients characteristically highly express E2F1, E2F2, and E2F-targeted genes. As shown in FIG. 19, the classic E2F1, E2F2, and E2F-targeted genes (such as AURKA, MYBL2, etc.) in SNF3 patients are also highly expressed, which is consistent with the previously identified characteristics of SNF3.

It has been previously considered that SNF4 patients characteristically highly express EGFR and RTK pathway-related genes. As can be seen from FIG. 21, SNF4 patients highly express RTK pathway genes such as EGFR, ALK, MET, and PDGFRA; As can be seen from FIG. 22, SNF4 patients have higher RTK pathway enrichment scores (NES > 1.8), indicating activation of the RTK pathway, which is consistent with the previously identified characteristics of SNF4.

### (8) SNF subtype method based on transcriptomic data

### 1) Development of SNF subtype method based on transcriptomic data

Referring to the method of Migliozzi et al. (Migliozzi, S. et al. Integrative multi-omics networks identify PKC δ and DNA-PK as master kinases of glioblastoma subtypes and guide targeted cancer therapy. Nat Cancer 4, 181-202 (2023)), we developed a random forest classifier to classify HR+/HER2- tumors into SNF subtypes using transcriptomic data from 351 samples of the CBCGA cohort described above. First, differential expression analysis was performed using the R package DESeq2 (v1.34), and the top 100 highly expressed genes for each subtype were retrieved based on the log₂(fold change) values. Secondly, we performed collinearity removal to reduce the candidate gene set. We removed all genes with a Spearman correlation coefficient greater than 0.75. Only retain the genes with the highest correlation to the corresponding SNF subtype, namely the SNF1-SNF4 highly expressed genes listed in Table 1. Finally, based on the expression of the remaining genes, using the multi-omics typing results of 351 samples from the CBCGA cohort as the gold standard, we developed a random forest classifier and measured its accuracy using ROC analysis. Based on the cohort of 351 samples, a transcriptome molecular typing prediction model (hereinafter referred to as the "NG model") was constructed using the random forest algorithm. This model included 309 predictive genes (i.e., classifier genes), with a molecular typing prediction accuracy of 84%, and the AUC of the 4 SNF subtypes was higher than 0.9. The NG modeling process is also described in the left panel of FIG. 43.

### 2) Simplification of the typing model

In order to further reduce the number of classifier genes to increase clinical application value, we simplified the 309 genes of the NG model based on the following procedure (right panel of FIG. 43):
1. A set of 319 candidate genes was determined, including 309 NG model-predicted genes and 10 manually added functional genes characteristic of each subtype from the SNF multi-omics typing (Table 3).

**Table 3: Characteristic genes of SNF subtypes**

| | |
|---|---|
| CD8A | SNF2 functional gene |
| CD8B | SNF2 functional gene |
| PRF1 | SNF2 functional gene |
| GZMB | SNF2 functional gene |
| CCND1 | SNF3 functional gene |
| E2F1 | SNF3 functional gene |
| MKI67 | SNF3 functional gene |
| RB1 | SNF3 functional gene |
| EGFR | SNF4 functional gene |
| PDGFRA | SNF4 functional gene |

### 2. Model Construction

The samples used for model construction are shown in the following table: Subtype numbers of each subtype in CBCGA (multi-omics gold standard cohort) and PCMC (accurate sequencing cohort).

| **Dataset** | **CBCGA (training set)** | **PCMC (test set)** | **Total** |
|---|---|---|---|
| Number of samples | 351 | 894 | 1245 |
| SNF1 | 86 | 251 | 337 |
| SNF2 | 89 | 96 | 185 |
| SNF3 | 118 | 342 | 460 |
| SNF4 | 58 | 205 | 263 |

2.1 According to the maximal relevance and minimal redundancy (mRMR) algorithm, the most relevant characteristic genes were selected from a candidate gene set of 319 genes, and the genes were ranked from largest to smallest based on the relevance of the candidate genes to the prediction, as shown in Table 4 below.

| **SNF1 gene** | **Overal l correl ation rankin 9** | **SNF2 gene** | **Overal l correl ation rankin 9** | **SNF3 gene** | **Overal l correl ation rankin 9** | **SNF4 gene** | **Overal l correl ation rankin 9** |
|---|---|---|---|---|---|---|---|
| LRRC31 | 1 | GLYATL2 | 74 | SLC4A10 | 6 | ANGPT4 | 2 |
| FMO5 | 3 | MSGN1 | 116 | PRAME | 33 | PTX3 | 12 |
| GALNT5 | 4 | ARSF | 117 | AMBP | 40 | DLK1 | 23 |
| ZBTB18 | 5 | PLA2G4E | 125 | IGDCC3 | 61 | SPANXA1 | 24 |
| CYP4A2 2 | 7 | DCD | 136 | LYZL2 | 63 | PKHD1L1 | 29 |
| SPAG6 | 8 | ORM2 | 143 | TMPRSS9 | 64 | MYOC | 39 |
| NKX3-1 | 9 | SEMG2 | 148 | PRLH | 69 | CAV3 | 41 |
| PYDC1 | 10 | ZP2 | 160 | ANK1 | 75 | CYP3A5 | 46 |
| ESRRG | 11 | SORCS3 | 167 | FAM25A | 78 | DPYS | 47 |
| SP5 | 13 | SCEL | 169 | SPDYC | 84 | LHCGR | 51 |
| PLA2G3 | 14 | SULT1C3 | 173 | TAS2R38 | 90 | C2orf40 | 57 |
| ZNF648 | 15 | DSPP | 175 | GPR139 | 91 | PCDH11X | 58 |
| ANKRD3 0A | 16 | FCRL4 | 177 | GDF1 | 92 | ACTC1 | 65 |
| FYB2 | 17 | MAGEA6 | 178 | CSMD3 | 93 | FGFBP1 | 67 |
| PIP | 18 | GNAT3 | 186 | KRT33A | 95 | MRGPRX 2 | 73 |
| CCDC17 5 | 19 | LHX5 | 192 | CGA | 98 | ADH4 | 76 |
| NTNG1 | 20 | C8B | 194 | COL2A1 | 101 | KCNA4 | 80 |
| HOXB6 | 21 | MYPN | 197 | ELFN2 | 107 | DES | 83 |
| CFAP15 7 | 22 | UGT1A4 | 202 | WFDC13 | 109 | SPHKAP | 87 |
| SLC4A4 | 25 | GABRQ | 204 | DSCAM | 111 | ABRA | 97 |
| MYO3B | 26 | OLIG3 | 205 | MYOG | 114 | CYP1A1 | 99 |
| AL45100 7.3 | 27 | UGT2B10 | 206 | TMEM179 | 122 | SERTM1 | 105 |
| SLC5A1 | 28 | AC010463 .1 | 207 | E2F1 | 124 | POTEM | 106 |
| KCNQ5 | 30 | PRF1 | 211 | SMIM32 | 126 | SLC15A5 | 110 |
| TRIM72 | 31 | CLCA2 | 215 | ATP12A | 127 | CDH12 | 113 |
| NAT1 | 32 | ACOD1 | 221 | VCX | 129 | MS4A6E | 119 |
| DNASE2 B | 34 | KIR3DX1 | 223 | DHRS2 | 132 | AC073082 .1 | 120 |
| ZNF385 B | 35 | SCN1A | 224 | CELF3 | 133 | OPRPN | 121 |
| CFAP65 | 36 | DKK1 | 225 | HMX3 | 138 | PRL | 128 |
| RASSF1 0 | 37 | MUC2 | 226 | GPR37L1 | 141 | DSG1 | 131 |
| NEK10 | 38 | MAGEA10 | 228 | GDPD2 | 144 | TRIM43B | 135 |
| POTEC | 42 | NR2E1 | 229 | TEX13B | 146 | PGA3 | 137 |
| HCRTR2 | 43 | SPATA3 | 231 | ZIC3 | 147 | OSTN | 140 |
| DLX2 | 44 | ZPBP2 | 235 | TCL1B | 149 | NPY2R | 145 |
| GAD1 | 45 | CTSE | 236 | UGT2A1 | 151 | KCNJ16 | 152 |
| ETNPPL | 48 | FGG | 238 | ASCL1 | 153 | SHOX | 158 |
| LTF | 49 | PRKAG3 | 239 | PRTN3 | 154 | SYT4 | 163 |
| AC0040 80.3 | 50 | AC233755 .2 | 240 | NAT8 | 156 | GABRA4 | 171 |
| CYP4X1 | 52 | IGSF23 | 243 | RB1 | 157 | CA10 | 172 |
| ZP4 | 53 | ERVV-1 | 245 | FAM196A | 159 | ADGRG4 | 179 |
| GABRR3 | 54 | OR2T34 | 246 | KRTAP4-1 | 161 | SAA1 | 182 |
| PEBP4 | 55 | HTR3A | 255 | SPINK13 | 162 | SLC7A14 | 184 |
| VIPR2 | 56 | COL6A5 | 256 | SLC22A24 | 164 | FAM205A | 187 |
| CACNA2 D3 | 59 | PSAPL1 | 258 | GSTA3 | 166 | EGFR | 191 |
| EVX1 | 60 | FAM9A | 260 | PCSK2 | 170 | IL13RA2 | 208 |
| RTP3 | 62 | AC141272 .1 | 261 | KRTAP10-1 | 174 | PDGFRA | 218 |
| NLRP13 | 66 | IVL | 262 | HS3ST6 | 176 | DDX3Y | 230 |
| FABP1 | 68 | WFDC12 | 264 | IP6K3 | 180 | SLC36A2 | 232 |
| TPH1 | 70 | STRA8 | 265 | SLC5A7 | 181 | FTMT | 248 |
| UCP1 | 71 | KRT4 | 266 | PTH2 | 183 | ARHGAP3 6 | 252 |
| DGAT2L 6 | 72 | GZMB | 267 | TEX19 | 185 | ANGPTL7 | 263 |
| KLK11 | 77 | OR56A3 | 268 | PABPC1L 2A | 188 | CLCA4 | 278 |
| IRX2 | 79 | PAX7 | 269 | KCNJ18 | 190 | | |
| SYT9 | 81 | GABBR2 | 271 | SLC22A9 | 195 | | |
| GHRH | 82 | S100A7A | 272 | ZDHHC22 | 196 | | |
| CLIC6 | 85 | MAGEA4 | 275 | NKX2-5 | 199 | | |
| HOXB8 | 86 | AC233755 .1 | 277 | CALHM1 | 200 | | |
| CYP2A7 | 88 | HOXD12 | 279 | DPYSL5 | 201 | | |
| NDNF | 89 | AMY1A | 281 | HSFX3 | 203 | | |
| SCUBE2 | 94 | AWAT2 | 282 | ZG16 | 209 | | |
| KLHL1 | 96 | KLHDC7B | 283 | AC092329 .3 | 210 | | |
| PPP4R4 | 100 | CP | 284 | KCNG4 | 212 | | |
| DLX1 | 102 | ACE2 | 286 | TH | 213 | | |
| SHROO M1 | 103 | IFNG | 288 | SST | 214 | | |
| IQCJ | 104 | C1QL2 | 289 | IFITM5 | 216 | | |
| LACRT | 108 | IL12RB2 | 293 | NDST4 | 217 | | |
| NEURO G2 | 112 | AKR1B15 | 295 | GFY | 219 | | |
| MYBPC1 | 115 | ISL2 | 296 | SVOP | 220 | | |
| GRIA2 | 118 | CASP14 | 297 | MCCD1 | 227 | | |
| IL1RAPL 1 | 123 | LY6D | 298 | LIN28A | 233 | | |
| CDH18 | 130 | CD8A | 299 | MYT1 | 234 | | |
| WFDC5 | 134 | PLEKHG7 | 301 | CHRNA9 | 237 | | |
| MUC5B | 139 | IL21 | 303 | CACNG2 | 242 | | |
| NEURO D1 | 142 | SLC15A1 | 304 | LRRC53 | 247 | | |
| SCGB3A 1 | 150 | NKX1-2 | 305 | DUXB | 249 | | |
| CLDN25 | 155 | IL22RA2 | 307 | FCRLB | 250 | | |
| CDH7 | 165 | GSDMC | 308 | SSTR5 | 254 | | |
| ANKRD3 4C | 168 | XIRP1 | 309 | NEUROD 4 | 257 | | |
| PRR20E | 189 | LBP | 311 | NR0B2 | 259 | | |
| SLC28A 3 | 193 | IGF2BP3 | 312 | CABP7 | 270 | | |
| RAB41 | 198 | S100A8 | 313 | ELAVL3 | 274 | | |
| KRTAP3 -3 | 222 | FABP7 | 315 | KCNH7 | 276 | | |
| SERPIN B10 | 241 | CD8B | 316 | TRH | 280 | | |
| SCGB1D 2 | 244 | VGLL1 | 317 | CDK5R2 | 285 | | |
| CHGB | 251 | A2ML1 | 318 | CST9 | 287 | | |
| WNT11 | 253 | CXCL9 | 319 | ACTL6B | 291 | | |
| NPY1R | 273 | | | CCND1 | 292 | | |
| SLCO1B 7 | 290 | | | LRTM2 | 294 | | |
| | | | | MKI67 | 300 | | |
| | | | | NEURL1 | 302 | | |
| | | | | SLC8A2 | 306 | | |
| | | | | RTBDN | 310 | | |
| | | | | MARCH4 | 314 | | |

10 manually added functional genes from Table 4 were excluded, the correlation ranking of the 309 predicted genes in Table 1 from largest to smallest was obtained, as shown in Table 5 below.

**Table 5**

| **SNF1 gene** | **Overall correl ation rankin 9** | **SNF2 gene** | **Overall correl ation rankin 9** | **SNF3 gene** | **Overall correl ation rankin 9** | **SNF4 gene** | **Overall correl ation rankin 9** |
|---|---|---|---|---|---|---|---|
| LRRC31 | 1 | GLYATL2 | 74 | SLC4A10 | 6 | ANGPT4 | 2 |
| FMO5 | 3 | MSGN1 | 116 | PRAME | 33 | PTX3 | 12 |
| GALNT5 | 4 | ARSF | 117 | AMBP | 40 | DLK1 | 23 |
| ZBTB18 | 5 | PLA2G4E | 124 | IGDCC3 | 61 | SPANXA1 | 24 |
| CYP4A2 2 | 7 | DCD | 135 | LYZL2 | 63 | PKHD1L1 | 29 |
| SPAG6 | 8 | ORM2 | 142 | TMPRSS9 | 64 | MYOC | 39 |
| NKX3-1 | 9 | SEMG2 | 147 | PRLH | 69 | CAV3 | 41 |
| PYDC1 | 10 | ZP2 | 158 | ANK1 | 75 | CYP3A5 | 46 |
| ESRRG | 11 | SORCS3 | 165 | FAM25A | 78 | DPYS | 47 |
| SP5 | 13 | SCEL | 167 | SPDYC | 84 | LHCGR | 51 |
| PLA2G3 | 14 | SULT1C3 | 171 | TAS2R38 | 90 | C2orf40 | 57 |
| ZNF648 | 15 | DSPP | 173 | GPR139 | 91 | PCDH11X | 58 |
| ANKRD3 0A | 16 | FCRL4 | 175 | GDF1 | 92 | ACTC1 | 65 |
| FYB2 | 17 | MAGEA6 | 176 | CSMD3 | 93 | FGFBP1 | 67 |
| PIP | 18 | GNAT3 | 184 | KRT33A | 95 | MRGPRX 2 | 73 |
| CCDC17 5 | 19 | LHX5 | 189 | CGA | 98 | ADH4 | 76 |
| NTNG1 | 20 | C8B | 191 | COL2A1 | 101 | KCNA4 | 80 |
| HOXB6 | 21 | MYPN | 194 | ELFN2 | 107 | DES | 83 |
| CFAP15 7 | 22 | UGT1A4 | 199 | WFDC13 | 109 | SPHKAP | 87 |
| SLC4A4 | 25 | GABRQ | 201 | DSCAM | 111 | ABRA | 97 |
| MYO3B | 26 | OLIG3 | 202 | MYOG | 114 | CYP1A1 | 99 |
| AL45100 7.3 | 27 | UGT2B10 | 203 | TMEM179 | 122 | SERTM1 | 105 |
| SLC5A1 | 28 | AC010463 .1 | 204 | SMIM32 | 125 | POTEM | 106 |
| KCNQ5 | 30 | CLCA2 | 211 | ATP12A | 126 | SLC15A5 | 110 |
| TRIM72 | 31 | ACOD1 | 216 | VCX | 128 | CDH12 | 113 |
| NAT1 | 32 | KIR3DX1 | 218 | DHRS2 | 131 | MS4A6E | 119 |
| DNASE2 B | 34 | SCN1A | 219 | CELF3 | 132 | AC073082 .1 | 120 |
| ZNF385 B | 35 | DKK1 | 220 | HMX3 | 137 | OPRPN | 121 |
| CFAP65 | 36 | MUC2 | 221 | GPR37L1 | 140 | PRL | 127 |
| RASSF1 0 | 37 | MAGEA10 | 223 | GDPD2 | 143 | DSG1 | 130 |
| NEK10 | 38 | NR2E1 | 224 | TEX13B | 145 | TRIM43B | 134 |
| POTEC | 42 | SPATA3 | 226 | ZIC3 | 146 | PGA3 | 136 |
| HCRTR2 | 43 | ZPBP2 | 230 | TCL1B | 148 | OSTN | 139 |
| DLX2 | 44 | CTSE | 231 | UGT2A1 | 150 | NPY2R | 144 |
| GAD1 | 45 | FGG | 233 | ASCL1 | 152 | KCNJ16 | 151 |
| ETNPPL | 48 | PRKAG3 | 234 | PRTN3 | 153 | SHOX | 156 |
| LTF | 49 | AC233755 .2 | 235 | NAT8 | 155 | SYT4 | 161 |
| AC0040 80.3 | 50 | IGSF23 | 238 | FAM196A | 157 | GABRA4 | 169 |
| CYP4X1 | 52 | ERVV-1 | 240 | KRTAP4-1 | 159 | CA10 | 170 |
| ZP4 | 53 | OR2T34 | 241 | SPINK13 | 160 | ADGRG4 | 177 |
| GABRR3 | 54 | HTR3A | 250 | SLC22A24 | 162 | SAA1 | 180 |
| PEBP4 | 55 | COL6A5 | 251 | GSTA3 | 164 | SLC7A14 | 182 |
| VIPR2 | 56 | PSAPL1 | 253 | PCSK2 | 168 | FAM205A | 185 |
| CACNA2 D3 | 59 | FAM9A | 255 | KRTAP10-1 | 172 | IL13RA2 | 205 |
| EVX1 | 60 | AC141272 .1 | 256 | HS3ST6 | 174 | DDX3Y | 225 |
| RTP3 | 62 | IVL | 257 | IP6K3 | 178 | SLC36A2 | 227 |
| NLRP13 | 66 | WFDC12 | 259 | SLC5A7 | 179 | FTMT | 243 |
| FABP1 | 68 | STRA8 | 260 | PTH2 | 181 | ARHGAP3 6 | 247 |
| TPH1 | 70 | KRT4 | 261 | TEX19 | 183 | ANGPTL7 | 258 |
| UCP1 | 71 | OR56A3 | 262 | PABPC1L 2A | 186 | CLCA4 | 272 |
| DGAT2L 6 | 72 | PAX7 | 263 | KCNJ18 | 188 | | |
| KLK11 | 77 | GABBR2 | 265 | SLC22A9 | 192 | | |
| IRX2 | 79 | S100A7A | 266 | ZDHHC22 | 193 | | |
| SYT9 | 81 | MAGEA4 | 269 | NKX2-5 | 196 | | |
| GHRH | 82 | AC233755 .1 | 271 | CALHM1 | 197 | | |
| CLIC6 | 85 | HOXD12 | 273 | DPYSL5 | 198 | | |
| HOXB8 | 86 | AMY1A | 275 | HSFX3 | 200 | | |
| CYP2A7 | 88 | AWAT2 | 276 | ZG16 | 206 | | |
| NDNF | 89 | KLHDC7B | 277 | AC092329 .3 | 207 | | |
| SCUBE2 | 94 | CP | 278 | KCNG4 | 208 | | |
| KLHL1 | 96 | ACE2 | 280 | TH | 209 | | |
| PPP4R4 | 100 | IFNG | 282 | SST | 210 | | |
| DLX1 | 102 | C1QL2 | 283 | IFITM5 | 212 | | |
| SHROO M1 | 103 | IL12RB2 | 286 | NDST4 | 213 | | |
| IQCJ | 104 | AKR1B15 | 288 | GFY | 214 | | |
| LACRT | 108 | ISL2 | 289 | SVOP | 215 | | |
| NEURO G2 | 112 | CASP14 | 290 | MCCD1 | 222 | | |
| MYBPC1 | 115 | LY6D | 291 | LIN28A | 228 | | |
| GRIA2 | 118 | PLEKHG7 | 292 | MYT1 | 229 | | |
| IL1RAPL 1 | 123 | IL21 | 294 | CHRNA9 | 232 | | |
| CDH18 | 129 | SLC15A1 | 295 | CACNG2 | 237 | | |
| WFDC5 | 133 | NKX1-2 | 296 | LRRC53 | 242 | | |
| MUC5B | 138 | IL22RA2 | 298 | DUXB | 244 | | |
| NEURO D1 | 141 | GSDMC | 299 | FCRLB | 245 | | |
| SCGB3A 1 | 149 | XIRP1 | 300 | SSTR5 | 249 | | |
| CLDN25 | 154 | LBP | 302 | NEUROD 4 | 252 | | |
| CDH7 | 163 | IGF2BP3 | 303 | NR0B2 | 254 | | |
| ANKRD3 4C | 166 | S100A8 | 304 | CABP7 | 264 | | |
| PRR20E | 187 | FABP7 | 306 | ELAVL3 | 268 | | |
| SLC28A 3 | 190 | VGLL1 | 307 | KCNH7 | 270 | | |
| RAB41 | 195 | A2ML1 | 308 | TRH | 274 | | |
| KRTAP3 -3 | 217 | CXCL9 | 309 | CDK5R2 | 279 | | |
| SERPIN B10 | 236 | | | CST9 | 281 | | |
| SCGB1D 2 | 239 | | | ACTL6B | 285 | | |
| CHGB | 246 | | | LRTM2 | 287 | | |
| WNT11 | 248 | | | NEURL1 | 293 | | |
| NPY1R | 267 | | | SLC8A2 | 297 | | |
| SLCO1B 7 | 284 | | | RTBDN | 301 | | |
| | | | | MARCH4 | 305 | | |

2.2 Using the SVM algorithm and through leave-one-out cross-validation, the TOP N genes (top N genes by correlation), Bottom N genes (bottom N genes by correlation), and Random N genes (random N genes) were respectively selected from 319 candidate genes for modeling and accuracy calculation sequentially, with the results shown in Table 6 below.

**Table 6**

| **Group** | **Number of genes (N)** | **Train.A CC** | **SNF1 (AUC)** | **SNF2 (AUC)** | **SNF3 (AUC)** | **SNF4 (AUC)** |
|---|---|---|---|---|---|---|
| Top | 30 | 0.8034 | 0.9663 | 0.9501 | 0.9564 | 0.9763 |
| Rando m | 30 | 0.77379 | 0.94355 | 0.92456 | 0.93813 | 0.91387 |
| Bottom | 30 | 0.7151 | 0.9045 | 0.9133 | 0.9284 | 0.8751 |
| Top | 60 | 0.8661 | 0.9828 | 0.9645 | 0.9732 | 0.9712 |
| Rando m | 60 | 0.85413 | 0.97069 | 0.96973 | 0.97694 | 0.9651 |
| Bottom | 60 | 0.7493 | 0.9192 | 0.9341 | 0.949 | 0.9031 |
| Top | 90 | 0.9003 | 0.9895 | 0.983 | 0.988 | 0.9876 |
| Rando m | 90 | 0.89088 | 0.98237 | 0.98539 | 0.99104 | 0.98006 |
| Bottom | 90 | 0.8034 | 0.9384 | 0.9533 | 0.9668 | 0.9406 |
| Top | 120 | 0.9117 | 0.9918 | 0.984 | 0.9909 | 0.984 |
| Rando m | 120 | 0.90683 | 0.9857 | 0.98941 | 0.99358 | 0.9859 |
| Bottom | 120 | 0.8462 | 0.96 | 0.9741 | 0.9843 | 0.9616 |
| Top | 150 | 0.9259 | 0.9938 | 0.9858 | 0.9924 | 0.9913 |
| Rando m | 150 | 0.92194 | 0.98885 | 0.99307 | 0.99562 | 0.99055 |
| Bottom | 150 | 0.8519 | 0.9698 | 0.9813 | 0.9856 | 0.9726 |
| Top | 180 | 0.9402 | 0.9954 | 0.9925 | 0.9948 | 0.9859 |
| Rando m | 180 | 0.9299 | 0.99095 | 0.99449 | 0.99689 | 0.99317 |
| Bottom | 180 | 0.8917 | 0.9823 | 0.9895 | 0.9927 | 0.9788 |
| Top | 210 | 0.9373 | 0.9954 | 0.9958 | 0.9975 | 0.9904 |
| Rando m | 210 | 0.93448 | 0.99218 | 0.99567 | 0.99758 | 0.99443 |
| Bottom | 210 | 0.906 | 0.9884 | 0.9934 | 0.9958 | 0.9877 |
| Top | 240 | 0.9459 | 0.9953 | 0.9973 | 0.9987 | 0.9959 |
| Rando m | 240 | 0.93732 | 0.9928 | 0.99611 | 0.99793 | 0.99527 |
| Bottom | 240 | 0.9202 | 0.9903 | 0.9971 | 0.997 | 0.992 |
| Top | 270 | 0.943 | 0.9951 | 0.9976 | 0.9985 | 0.9966 |
| Rando m | 270 | 0.94188 | 0.99393 | 0.99679 | 0.99848 | 0.99599 |
| Bottom | 270 | 0.9373 | 0.9945 | 0.9972 | 0.9983 | 0.9945 |
| Top | 300 | 0.943 | 0.9951 | 0.9976 | 0.9984 | 0.9965 |
| Rando m | 300 | 0.94529 | 0.99477 | 0.99752 | 0.9986 | 0.99671 |
| Bottom | 300 | 0.9373 | 0.9946 | 0.9976 | 0.9984 | 0.9964 |

2.3 Evaluation of performance under different numbers of genes: The 319 candidate genes in the set were ranked according to correlation, with every 30 genes divided into one interval. The number of genes corresponding to the highest accuracy in each interval was selected. Then, based on the corresponding genes, an SVM model was constructed using the entire cohort of 351 samples ("multi-omics gold standard cohort"), and the accuracy as well as the AUC of each subtype were respectively calculated. The results are shown in Table 7 below.

**Table 7**

| **Range of number of genes** | **Number of genes corresponding to optimal performance** | **LOOC V.ACC** | **Train. ACC** | **SNF1 (AUC)** | **SNF2 (AUC)** | **SNF3 (AUC)** | **SNF4 (AUC)** | **LOOC V.ACC** |
|---|---|---|---|---|---|---|---|---|
| [1,30] | 23 | 0.612 5 | 0.789 2 | 0.969 2 | 0.935 1 | 0.949 5 | 0.964 6 | 0.612 5 |
| [31,60] | 41 | 0.646 7 | 0.831 9 | 0.981 5 | 0.956 7 | 0.969 1 | 0.973 9 | 0.646 7 |
| [61,90] | 90 | 0.692 3 | 0.900 3 | 0.989 5 | 0.983 | 0.988 | 0.987 6 | 0.692 3 |
| [91,120] | 112 | 0.726 5 | 0.911 7 | 0.991 6 | 0.984 5 | 0.990 5 | 0.987 9 | 0.726 5 |
| [121,150] | 141 | 0.726 5 | 0.923 1 | 0.993 5 | 0.984 | 0.992 2 | 0.986 4 | 0.726 5 |
| [151,180] | 178 | 0.743 6 | 0.940 2 | 0.995 4 | 0.993 1 | 0.994 8 | 0.985 9 | 0.743 6 |
| [181,210] | 210 | 0.777 8 | 0.937 3 | 0.995 4 | 0.995 8 | 0.997 5 | 0.990 4 | 0.777 8 |
| [211,240] | 211 | 0.777 8 | 0.940 2 | 0.995 4 | 0.996 7 | 0.997 9 | 0.991 | 0.777 8 |
| [241,270] | 257 | 0.777 8 | 0.943 | 0.995 3 | 0.997 5 | 0.998 8 | 0.996 7 | 0.777 8 |
| [271,300] | 271 | 0.763 5 | 0.943 | 0.995 1 | 0.997 6 | 0.998 5 | 0.996 4 | 0.763 5 |

### 3. Balanced gene distribution

3.1 Random selection of TOP genes may lead to uneven distribution of predictive genes among subtypes. To ensure that a considerable number of genes of each subtype was included for modeling, the TOP5-TOP50 genes related to each subtype were respectively selected for modeling.

3.3 Performance evaluation: The prediction accuracy of the model in CBCGA, the mean squared error of the proportion of each subtype in the test set (PCMC) and training set (CBCGA), and the AUC for predicting SNF subtypes were evaluated.

The results are shown in Table 8 below.

**Table 8**

| **Number of respective TOP genes used for each subtype** | **Total number of genes** | **Train. ACC** | **Test. RMSE** | **SNF1** | **SNF2** | **SNF3** | **SNF4** |
|---|---|---|---|---|---|---|---|
| 5 | 20 | 0.809 1 | 4.19 | 0.949 7 | 0.904 2 | 0.952 9 | 0.939 2 |
| 10 | 40 | 0.834 8 | 4.04 | 0.974 | 0.952 2 | 0.978 8 | 0.970 7 |
| 15 | 60 | 0.906 | 2.90 | 0.984 | 0.985 2 | 0.994 8 | 0.981 9 |
| 20 | 80 | 0.917 4 | 3.41 | 0.985 7 | 0.981 2 | 0.991 5 | 0.981 4 |
| 25 | 100 | 0.917 4 | 2.69 | 0.987 8 | 0.991 7 | 0.996 1 | 0.980 8 |
| 30 | 120 | 0.925 9 | 2.62 | 0.989 4 | 0.992 5 | 0.996 8 | 0.991 5 |
| 35 | 140 | 0.923 1 | 1.80 | 0.991 | 0.992 5 | 0.998 1 | 0.993 2 |
| 40 | 160 | 0.937 3 | 1.94 | 0.992 5 | 0.994 | 0.998 4 | 0.993 2 |
| 45 | 180 | 0.940 2 | 3.75 | 0.992 1 | 0.994 6 | 0.998 6 | 0.994 2 |
| 50 | 200 | 0.937 3 | 4.31 | 0.992 9 | 0.995 6 | 0.998 6 | 0.995 4 |

As an example, the TOP25 genes related to each subtype are used and shown in Table 9:

**Table 9**

| **SNF1 gene** | **Overall ranking** | **SNF2 gene** | **Overall ranking** | **SNF3 gene** | **Overall ranking** | **SNF4 gene** | **Overall ranking** |
|---|---|---|---|---|---|---|---|
| LRRC31 | 1 | GLYATL2 | 74 | SLC4A10 | 6 | ANGPT4 | 2 |
| FMO5 | 3 | MSGN1 | 116 | PRAME | 33 | PTX3 | 12 |
| GALNT5 | 4 | ARSF | 117 | AMBP | 40 | DLK1 | 23 |
| ZBTB18 | 5 | PLA2G4E | 125 | IGDCC3 | 61 | SPANXA 1 | 24 |
| CYP4A2 2 | 7 | DCD | 136 | LYZL2 | 63 | PKHD1L 1 | 29 |
| SPAG6 | 8 | ORM2 | 143 | TMPRSS 9 | 64 | MYOC | 39 |
| NKX3-1 | 9 | SEMG2 | 148 | PRLH | 69 | CAV3 | 41 |
| PYDC1 | 10 | ZP2 | 160 | ANK1 | 75 | CYP3A5 | 46 |
| ESRRG | 11 | SORCS3 | 167 | FAM25A | 78 | DPYS | 47 |
| SP5 | 13 | SCEL | 169 | SPDYC | 84 | LHCGR | 51 |
| PLA2G3 | 14 | SULT1C3 | 173 | TAS2R3 8 | 90 | C2orf40 | 57 |
| ZNF648 | 15 | DSPP | 175 | GPR139 | 91 | PCDH11 X | 58 |
| ANKRD3 0A | 16 | FCRL4 | 177 | GDF1 | 92 | ACTC1 | 65 |
| FYB2 | 17 | MAGEA6 | 178 | CSMD3 | 93 | FGFBP1 | 67 |
| PIP | 18 | GNAT3 | 186 | KRT33A | 95 | MRGPR X2 | 73 |
| CCDC17 5 | 19 | LHX5 | 192 | CGA | 98 | ADH4 | 76 |
| NTNG1 | 20 | C8B | 194 | COL2A1 | 101 | KCNA4 | 80 |
| HOXB6 | 21 | MYPN | 197 | ELFN2 | 107 | DES | 83 |
| CFAP15 7 | 22 | UGT1A4 | 202 | WFDC13 | 109 | SPHKAP | 87 |
| SLC4A4 | 25 | GABRQ | 204 | DSCAM | 111 | ABRA | 97 |
| MYO3B | 26 | OLIG3 | 205 | MYOG | 114 | CYP1A1 | 99 |
| AL45100 7.3 | 27 | UGT2B10 | 206 | TMEM17 9 | 122 | SERTM1 | 105 |
| SLC5A1 | 28 | AC010463. 1 | 207 | E2F1 | 124 | POTEM | 106 |
| KCNQ5 | 30 | PRF1 | 211 | SMIM32 | 126 | SLC15A5 | 110 |
| TRIM72 | 31 | CLCA2 | 215 | ATP12A | 127 | CDH12 | 113 |

In the above tables, Train.ACC represents the accuracy of the model in predicting the SNF typing of all samples in the CBCGA after modeling using the CBCGA cohort; SNFx(AUC) represents the AUC of the model in predicting each subtype respectively; test.RMSE represents the mean squared error of the proportion of each subtype in the test set (PCMC) and the training set (CBCGA), where CBCGA is the gold standard typing result, and PCMC is the typing result using the NG model.

The above results show that multiple simplified gene combination models of the 319 candidate genes can achieve the SNF typing of the present invention with high accuracy and AUC.

In addition, according to the modeling ranking of 319 genes, the correlation rankings of the 10 manually added functional genes are all relatively low, with the highest ranking being 124, corresponding to the gene E2F1, which has a relatively small impact on the modeling of candidate genes; therefore, it is unnecessary to perform re-modeling validation of the simplified model with 309 genes. The conclusion of the simplified gene combination confirmed by modeling based on 319 genes is equally applicable to the simplified model of 309 genes.

### (9) Establishment of drug response testing platform

### 1) Preparation and culture of patient-derived organoids (PDO)

A biobank for organoid storage was established using a recognized method reported in prior literature (Gong Y, Ji P, Yang YS, Xie S, Yu TJ, Xiao Y, et al. Metabolic-Pathway-Based Subtyping of Triple-Negative Breast Cancer Reveals Potential Therapeutic Targets. CELL METAB. 2021;33(1):51-64 doi 10.1016/j.cmet.2020.10.012). Briefly, the fresh breast cancer tissue is cut into small pieces using a sterile surgical knife. The tissue was digested and resuspended in 10 mL of TAC buffer, incubated for 3 minutes to remove red blood cells, and passed through a 100 mm cell strainer (Corning). For passaging, 5 ml of harvesting solution (Trevigen, 3700-100-01) was used to digest the basement membrane extract (BME), which was incubated on ice for 1 hour. Subsequently, the organoids were centrifuged at 350 g for 5 minutes, washed in digestion buffer, and centrifuged. Next, 3 mL of TrypLE Express (Invitrogen) was added, and the organoids were incubated at room temperature for 3 minutes and then mechanically dissociated into small cell clusters using a pipette. The organoids were passaged every 2-3 weeks at a dilution ratio of 1:2-3.

### 2) Drug response testing of PDO

Drug response testing of triple-negative breast cancer (TNBC) organoids was conducted according to the method of the published paper (Xiao Y, Ma D, Yang YS, Yang F, Ding JH, Gong Y, et al. Comprehensive metabolomics expands precision medicine for triple-negative breast cancer. CELL RES. 2022;32(5):477-90 doi 10.1038/s41422-022-00614-0.). Organoids without undergoing organoid drug treatment were harvested in good condition and digested into single cells. 25 microliters of organoid suspension was added to black cell-repellent surface of a 384-well plate with transparent bottoms (Greiner 781976-SIN) at 1 × 10³ to 3 × 10³ cells per well, and cultured for an additional 5-6 days prior to drug treatment. Organoids were co-cultured with the drug for 1 week and then tested for survival. According to the manufacturer's instructions, organoid cell survival rate was assessed by the CellTiter-Glo 3D cell survival assay (Promega, G9683).

### 3) Evaluation of the mechanism of endocrine therapy efficacy

### SET index analysis

The SET index is a marker indicating patient sensitivity to endocrine therapy, as defined in the literature (Speers CW, Symmans WF, Barlow WE, Trevarton A, The S, Du L, et al. Evaluation of the Sensitivity to Endocrine Therapy Index and 21-Gene Breast Recurrence Score in the SWOG S8814 Trial. J CLIN ONCOL. 2023:O2201499 doi 10.1200/JCO.22.01499; Du L, Yau C, Brown-Swigart L, Gould R, Krings G, Hirst GL, et al. Predicted sensitivity to endocrine therapy for stage II-III hormone receptor-positive and HER2-negative (HR+/HER2-) breast cancer before chemo-endocrine therapy. ANN ONCOL. 2021;32(5):642-51 doi 10.1016/j.annonc.2021.02.011.; Sinn BV, Fu C, Lau R, Litton J, Tsai TH, Murthy R, et al. SET(ER/PR): a robust 18-gene predictor for sensitivity to endocrine therapy for metastatic breast cancer. NPJ Breast Cancer. 2019; 5:16 doi 10.1038/s41523-019-0111-0.).

### Single-sample gene set enrichment analysis (ssGSEA)

For single-sample gene set enrichment analysis, the gene sets listed in Supplementary Table S2 were used, and the "GSVA" function in R was employed to calculate the enrichment of the tamoxifen (TAM)/SERD response module and endocrine therapy resistance pathways.

### 4) Patient-derived tumor fragment (PDTF) platform

### PDTF culture

As previously reported, the collected tissue materials suitable for PDTF culture were cut on ice into small tumor fragments of 1-2 mm³ in size (Voabil P, de Bruijn M, Roelofsen LM, Hendriks SH, Brokamp S, van den Braber M, et al. An ex vivo tumor fragment platform to dissect response to PD-1 blockade in cancer. NAT MED. 2021;27(7):1250-61 doi 10.1038/s41591-021-01398-3.). Single PDTFs from different regions within the tumor were mixed to ensure uniform representation of the entire tumor. A single PDTF was embedded in the artificial extracellular matrix prepared as follows: tumor culture medium supplemented with 1.1% sodium bicarbonate, 1 mM sodium pyruvate, 1× MEM non-essential amino acids, 2 mM L-glutamine, 10% fetal bovine serum, 1% penicillin-streptomycin, and collagen (final concentration of 1 mg/ml) was mixed with ice-cold Matrigel (final concentration of 4 mg/ml). A 96-well plate was taken, and 40 µl of matrix was applied to each well as the basal layer, followed by exposure at 37°C for 20-30 minutes to cure. A single PDTF of each well was placed on top of the pre-cured matrix, and then a second layer of 40 µl matrix was placed. Then the plate was placed in an incubator at 37°C for 20-30 minutes to wait for curing. Next, 140 µl of tumor culture medium was added to the top, wherein the tumor culture medium was supplemented with nivolumab (final concentration 20 µg/ml) or human anti-β-Gal-hIgG4 as a control. For each condition, 8 PDTFs were used, and before flow cytometry analysis, the PDTF cultures were maintained at 37°C for 48 hours.

### PDTF flow cytometry analysis

PDTF was analyzed by flow cytometry to assess T cell activation after culture with nivolumab or IgG, thereby determining the tumor immune environment. As described in the literature, antibodies from BioLegend were used to detect T cell activation (Voabil P, de Bruijn M, Roelofsen LM, Hendriks SH, Brokamp S, van den Braber M, et al. An ex vivo tumor fragment platform to dissect response to PD-1 blockade in cancer. NAT MED. 2021;27(7):1250-61 doi 10.1038/s41591-021-01398-3.). PDTFs manually retrieved from the matrix for flow cytometry analysis were enzymatically digested into single-cell suspensions under each experimental condition by slow rotation at 37°C for 1-2 hours in a digestion mixture (DMEM/F12 medium supplemented with 1% penicillin-streptomycin, 10% bovine serum albumin, 0.5 µg/ml hydrocortisone, 5 µM Y-27623, 1X insulin, 1 mg/ml type IV collagenase [Worthington], hyaluronidase [Sigma], and pulmozyme [Sigma]). The digested sample was filtered twice through a 70 µM filter, washed in PBS, resuspended in 100 µl PBS, and incubated with Fc receptor blocker (BioLegend) and Zombie NIR (BioLegend) at 4°C for 20 minutes. Cells were washed and resuspended in 50 µl of staining buffer (BioLegend) containing the above antibodies, and incubated at 4°C for 20 minutes. After washing twice, the cells were transferred to 100 µl of cell staining buffer and subjected to flow cytometry analysis.

### Analysis of secreted mediators

In order to analyze cytokines, chemokines, and cytotoxic mediators, the supernatant of PDTF cultures was collected after 48 hours of culture. The supernatant was immediately frozen and stored at - 80°C. The supernatant was thawed on ice. Except for single fragment analysis, the supernatants under each condition were combined, and according to the manufacturer's instructions, the presence of the above cytokines and chemokines was detected using LEGENDplex Human Th Cytokine, Human Proinflammatory Chemokine (BioLegend), or IFN/TNF Human ELISA kits (BioLegend).

### 5) Quantitative and statistical analysis

Frequency tables and summary statistics were used to characterize data distribution. Wilcoxon test and Kruskal-Wallis test were used to compare continuous variables and categorical variables, and Pearson chi-square test and Fisher's exact test were used to compare unordered categorical variables. Survival curves were established using the Kaplan-Meier product-limit method and compared with the log-rank test to obtain RFS and PFS. RFS was defined as the time from diagnosis to the first recurrence or diagnosis of contralateral breast cancer. PFS was defined as the interval from the start of treatment to disease progression or death due to any cause (whichever occurs first), or to the time of the last PFS assessment for patients with progression-free survival. Univariate and multivariate Cox proportional hazards regression models were constructed to evaluate the hazard ratio (HR) and the corresponding 95% confidence interval (CI). All analyses were performed using R software package version 3.6.1 (https://cran.r-project.org/).

### (10) Sensitivity of SNF1 subtype to endocrine therapy

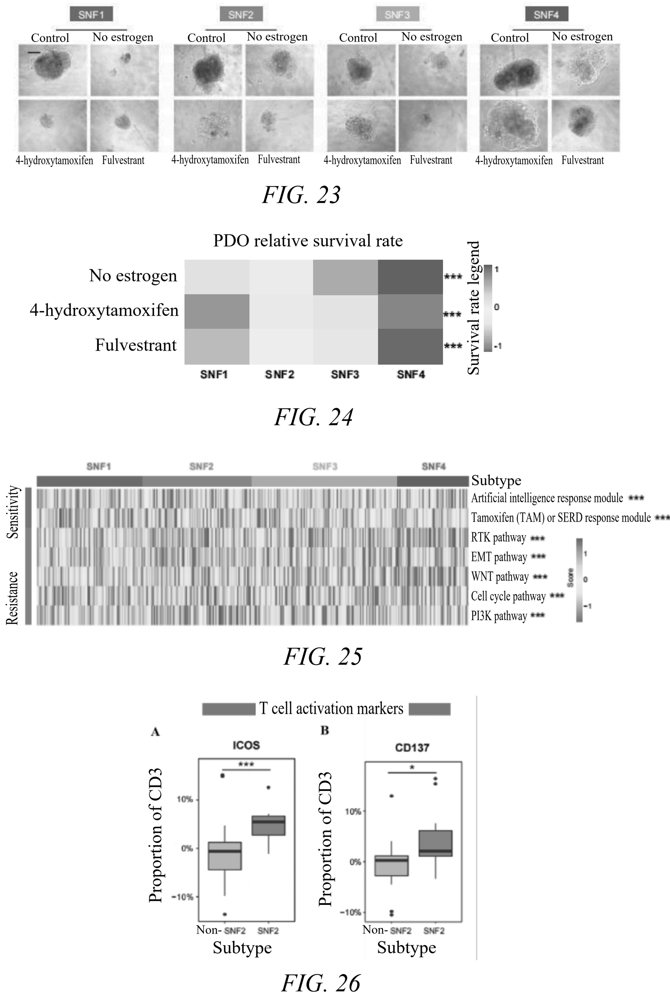

The sensitivity of four subtypes of PDOs (n = 88) to three traditional endocrine therapies (estrogen deprivation, 4-hydroxytamoxifen (4OHtam), and fulvestrant) was compared on a PDO drug testing platform. It was observed that SNF1-PDO exhibited the lowest survival rate among the three traditional endocrine therapies, whereas SNF4-PDO exhibited a relatively higher survival rate (FIGs. 23 and 24). In order to investigate the potential mechanism of SNF1 tumor sensitivity to endocrine therapy, the endocrine therapy-related pathways in the multi-omics data from Fudan University Shanghai Cancer Center were analyzed. A high enrichment of the SET index was observed in SNF1 tumors, and the endocrine therapy response module was also highly expressed in SNF1 tumors (FIG. 25). In addition, the expression of endocrine therapy resistance pathways was also compared among the four subtypes. The heatmap showed that the endocrine therapy resistance pathways (cell cycle pathway, RTK pathway, WNT pathway, EMT pathway, and PI3K pathway) in SNF1 subtype tumors were decreased in expression (FIG. 25). This result demonstrated that patients of the SNF1 subtype were sensitive to endocrine therapy, with relatively high activation of response modules and relatively low expression of various resistance modules.

### (11) Sensitivity of SNF2 subtypes to immunotherapy

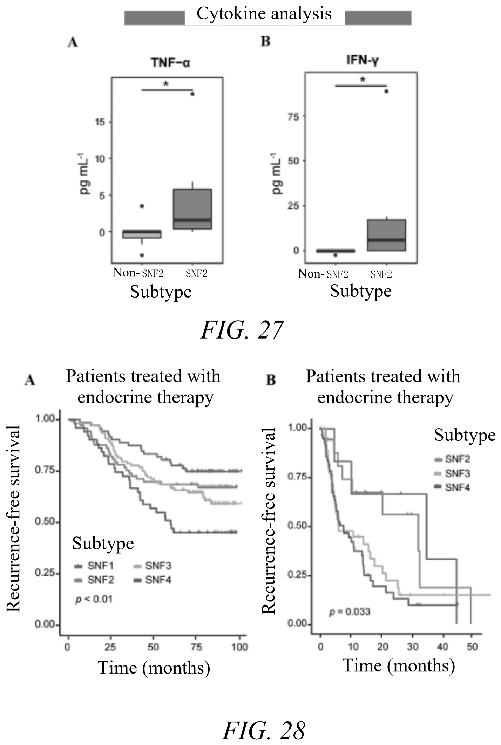

As described in the literature (Xi Jin, Yi-Fan Zhou, Ding Ma, Shen Zhao, Cai-Jin Lin, Yi Xiao, et al. Integrative Molecular Classification of HR+/HER2- Breast Cancer Guides Precision Treatment. Nature Genetics under minor revision. 2023.02.26.), SNF2 subtype tumors are rich in immune cells, and therefore may be sensitive to immunotherapy, especially immune checkpoint therapy (such as PD-1 antibody, PD-L1 antibody, CTLA4 antibody, etc.). To verify this hypothesis, we utilized the PDTF platform to analyze the immune response of human tumor tissues to ex vivo PD-1 blockade. Using cytokines and T cell activation markers as readouts, the effect of PD-1 blockade on 49 PDTFs after incubation with anti-PD-1 antibody for 48 hours was analyzed. The results of flow cytometry showed that, compared with other subtypes, the CD3+ total T activation markers (ICOS, CD137) of the SNF2 subtype increased more significantly from baseline after immunotherapy (FIGs. 26A and 26B). Importantly, in the SNF2 subtype, PD-1 blockade not only significantly increased those parameters directly reflecting T cell activation, but also significantly increased cytokines (TNF-α and IFN-y) (FIGs. 27A and 27B).

### (12) Susceptibility of the SNF3 subtype to treatment with CDK4/6 inhibitor and PARP inhibitor

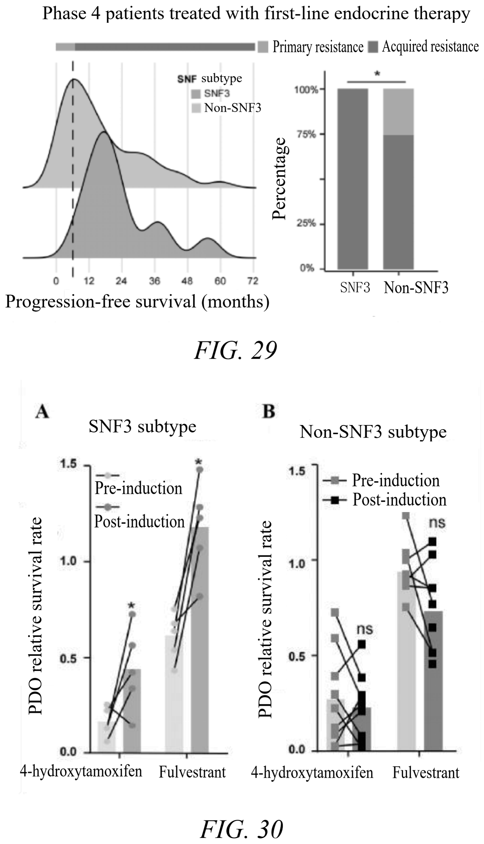

The SNF3 subtype exhibited different sensitivities to endocrine therapy in real-world cohorts of early and advanced HR⁺/HER2⁻ breast cancer (FIGs. 28A and 28B); therefore, it is necessary to explore its intrinsic mechanisms and identify new therapeutic strategies. PFS time analysis of first-line endocrine therapy in phase 4 patients from a real-world cohort revealed that most SNF3 subtype patients experienced progression after 6 months of endocrine therapy (FIG. 29), indicating that SNF3 subtype patients may develop secondary resistance to endocrine therapy administered according to the international consensus guidelines for advanced breast cancer. In order to gain an in-depth understanding of the biological characteristics of the SNF3 subtype, low-concentration 4-hydroxytamoxifen was used to induce PDO over an extended period to establish a model for studying acquired resistance to endocrine therapy (Xi Jin, Yi-Fan Zhou, Ding Ma, Shen Zhao, Cai-Jin Lin, Yi Xiao, et al. Integrative Molecular Classification of HR+/HER2- Breast Cancer Guides Precision Treatment. Nature Genetics under minor revision. 2023.02.26.). Surprisingly, unlike other subtypes, after long-term induction with 4-hydroxytamoxifen, SNF3-PDO became less sensitive to endocrine therapy (FIGs. 30A and 30B). The above results indicated that endocrine therapy alone may be insufficient for the SNF3 subtype and that intensified treatment was required.

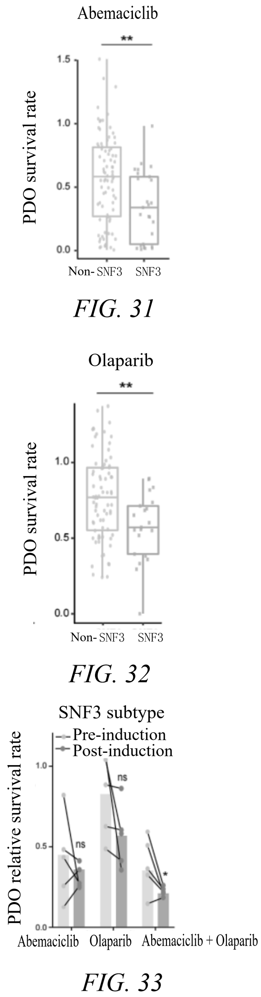

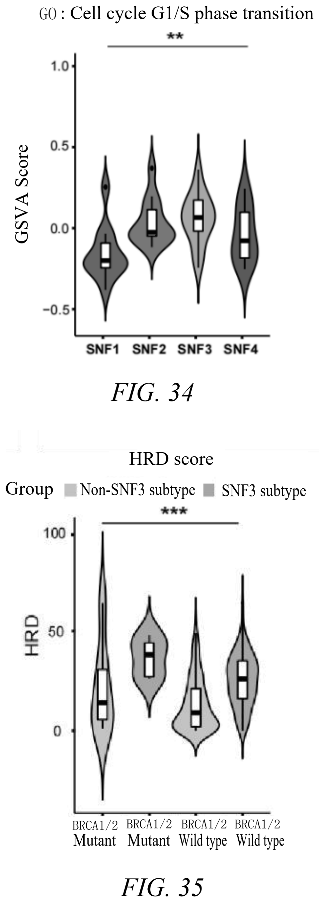

In view of the high enrichment of SNF3 tumors in homologous recombination deficiency score, chromosomal instability, and cell cycle activity, the response of SNF3 PDOs to CDK4/6 inhibitors and PARP inhibitors was tested. The growth ability of SNF3-PDO was significantly inhibited by the CDK4/6 inhibitor abemaciclib (FIG. 31) and the PARP inhibitor olaparib (FIG. 32). Interestingly, compared with each drug alone, the combination of abemaciclib and olaparib significantly reduced the survival rate of SNF3-PDO, and this effect was more pronounced after induction with 4-hydroxytamoxifen (FIG. 33). RNA-seq of PDO indicated that the cell cycle signaling pathway of SNF3-PDO was more active than that of other subtypes (FIG. 34). In addition, regardless of BRCA mutation, the homologous recombination deficiency score of SNF3 tumors was much higher (FIG. 35). The above results indicated the therapeutic susceptibility of the SNF3 subtype to CDK4/6 inhibitors and PARP inhibitors; therefore, CDK4/6 inhibitors and PARP inhibitors or combinations thereof may be potential accurate treatment strategies for SNF3 tumors.

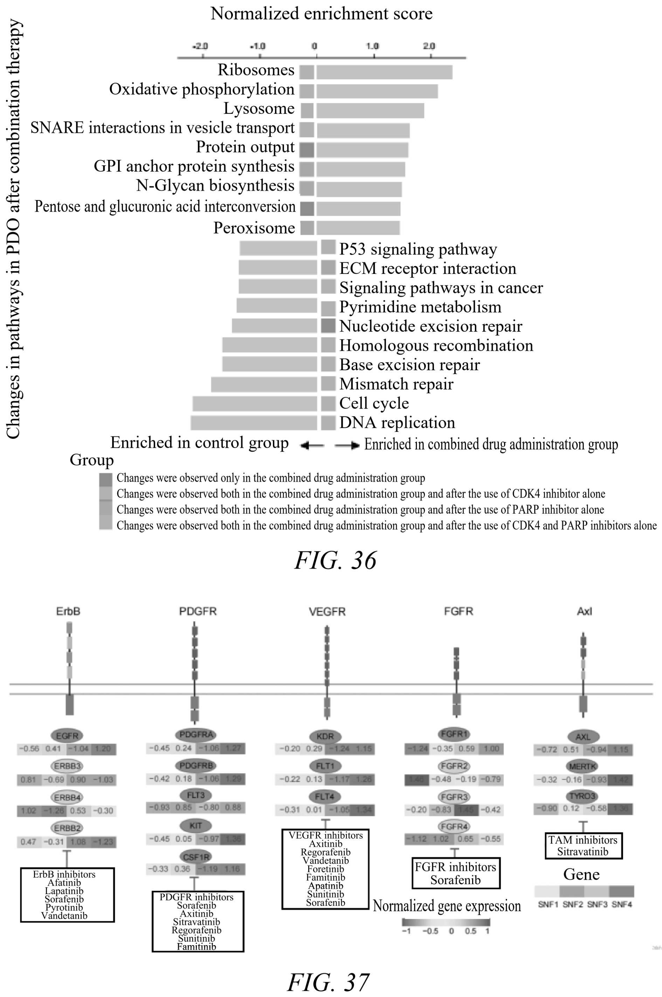

To further investigate the mechanism behind drug combinations, high-throughput transcriptome analysis was conducted on a drug testing platform called DRUG-seq (small scale high-throughput transcriptome sequencing) (Ye C, Ho DJ, Neri M, Yang C, Kulkarni T, Randhawa R, et al. DRUG-seq for miniaturized high-throughput transcriptome profiling in drug discovery. NAT COMMUN. 2018;9(1):4307 doi 10.1038/s41467-018-06500-x.). DRUG-seq showed that after combined administration, oncogenic signaling pathways related to cell cycle and DNA damage repair in SNF3 tumors were significantly downregulated (FIG. 36). Not exactly the same as changes in signaling pathways after monotherapy, some new signaling pathways have changed dramatically after combination therapy, suggesting a potential mechanism for combined administration of SNF3 tumors.

### (13) Inhibition of the receptor tyrosine kinase pathway can suppress the progression of the SNF4 subtype

In view of the enrichment of receptor tyrosine kinase signaling pathway in the SNF4 subtype, the response of the four subtypes to tyrosine kinase inhibitors was evaluated. According to the literature method (Lemmon MA, Schlessinger J. Cell signaling by receptor tyrosine kinases. CELL. 2010;141(7):1117-34 doi 10.1016/j.cell.2010.06.011.), 20 receptor tyrosine kinase subfamilies and their main receptors were identified. To screen key therapeutic targets, the expression of receptors in the receptor tyrosine kinase pathway was first compared, and it was found that in the SNF4 subtype, most receptors in some receptor tyrosine kinase pathways (such as PDGFR and VEGFR pathways) were significantly highly expressed (FIG. 37).

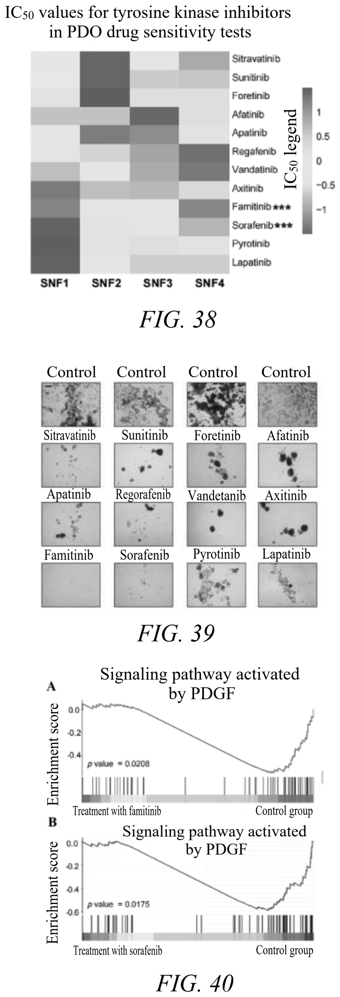

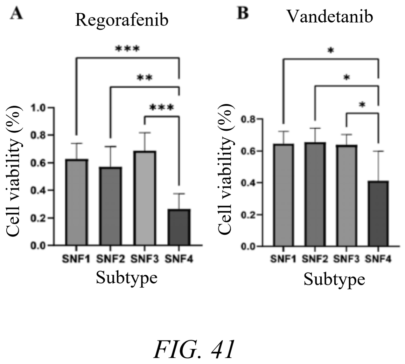

Next, based on the identified key receptors, 12 tyrosine kinase inhibitors were selected to construct a "tyrosine kinase inhibitor combination," including tyrosine kinase inhibitors targeting the ErbB family (afatinib, lapatinib, pyrotinib), the PDGFR family (axitinib, sunitinib), the VEGFR family (foretinib, apatinib), the TAM receptor (sitravatinib), as well as some multi-target tyrosine kinase inhibitors (sorafenib, vandetanib, regorafenib, famitinib). The combination is used for PDO drug sensitivity testing to verify the tyrosine kinase inhibitor most sensitive to SNF4 tumors (FIG. 38). The results showed that in SNF4 tumors, the IC50 of most tyrosine kinase inhibitors, especially four drugs famitinib, sorafenib, regorafenib, and vandetanib, was lower than that of other subtypes (FIGs. 38 and 39). After treatment with famitinib (FIG. 40A) and sorafenib (FIG. 40B), the signaling pathway activated by PDGF in SNF4 tumor was significantly downregulated. After treatment with regorafenib (FIG. 41A) and vandetanib (FIG. 41B), the cell viability of SNF4 tumors was significantly decreased (Wilcoxon test, *, p < 0.05; **, p < 0.01; ***, p < 0.001). Combining the drug sensitivity results of the above 12 tyrosine kinase inhibitors and the targets of the above four drugs, VEGFR and PDGFR signaling were preliminarily identified as potential therapeutic vulnerabilities of SNF4 tumors.

### (14) Real-world study

We established a multi-omics cohort (n = 351), a multicenter real-world clinical cohort (n = 643), a prospective clinical cohort (MULAN trial), and a drug testing platform, which platform included organoids derived from HR+/HER2- breast cancer patients (n = 126) and patient-derived tumor fragments (n = 49). By integrating "Bench" and "Bedside" data, we conducted comprehensive preclinical and clinical translational studies on accurate strategies for HR+/HER2- breast cancer.

We established a comprehensive classification system for HR+/HER2- breast cancer, including four distinct subtypes. Through clinical cohort studies, omics analyses, and functional assays, our research demonstrated the efficacy and fundamental mechanisms of accurate treatment methods targeting each subtype: for the classic luminal intraluminal subtype, only endocrine therapy was used; for the proliferative subtype, CDK4/6 inhibitor and PARP inhibitor were added; for the immunomodulatory subtype, immunotherapy was used; for the receptor tyrosine kinase-driven subtype, tyrosine kinase inhibitors were used. Using clinically applicable subtype classification methods, we validated in a real-world cohort that matched accurate treatment strategies were superior to unmatched methods, with the median progression-free survival of patients with refractory advanced HR+/HER2- breast cancer nearly doubled (9.83 months [95% Cl, 5.74-13.93] vs 4.77 months [95% Cl, 3.35-6.18]; Hazard ratio 0.64 [95% Cl, 0.41-0.99]). Importantly, the first stage analysis of the MULAN phase II umbrella clinical trial (NCT04355858) confirmed that subtype-directed accurate treatment exhibited a higher objective response rate (88.9%, 95% CI: 51.7%-99.7%).

Our study emphasized the superiority of subtype-directed accurate treatment strategies for HR+/HER2- breast cancer, improved the traditional "one-size-fits-all" therapy, and promoted the clinical translation of accurate treatment strategies from the laboratory.

### METHODS

### Clinical cohort

### Multiomics cohort

Using the CBCGA cohort as described above, a total of 579 patients with HR+/HER2- breast cancers were included, among which, 512 had whole-exome sequencing (WES) data, 527 had somatic CNA (SCNA) data, and 569 had RNA sequencing data, and multiomics SNF subtype classification was available for 351 patients. Follow-up of patients was completed on June 30, 2021, and the median length of follow-up was 83.0 months.

### Real-world retrospective clinical cohorts

This study involved two retrospective real-world cohorts.

### Real-world cohorts of patients with early-stage HR+/HER2- breast cancer

We retrospectively reviewed patients who received surgical resection at FUSCC from January 2014 to December 2021. Patients were selected according to the following criteria: 1) tumor specimens were histopathologically confirmed as HR+/HER2- unilateral invasive carcinoma; 2) digital pathology images of their tumors were available; 3) no evidence of distant metastasis; 4) complete medical history for further analysis. Their clinico-pathological characteristics, including tumor size, lymph node status, histological grade as well as ER, PR and Ki67 expression were obtained from their original medical records.

### Real-world cohorts of patients with advanced HR+/HER2- breast cancer

For deeper insights into the landscape of metastatic HR+/HER2- breast cancer, we constructed a real-world cohort of patients with advanced HR+/HER2- breast cancer from FUSCC and invited other three centers to participate in our real-world study. 462 patients were finally included according to the following criteria: 1) treated at the four medical centers from January 2014 to December 2021; 2) tumor specimens sampled by biopsy or surgical resection were histopathologically confirmed as metastatic sites of HR+/HER2- breast cancer; 3) acquirable digital pathology images; 4) complete medical history for further analysis of their therapeutic responses. Patients within this cohort received fulvestrant backbone treatment or targeted therapy including CDK4/6 inhibitors and TKIs. Their tumor progression were evaluated according to RECIST v1.1.

### Prospective clinical cohort

The prospective clinical cohort of HR+/HER2- breast cancer comes from the MULAN clinical trial (ClinicalTrials.gov Identifier: NCT04355858). To be enrolled in this study, patients must meet all of the following inclusion criteria: 1) treated at the FUSCC from April 2020 to May 2023; 2) histologically confirmed HR+/HER2- invasive breast cancer; 3) locally advanced breast cancer (incapable of receiving treatment); 3) locally advanced breast cancer (incapable of receiving radical local treatment) or recurrent metastatic breast cancer; 4) patients with HR+/HER2- advanced breast cancer who have a history of endocrine resistance (previously treated with CDK4/6 inhibitors) and are currently in the stage of disease progression are eligible if they meet one of the following conditions: (1) patients who meet the criteria for primary endocrine resistance (recurrence or metastasis within two years after starting adjuvant endocrine therapy, or disease progression within 6 months of endocrine therapy in the advanced stage) and have received at least one round of chemotherapy in the advanced stage; (2) patients with liver metastases who have failed at least one round of endocrine therapy and one round of chemotherapy in the advanced stage; (3) patients without liver metastases who have failed at least two rounds of endocrine therapy and one round of chemotherapy in the advanced stage.

### RESULTS

### Multipronged precision medicine exploration: research cohorts and study design

To optimize the traditional "one-size-fits-all" treatment strategy for HR+/HER2- breast cancer, we established molecular subtypes based on multiomics cohort (n = 351) and successfully divided the HR+/HER2- populations into four SNF subtypes.

A multi-pronged approach to precision therapy exploration was employed in this study (Figure 44B). We established multicenter real-world retrospective clinical cohorts (n = 643) of HR+/HER2- breast cancer to further explore the clinical characteristics, prognosis, and treatment sensitivity of the four subtypes. Moreover, as described above, we constructed PDO (n = 126) and PDTF models (n = 49) for drug testing and proposed subtyping-directed precision treatment strategies. Finally, we validated the efficacy of the precision treatment strategies in the prospective clinical cohort of HR+/HER2- breast cancer. According to the deep learning-based digital pathological classification process, we performed SNF subtype classification on patients using hematoxylin and eosin (H&E)-stained sections in our study. Overall, we conducted a comprehensive and in-depth study of subtype-directed precision treatment strategies for HR+/HER2- breast cancer to achieve translation from multiomics analysis to functional assays and from bench to bedside.

### Clinical characteristics of the four subtypes in real-world cohorts

We analyzed the clinical characteristics of the four SNF subtypes (determined by digital pathology) in multicenter real-world cohorts including patients with HR+/HER2- breast cancer (Figure 45A). In a real-world cohort of early-stage HR+/HER2- breast cancer (early cohort, n = 216), the pathological characteristics of the four SNF subtypes were similar to those in a multiomics cohort, as described herein. The morphological features of each SNF subtype were as follows: SNF1 subtype, partially conserved the morphology of normal breast gland; SNF2 subtype, high immune cell infiltration; SNF3 subtype, high abundance of atypical tumor cells; and SNF4 subtype, enrichment of tumor cell clusters with surrounding fibroblasts. ER expression was lower in the SNF2 subtype in the real-world cohort of HR+/HER2- breast cancers. In addition, the SNF3 tumors had a significantly higher Ki-67 index measured through immunohistochemical (IHC) staining. More importantly, the proportion of each subtype in the real-world early cohort was also consistent with that in the multi-omics cohort of the same early-stage patients. These results matched the subtype characteristics of multiomics classification, which demonstrated the feasibility of distinguishing the four subtypes based on digital pathology.

### Response to conventional therapy of patients grouped by subtypes in real-world cohorts

In the real-world cohort, we also analyzed the response of the subtypes to traditional clinical treatments. Among patients with HR+/HER2- breast cancer receiving endocrine therapy after surgery in the early-stage cohort, patients with the SNF4 subtype were associated with worse relapse-free survival (RFS), whereas SNF1 patients were associated with better RFS (log-rank p < 0.01, Figure 45B). We also explored whether tumor size and lymph node status were potentially confounding factors in our analyses, and found no significant imbalances across variables among the four SNF types. Compared with the early cohort, the proportion of patients with the SNF4 subtype was significantly increased in the real-world cohorts of advanced HR+/HER2- breast cancer (advanced cohort), suggesting a poor prognosis of SNF4 (p < 0.01, Figure 45C). In the advanced cohort of Fudan University Shanghai Cancer Center (FUSCC, n = 277), the SNF3 and SNF4 subtypes with fulvestrant backbone treatment were associated with worse progression-free survival (PFS) (p = 0.033, Figure 45D). Surprisingly, we found that the SNF3 subtype was more likely to achieve a partial response among patients treated with CDK4/6i (p < 0.05, Figure 45E). Significant differences in treatment responses suggested the limitations of the traditional "one-size-fits-all" treatment strategy and the necessity of precision treatments guided by the four molecular subtypes.

### Prospective clinical cohort validation of subtyping-directed precision treatment strategies

To validate the precision treatment strategies for the four subtypes, we conducted an efficacy analysis in the prospective clinical cohort of HR+/HER2- breast cancer grouped by SNF subtype in the MULAN phase II umbrella clinical trial (NCT04355858) (Figure 46A). At the cutoff date of May 2023, we found that arm II (CDK4/6i with PARPi therapy) and arm V (anti-PD-1backbone therapy) had enrolled enough patients to meet the prespecified boundary for first-stage analysis according to the study protocol. In the first-stage of arm II and V, the objective response rate (ORR) reached 85.7% (95% Cl: 42.1%-99.6%) and 57.1% (95% Cl: 18.4%-90.1%), respectively (Figure 46B-C). According to Simon's two-stage design, arm II and arm V of the MULAN study will proceed to the next stage due to their remarkable efficacy. Safety data were consistent with the known safety profiles of relevant drugs.

In our exploratory analysis of the MULAN trial, we found that patients with the SNF2 subtype in arm V (identified based on digital pathology through deep learning) had a longer duration of response to immunotherapy and more patients achieved partial response (PR) according to RECIST v1.1, when compared with other subtypes (Figure 7B). Moreover, even after progressing following multiple lines (≥5 lines) of previous therapy in the metastatic setting, patients with advanced SNF2 breast cancer achieved PR and remained stable long-term after immunotherapy. Additionally, among patients receiving combination therapy with CDK4/6i and PARPi in arm II, all four patients with SNF3 subtype and advanced breast cancer achieved PR, with one patient achieving over 12 months of duration of response and another having more than 70% tumor regression in diffuse intrahepatic metastasis (Figure 7C). Notably, in the clinical study MULAN, patients who received a precision treatment strategy guided by SNF subtypes (matched group) were more likely to achieve objective responses compared to unmatched group (88.9% [95%Cl: 51.7%-99.7%] vs 40.0% [95% CI: 5.3%-85.3%], p = 0.095, Figure 7D). Furthermore, patients with the SNF4 subtype, which was resistant to endocrine therapy, were found to be relatively sensitive to TKI therapy in the real-world clinical cohort of advanced HR+/HER2- breast cancer (Figure 46E). These findings demonstrated the benefits of matched therapies following a subtyping-directed precision treatment strategy for specific subtypes.

In order to assess the overall benefit of subtyping-directed precision treatment strategies in a real-world clinical cohort of advanced HR+/HER2- breast cancer, we included patients who had received three or more previous lines of therapies in the metastatic setting (Supplementary Table S10). Patients who underwent precision treatment guided by SNF subtyping were classified to the matched group, while those who did not receive this type of treatment were classified to the unmatched group. Supplementary Table S11 provides detailed criteria for determining which group a patient belongs to according to the SNF subtyping-directed precision treatment strategy. Notably, patients with a specific SNF subtype who received matched therapy following a subtyping-directed precision treatment strategy had a significantly better progression-free survival than those patients who received unmatched physician-chosen therapies (9.83 months [95% Cl, 5.74-13.93] vs 4.77 months [95% Cl, 3.35-6.18]; hazard ratio 0.64 [95% Cl, 0.41-0.99], p = 0.045; Figure 46F). Examples of patients achieving objective responses are shown in Figure 7G.

Altogether, our study of the clinical cohort highlighted the superiority of subtyping-directed precision treatment strategies for HR+/HER2- breast cancer, paving the way for further clinical application. These findings demonstrated the potential benefits of personalized medicine approaches and underscored the importance of using precision treatment strategies tailored to specific subtypes for the treatment of HR+/HER2- breast cancer.

## Claims

1. A system for typing HR-positive/HER2-negative breast cancer, **characterized in that** the system comprises:
1) a whole-slide digital pathology image data preprocessing module, wherein the module is configured to segment whole-slide digital pathology images of tumor tissue samples of the HR-positive/HER2-negative breast cancer into multiple enlarged image patches;
2) a first neural network module, wherein the first neural network module is a tissue type segmentation neural network module, and is configured to classify the image patches into different tissue types and assign corresponding tissue labels;
3) a second neural network module connected in series with the first neural network module, wherein the second neural network module is a subtype prediction neural network module, and is configured to perform the typing on the samples based on the image patches with the tissue labels;
wherein the typing refers to dividing HR-positive/HER2-negative breast cancer into the following four subtypes: classic luminal subtype, immunomodulatory subtype, proliferative subtype, and receptor tyrosine kinase-driven subtype.

2. The system of claim 1, **characterized in that** the first neural network module and the second neural network module are constructed by artificial intelligence deep learning based on whole-slide digital pathology images of tumor tissue samples from patients with known HR-positive/HER2-negative breast cancer subtypes.

3. The system of claim 1 or 2, **characterized in that** the tissue types are tumor, stroma, immune infiltration, normal gland, and necrosis or hemorrhage, and the tissue labels are tumor, stroma, immune infiltration, normal gland, and necrosis or hemorrhage.

4. The system of claim 1 or 2, **characterized in that** the tissue labels are tumor, stroma, and/or immune infiltration.

5. The system of claim 1 or 2, **characterized in that** the artificial intelligence deep learning comprises training and validating a convolutional neural network model using the image patches with the tissue labels and corresponding HR-positive/HER2-negative breast cancer subtypes of the image patches with the tissue labels.

6. The system of claim 5, **characterized in that** the artificial intelligence deep learning comprises training and validating the convolutional neural network model using a three-fold cross-validation algorithm.

7. The system of claim 5, **characterized in that** the artificial intelligence deep learning comprises training and validating the convolutional neural network model by employing a nonlinear classifier of an artificial intelligence support vector machine and a one-versus-many method.

8. The system of claim 5, **characterized in that** a tissue type segmentation neural network model is obtained by neural network modeling using a ResNet-18 network architecture.
